# EUROPEAN PATENT APPLICATION

(11) **EP 4 122 953 A1**
(43) Date of publication of application: **25.01.2023**
(21) Application number: 21771868.3
(22) Date of filing: 18.03.2021
(51) Int. Cl.: C07K 14/55, C07K 14/705, C12N 15/62, C12N 15/85, A61K 38/16, A61P 35/00, A61P 31/00, A61K 38/00

(54) **FUSION PROTEIN COMPRISING IL-2 PROTEIN AND CD80 PROTEIN FRAGMENT OR VARIANT THEREOF, AND USES THEREOF**

(30) Priority: 18.03.2020 KR 20200033232; 19.03.2020 KR 20200034031
(71) Applicant: GI Innovation, Inc., Seoul 05855 (KR)
(72) Inventor: JANG, Myung, Ho, Seoul 03770 (KR); PARK, Jae, Chan, Seoul 05853 (KR)
(74) Representative: HGF
(86) International application number: PCT/KR2021/003334
(87) International publication number: WO 2021/187904

(57) **Abstract**

The present invention relates to a fusion protein dimer comprising a modified IL-2 protein and a CD80 protein fragment or a variant thereof. The fusion protein comprising an IL-2 protein and a CD80 protein fragment or a variant thereof can not only activate immune cells owing to IL-2, but also effectively regulate Treg cells owing to CD80. Therefore, the fusion protein can efficiently attack cancer cells, and thus can be usefully employed for treatment of cancer or an infectious disease.

## Description

### Technical Field

The present invention relates to a fusion protein comprising an IL-2 protein and a CD80 protein fragment or a variant thereof and a use thereof. Specifically, the present invention relates to a novel fusion protein for treatment of cancer having an immune enhancing efficacy.

### Background Art

Interleukin 2 (IL-2), also called T-cell growth factor (TCGF), is a globular glycoprotein that plays a central role in lymphocyte production, survival, and homeostasis. IL-2 has a protein size of 15.5 kDa to 16 kDa and consists of 133 amino acids. IL-2 mediates various immune actions by binding to an IL-2 receptor composed of three distinct subunits.

In addition, IL-2 is synthesized mainly by activated T cells, in particular by CD4+ helper T cells. IL-2 stimulates proliferation and differentiation of T cells, and induces production of cytotoxic T lymphocytes (CTLs) and differentiation of peripheral blood lymphocytes into cytotoxic cells and lymphokine-activated killer cells (LAK cells).

Furthermore, IL-2 is involved in proliferation and differentiation of B cells, promotes immunoglobulin synthesis by B cells, and stimulates production, proliferation, and activation of natural killer cells (NK cells). Therefore, IL-2 is used as an anticancer agent, because it can increase lymphocyte populations and increase the function of the immune cells in the living body. Currently, therapy with IL-2 has been approved and used for patients with metastatic renal cell carcinoma and malignant melanoma.

However, IL-2 has a dual function in immune responses in that it is important not only for mediating an increase in number of immune cells and activity thereof, but also for maintaining immune tolerance. In addition, it has been reported that IL-2 may not be optimal for inhibiting tumor growth. The reason is that in the presence of IL-2, activation-induced cell death (AICD) may occur in the resulting cytotoxic T lymphocytes and immune responses may be inhibited by IL-2 dependent regulatory T cells (Treg cells) (Imai et al., Cancer Sci 98, 416-423, 2007).

In addition, severe cardiovascular, pulmonary, renal, hepatic, gastrointestinal, neuronal, dermatological, hematological, and systemic side effects occur in patients who have received immunotherapy with IL-2. Therefore, various IL-2 mutants have been studied to improve therapeutic efficacy of IL-2 and minimize side effects thereof (US 5,229,109 B). However, there are still many problems to be solved in order to utilize IL-2 for pharmacological purposes.

Meanwhile, CD80, also known as B7-1, is a member of the B7 family of membrane-bound proteins that are involved in immune regulation by binding to its ligand by way of delivering costimulatory responses and coinhibitory responses. CD80 is a transmembrane protein expressed on the surface of T cells, B cells, dendritic cells, and monocytes. CD80 is known to bind CD28, CTLA4 (CD152), and PD-L1. CD80, CD86, CTLA4, and CD28 are involved in a costimulatory - coinhibitory system. For example, they regulate activity of T cells and are involved in proliferation, differentiation, and survival of T cells.

For example, when CD80 and CD86 interact with CD28, costimulatory signals are generated to activate T cells. Eventually, CD80 binds to CTLA4 expressed on the surface of activated T cells and stimulates CTLA4 to be upregulated. As a result, CD80 inhibits T cell responses prior to immune response operation caused by CD80/CD28 interaction. This feedback loop allows for fine regulation of immune responses.

In addition, CD80 is known to bind PD-L1, another B7 family member, with affinity similar to that with which CD28 binds PD-L1. PD-L1 is known as one of two ligands for programmed death-1 (PD-1) protein, and PD-L1 is known to be involved in T cell regulation. Binding of CD80 to PD-L1 is another mechanism that can block PD-1/PD-L1 interaction, which may prevent inhibition of T cell responses in tumors. At the same time, however, an increase in CD80 levels causes CD80 to bind to CD28 so that CTLA4 is induced, thereby inducing or inhibiting T cell responses.

### Detailed Description of Invention

### Technical Problem

The present inventors have studied to develop IL-2 which is safe and effective. As a result, the present inventors have discovered that a novel fusion protein comprising, in one molecule, an IL-2 protein and a CD80 protein fragment or a variant thereof can activate immune cells and effectively regulate Treg cells, thereby completing the present invention.

### Solution to Problem

In order to achieve the above object, in an aspect of the present invention, there is provided a fusion protein comprising an IL-2 protein and a CD80 protein fragment or a variant thereof.

In another aspect of the present invention, there is provided a fusion protein dimer obtained by binding two of the fusion proteins to each other.

In yet another aspect of the present invention, there is provided a polynucleotide encoding the fusion protein.

In still yet another aspect of the present invention, there is provided a vector comprising the polynucleotide.

In still yet another aspect of the present invention, there is provided a transformed cell into which the vector has been introduced.

In still yet another aspect of the present invention, there is provided a pharmaceutical composition for preventing or treating cancer or an infectious disease, comprising, as an active ingredient, the fusion protein or the fusion protein dimer.

In still yet another aspect of the present invention, there is provided a use of the fusion protein for treatment of cancer or an infectious disease.

In still yet another aspect of the present invention, there is provided a use of the fusion protein for manufacture of a medicament for treating cancer or an infectious disease.

### Effects of the Invention

A fusion protein comprising an IL-2 protein and a CD80 protein fragment or a variant thereof can not only activate immune cells owing to IL-2, but also effectively regulate Treg cells owing to CD80. Therefore, the fusion protein can efficiently attack cancer cells, and thus can be usefully employed for treatment of cancer or an infectious disease.

### Brief Description of Drawings

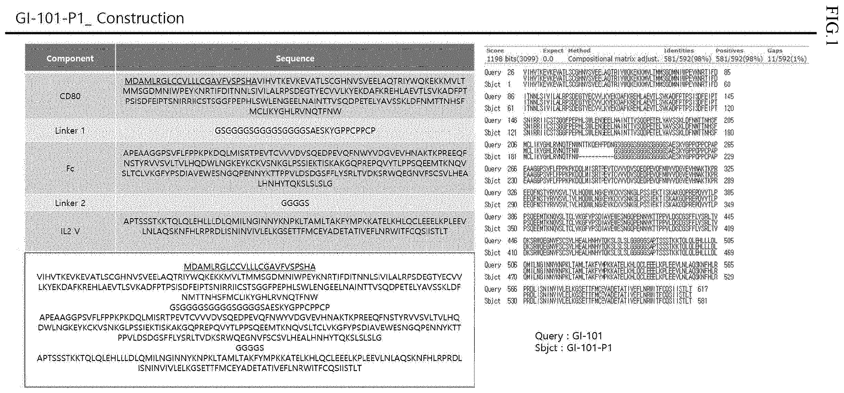

FIG. 1 illustrates components of GI-101_P1 and peptide sequences thereof (CD80: SEQ ID NO: 38, Linker 1: SEQ ID NO: 3, Fc: SEQ ID NO: 4, Linker 2: SEQ ID NO: 5, IL2V: SEQ ID NO: 6, GI101_P1: SEQ ID NO: 39).

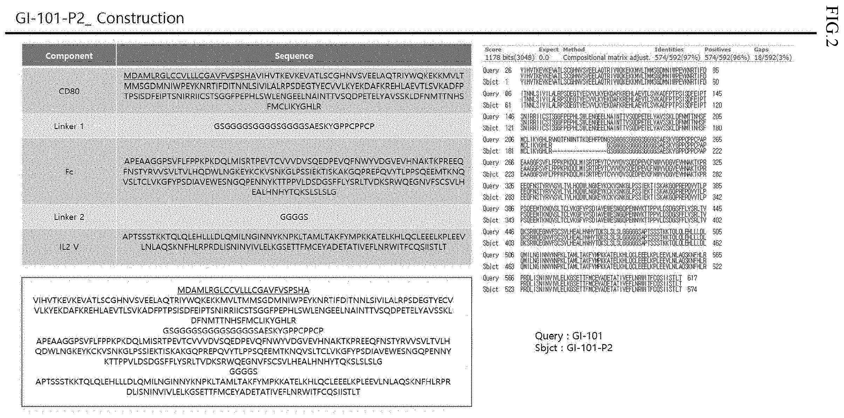

FIG. 2 illustrates components of GI-101_P2 and peptide sequences thereof (CD80: SEQ ID NO: 40, Linker 1: SEQ ID NO: 3, Fc: SEQ ID NO: 4, Linker 2: SEQ ID NO: 5, IL2V: SEQ ID NO: 6, GI101 P2: SEQ ID NO: 41).

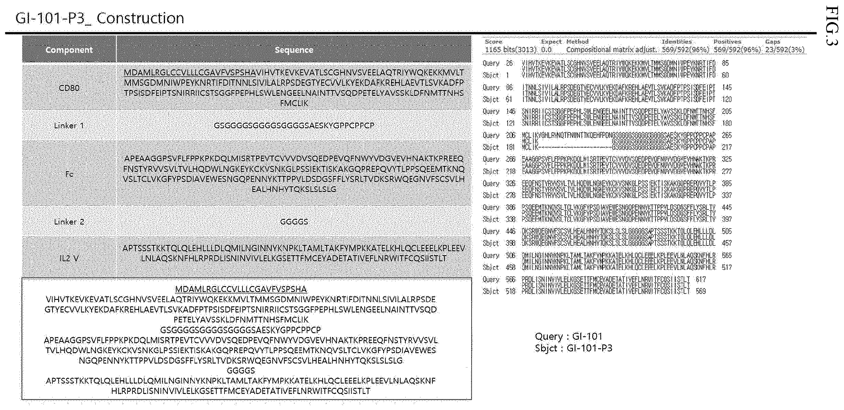

FIG. 3 illustrates components of GI-101_P3 and peptide sequences thereof (CD80: SEQ ID NO: 42, Linker 1: SEQ ID NO: 3, Fc: SEQ ID NO: 4, Linker 2: SEQ ID NO: 5, IL2V: SEQ ID NO: 6, GI101_P3: SEQ ID NO: 43).

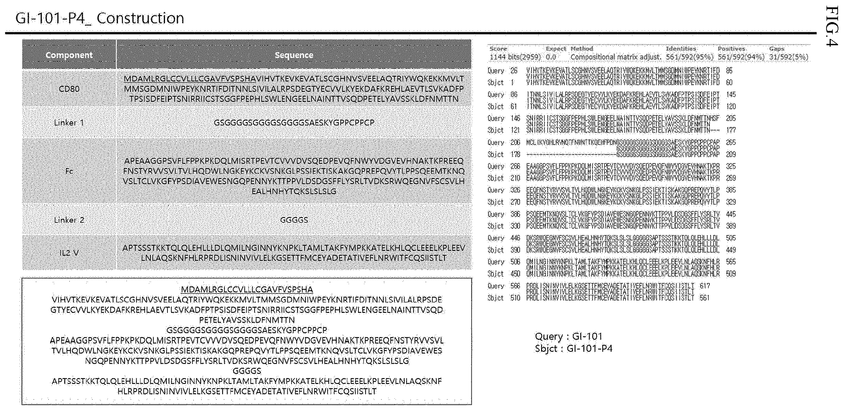

FIG. 4 illustrates components of GI-101_P4 and peptide sequences thereof (CD80: SEQ ID NO: 44, Linker 1: SEQ ID NO: 3, Fc: SEQ ID NO: 4, Linker 2: SEQ ID NO: 5, IL2V: SEQ ID NO: 6, GI101_P4: SEQ ID NO: 45).

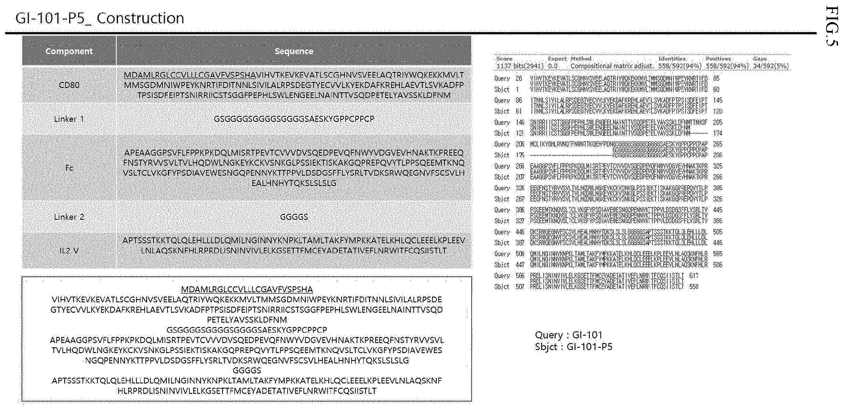

FIG. 5 illustrates components of GI-101_P5 and peptide sequences thereof (CD80: SEQ ID NO: 46, Linker 1: SEQ ID NO: 3, Fc: SEQ ID NO: 4, Linker 2: SEQ ID NO: 5, IL2V: SEQ ID NO: 6, GI101_P5: SEQ ID NO: 47).

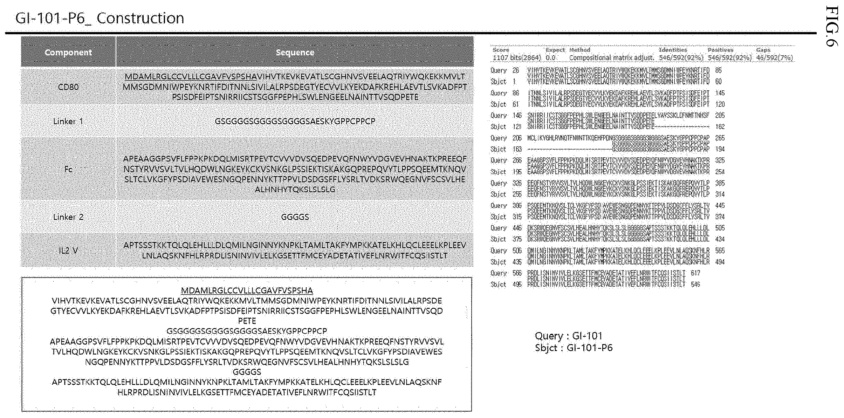

FIG. 6 illustrates components of GI-101_P6 and peptide sequences thereof (CD80: SEQ ID NO: 48, Linker 1: SEQ ID NO: 3, Fc: SEQ ID NO: 4, Linker 2: SEQ ID NO: 5, IL2V: SEQ ID NO: 6, GI101_P6: SEQ ID NO: 49).

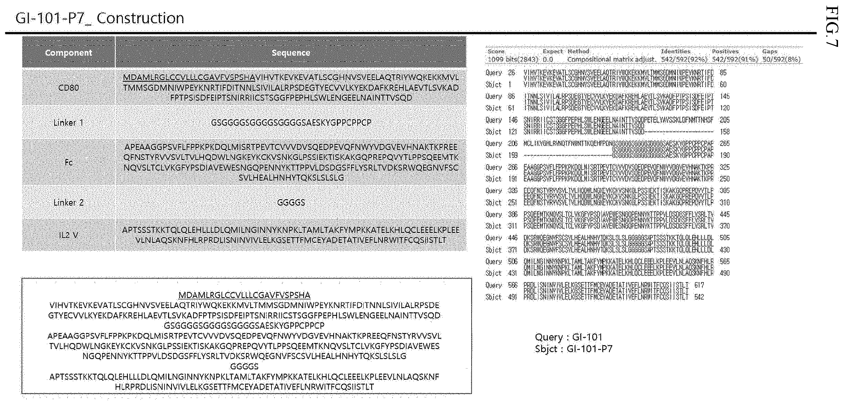

FIG. 7 illustrates components of GI-101_P7 and peptide sequences thereof (CD80: SEQ ID NO: 50, Linker 1: SEQ ID NO: 3, Fc: SEQ ID NO: 4, Linker 2: SEQ ID NO: 5, IL2V: SEQ ID NO: 6, GI101 P7: SEQ ID NO: 51).

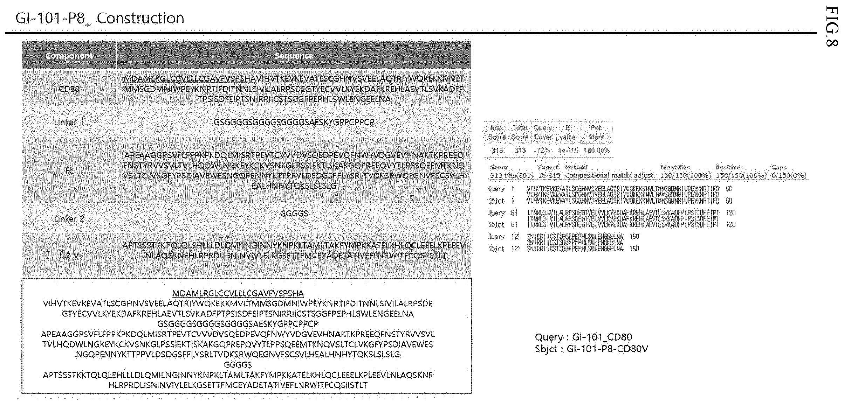

FIG. 8 illustrates components of GI-101_P8 and peptide sequences thereof (CD80: SEQ ID NO: 52, Linker 1: SEQ ID NO: 3, Fc: SEQ ID NO: 4, Linker 2: SEQ ID NO: 5, IL2V: SEQ ID NO: 6, GI101_P8: SEQ ID NO: 53).

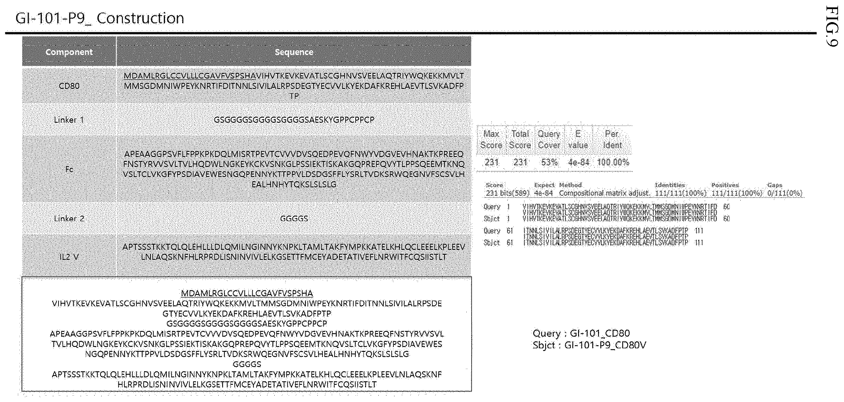

FIG. 9 illustrates components of GI-101_P9 and peptide sequences thereof (CD80: SEQ ID NO: 54, Linker 1: SEQ ID NO: 3, Fc: SEQ ID NO: 4, Linker 2: SEQ ID NO: 5, IL2V: SEQ ID NO: 6, GI101_P9: SEQ ID NO: 55).

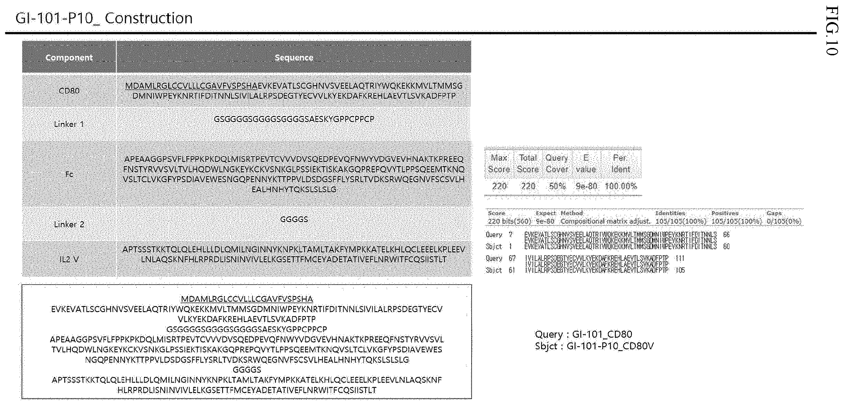

FIG. 10 illustrates components of GI-101_P10 and peptide sequences thereof (CD80: SEQ ID NO: 56, Linker 1: SEQ ID NO: 3, Fc: SEQ ID NO: 4, Linker 2: SEQ ID NO: 5, IL2V: SEQ ID NO: 6, GI101_P10: SEQ ID NO: 57).

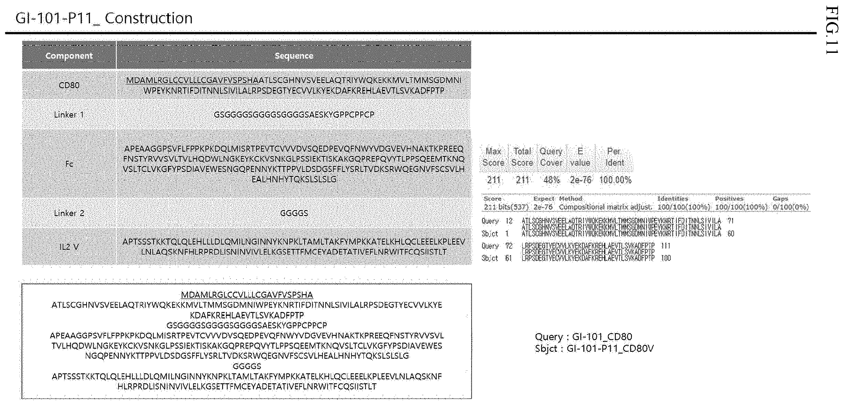

FIG. 11 illustrates components of GI-101_P11 and peptide sequences thereof (CD80: SEQ ID NO: 58, Linker 1: SEQ ID NO: 3, Fc: SEQ ID NO: 4, Linker 2: SEQ ID NO: 5, IL2V: SEQ ID NO: 6, GI101 P11: SEQ ID NO: 59).

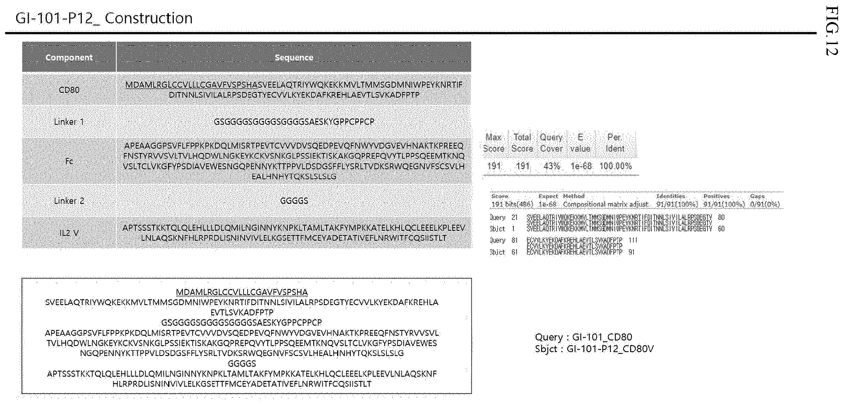

FIG. 12 illustrates components of GI-101_P12 and peptide sequences thereof (CD80: SEQ ID NO: 60, Linker 1: SEQ ID NO: 3, Fc: SEQ ID NO: 4, Linker 2: SEQ ID NO: 5, IL2V: SEQ ID NO: 6, GI101 P12: SEQ ID NO: 61).

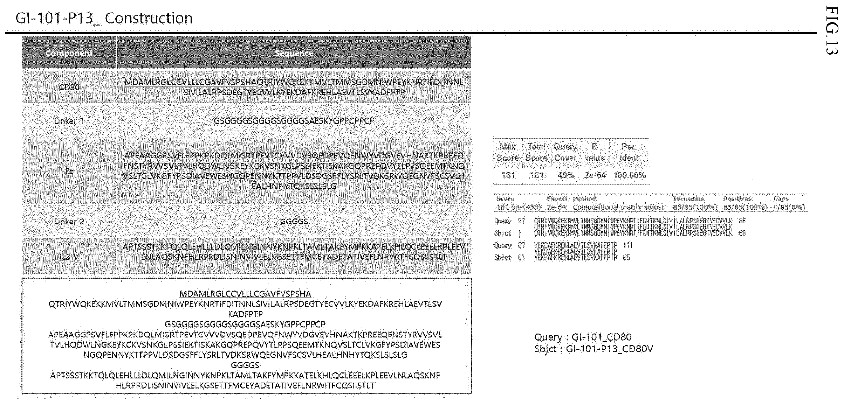

FIG. 13 illustrates components of GI-101_P13 and peptide sequences thereof (CD80: SEQ ID NO: 62, Linker 1: SEQ ID NO: 3, Fc: SEQ ID NO: 4, Linker 2: SEQ ID NO: 5, IL2V: SEQ ID NO: 6, GI101 P13: SEQ ID NO: 63).

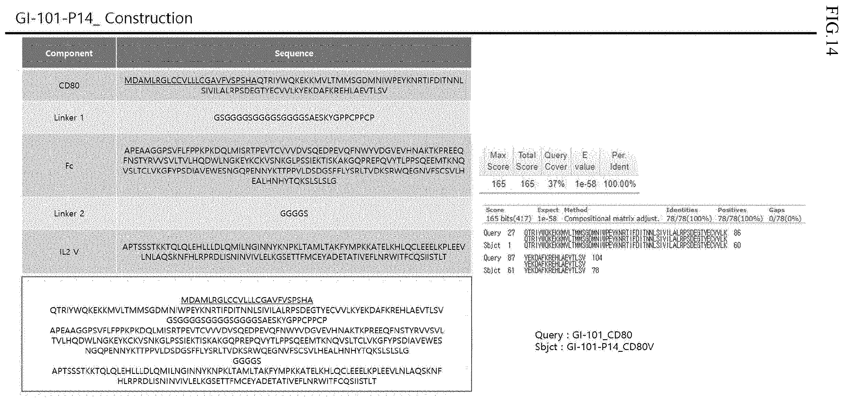

FIG. 14 illustrates components of GI-101_P14 and peptide sequences thereof (CD80: SEQ ID NO: 64, Linker 1: SEQ ID NO: 3, Fc: SEQ ID NO: 4, Linker 2: SEQ ID NO: 5, IL2V: SEQ ID NO: 6, GI101_P14: SEQ ID NO: 65).

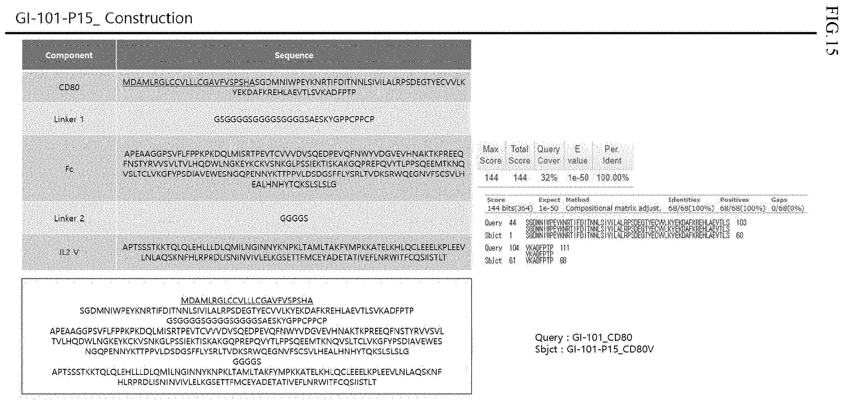

FIG. 15 illustrates components of GI-101_P15 and peptide sequences thereof (CD80: SEQ ID NO: 66, Linker 1: SEQ ID NO: 3, Fc: SEQ ID NO: 4, Linker 2: SEQ ID NO: 5, IL2V: SEQ ID NO: 6, GI101 P15: SEQ ID NO: 67).

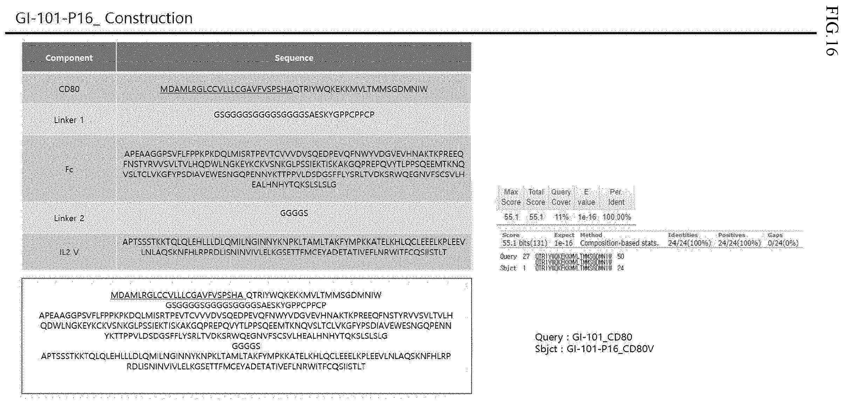

FIG. 16 illustrates components of GI-101_P16 and peptide sequences thereof (CD80: SEQ ID NO: 68, Linker 1: SEQ ID NO: 3, Fc: SEQ ID NO: 4, Linker 2: SEQ ID NO: 5, IL2V: SEQ ID NO: 6, GI101 P16: SEQ ID NO: 69).

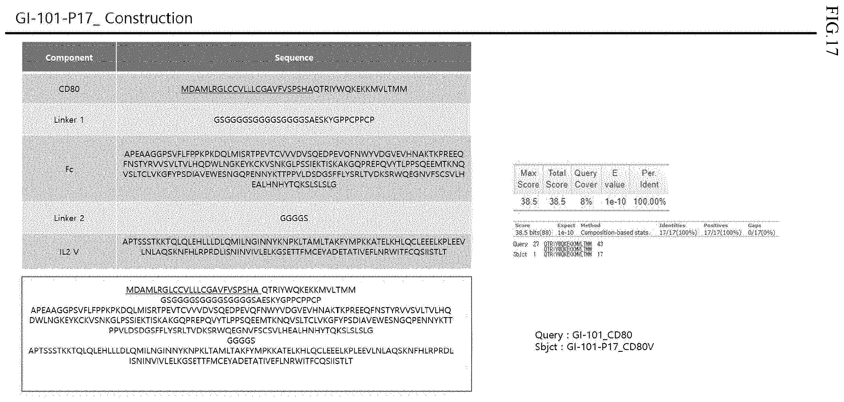

FIG. 17 illustrates components of GI-101_P17 and peptide sequences thereof (CD80: SEQ ID NO: 70, Linker 1: SEQ ID NO: 3, Fc: SEQ ID NO: 4, Linker 2: SEQ ID NO: 5, IL2V: SEQ ID NO: 6, GI101 P17: SEQ ID NO: 71).

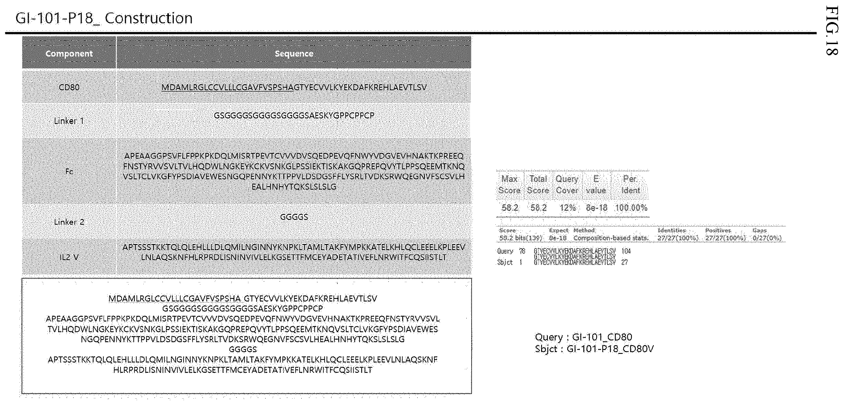

FIG. 18 illustrates components of GI-101_P18 and peptide sequences thereof (CD80: SEQ ID NO: 72, Linker 1: SEQ ID NO: 3, Fc: SEQ ID NO: 4, Linker 2: SEQ ID NO: 5, IL2V: SEQ ID NO: 6, GI101 P18: SEQ ID NO: 73).

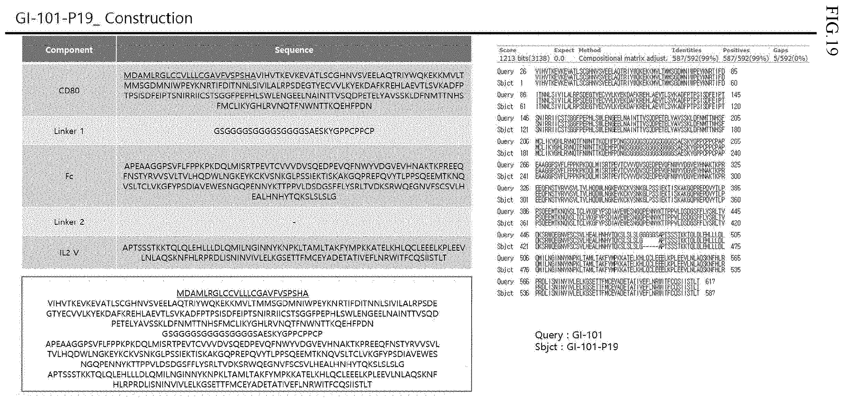

FIG. 19 illustrates components of GI-101_P19 and peptide sequences thereof (CD80: SEQ ID NO: 74, Linker 1: SEQ ID NO: 3, Fc: SEQ ID NO: 4, IL2V: SEQ ID NO: 6, GI101_P19: SEQ ID NO: 75).

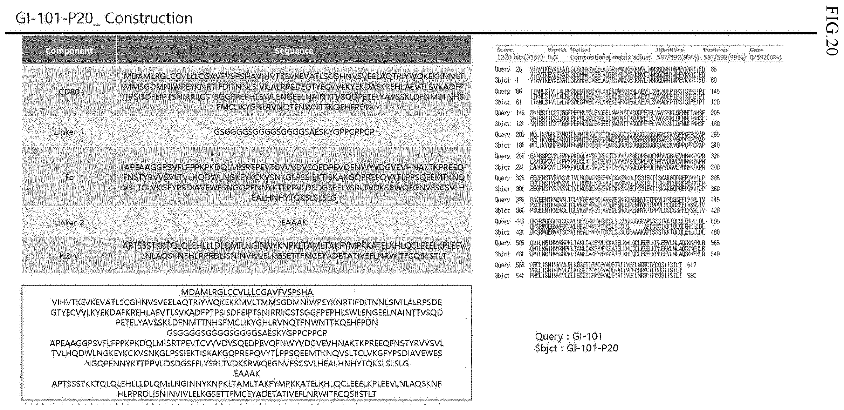

FIG. 20 illustrates components of GI-101_P20 and peptide sequences thereof (CD80: SEQ ID NO: 74, Linker 1: SEQ ID NO: 3, Fc: SEQ ID NO: 4, Linker 2: SEQ ID NO: 76, IL2V: SEQ ID NO: 6, GI101_P20: SEQ ID NO: 77).

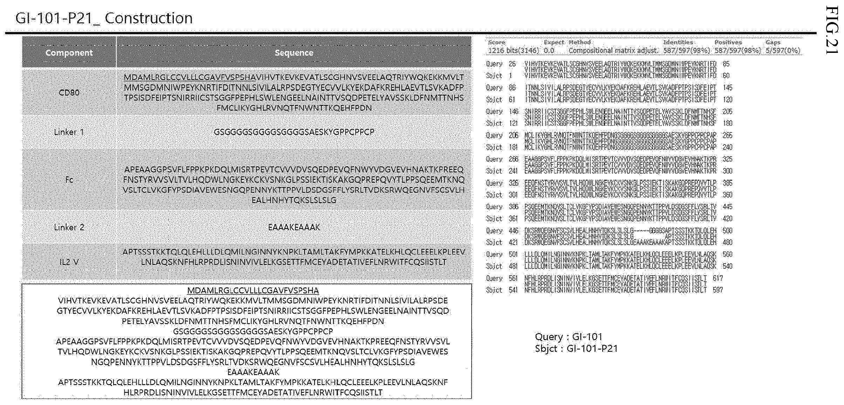

FIG. 21 illustrates components of GI-101_P21 and peptide sequences thereof (CD80: SEQ ID NO: 74, Linker 1: SEQ ID NO: 3, Fc: SEQ ID NO: 4, Linker 2: SEQ ID NO: 78, IL2V: SEQ ID NO: 6, GI101 P21: SEQ ID NO: 79).

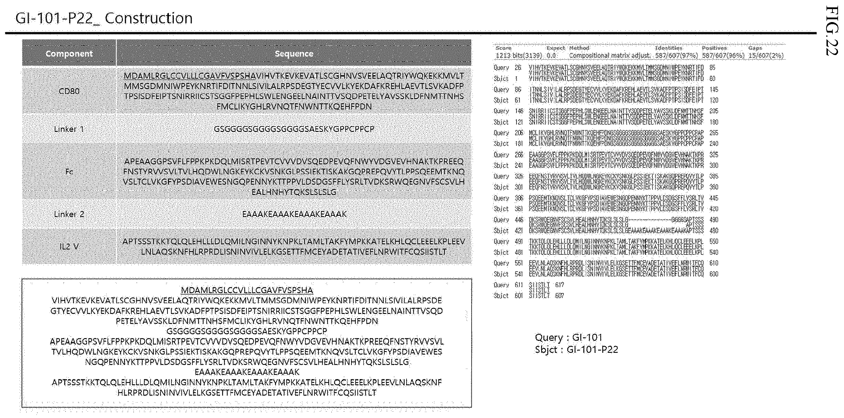

FIG. 22 illustrates components of GI-101_P22 and peptide sequences thereof (CD80: SEQ ID NO: 74, Linker 1: SEQ ID NO: 3, Fc: SEQ ID NO: 4, Linker 2: SEQ ID NO: 80, IL2V: SEQ ID NO: 6, GI101 P22: SEQ ID NO: 81).

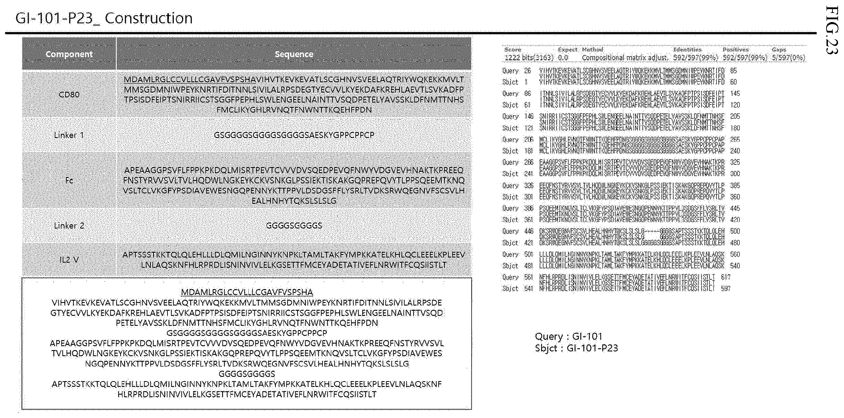

FIG. 23 illustrates components of GI-101_P23 and peptide sequences thereof (CD80: SEQ ID NO: 74, Linker 1: SEQ ID NO: 3, Fc: SEQ ID NO: 4, Linker 2: SEQ ID NO: 82, IL2V: SEQ ID NO: 6, GI101 P23: SEQ ID NO: 83).

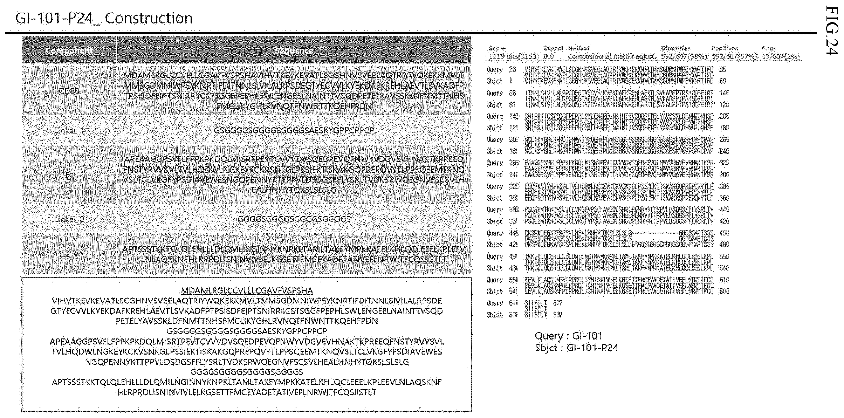

FIG. 24 illustrates components of GI-101_P24 and peptide sequences thereof (CD80: SEQ ID NO: 74, Linker 1: SEQ ID NO: 3, Fc: SEQ ID NO: 4, Linker 2: SEQ ID NO: 84, IL2V: SEQ ID NO: 6, GI101 P24: SEQ ID NO: 85).

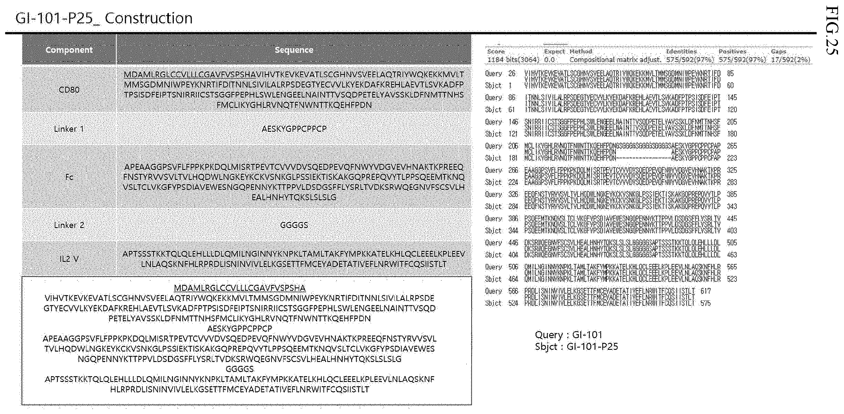

FIG. 25 illustrates components of GI-101_P25 and peptide sequences thereof (CD80: SEQ ID NO: 74, Linker 1: SEQ ID NO: 86, Fc: SEQ ID NO: 4, Linker 2: SEQ ID NO: 5, IL2V: SEQ ID NO: 6, GI101 P25: SEQ ID NO: 87).

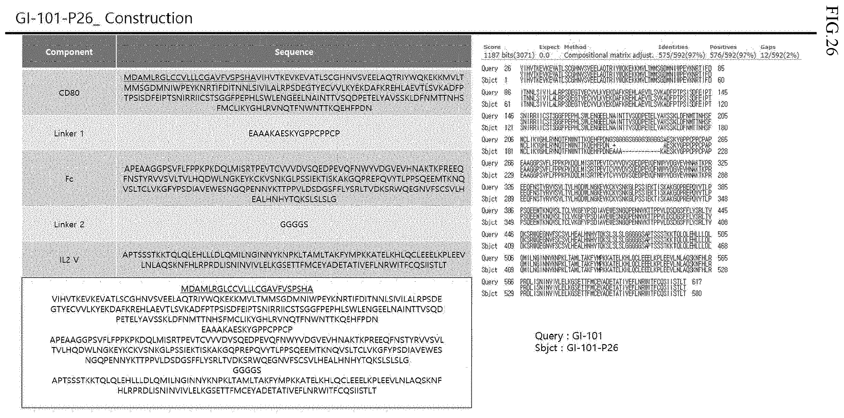

FIG. 26 illustrates components of GI-101_P26 and peptide sequences thereof (CD80: SEQ ID NO: 74, Linker 1: SEQ ID NO: 88, Fc: SEQ ID NO: 4, Linker 2: SEQ ID NO: 5, IL2V: SEQ ID NO: 6, GI101 P26: SEQ ID NO: 89).

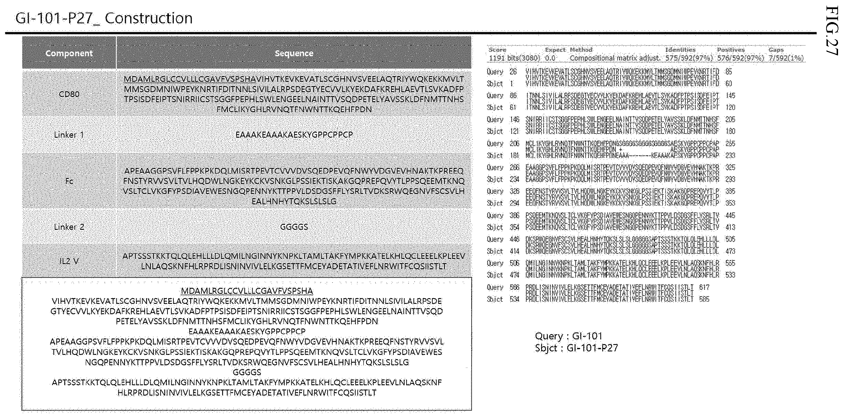

FIG. 27 illustrates components of GI-101_P27 and peptide sequences thereof (CD80: SEQ ID NO: 74, Linker 1: SEQ ID NO: 90, Fc: SEQ ID NO: 4, Linker 2: SEQ ID NO: 5, IL2V: SEQ ID NO: 6, GI101 P27: SEQ ID NO: 91).

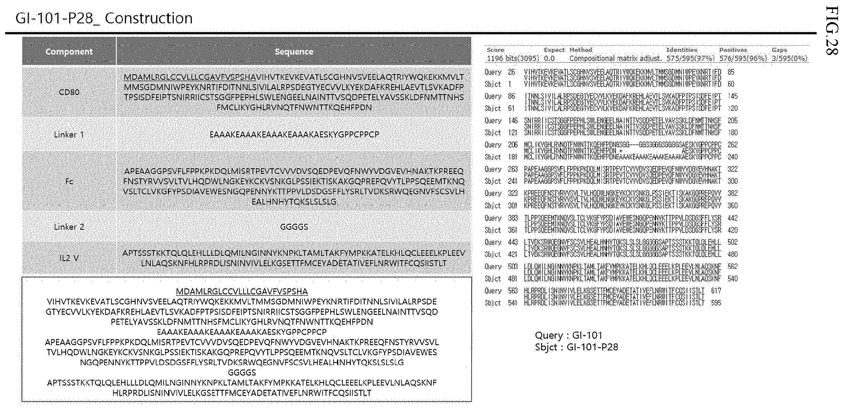

FIG. 28 illustrates components of GI-101_P28 and peptide sequences thereof (CD80: SEQ ID NO: 74, Linker 1: SEQ ID NO: 92, Fc: SEQ ID NO: 4, Linker 2: SEQ ID NO: 5, IL2V: SEQ ID NO: 6, GI101 P28: SEQ ID NO: 93).

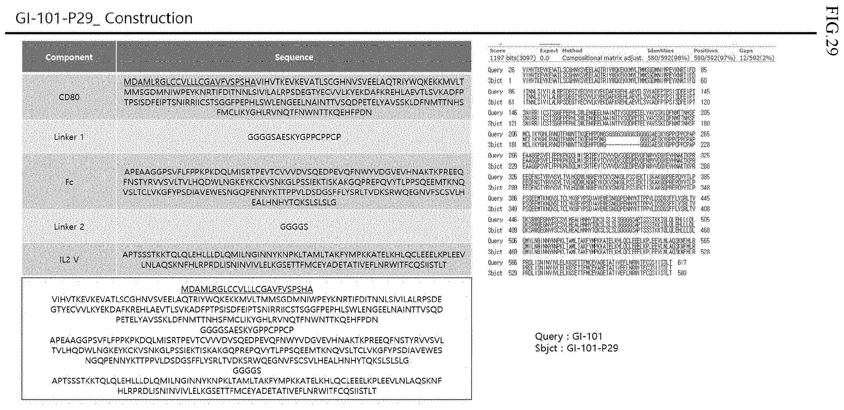

FIG. 29 illustrates components of GI-101_P29 and peptide sequences thereof (CD80: SEQ ID NO: 74, Linker 1: SEQ ID NO: 94, Fc: SEQ ID NO: 4, Linker 2: SEQ ID NO: 5, IL2V: SEQ ID NO: 6, GI101 P29: SEQ ID NO: 95).

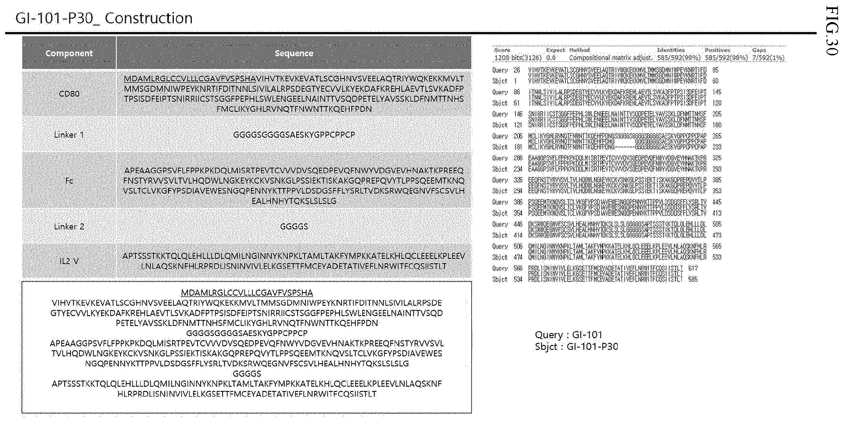

FIG. 30 illustrates components of GI-101_P30 and peptide sequences thereof (CD80: SEQ ID NO: 74, Linker 1: SEQ ID NO: 96, Fc: SEQ ID NO: 4, Linker 2: SEQ ID NO: 5, IL2V: SEQ ID NO: 6, GI101_P30: SEQ ID NO: 97).

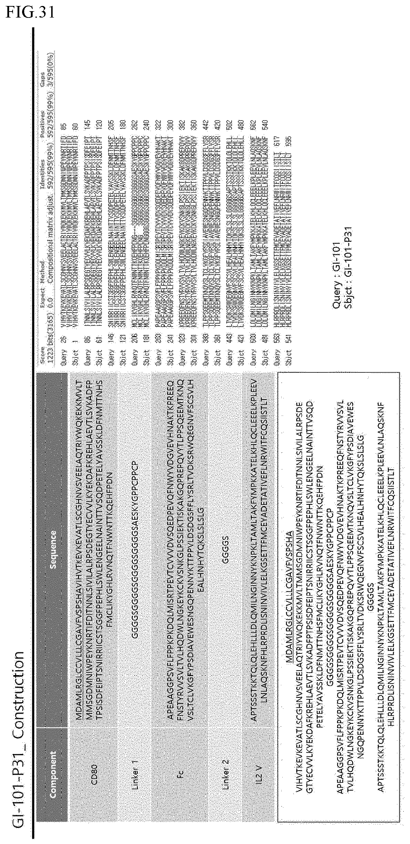

FIG. 31 illustrates components of GI-101_P31 and peptide sequences thereof (CD80: SEQ ID NO: 74, Linker 1: SEQ ID NO: 98, Fc: SEQ ID NO: 4, Linker 2: SEQ ID NO: 5, IL2V: SEQ ID NO: 6, GI101 P31: SEQ ID NO: 99).

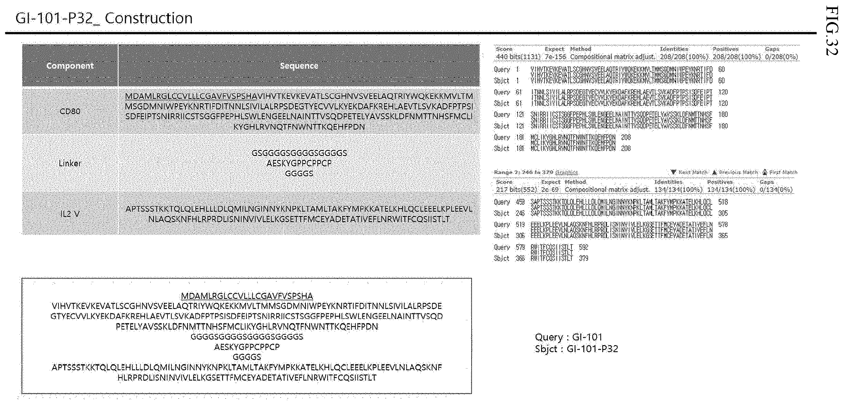

FIG. 32 illustrates components of GI-101_P32 and peptide sequences thereof (CD80: SEQ ID NO: 74, Linker: SEQ ID NO: 100, IL2V: SEQ ID NO: 6, GI101_P32: SEQ ID NO: 101).

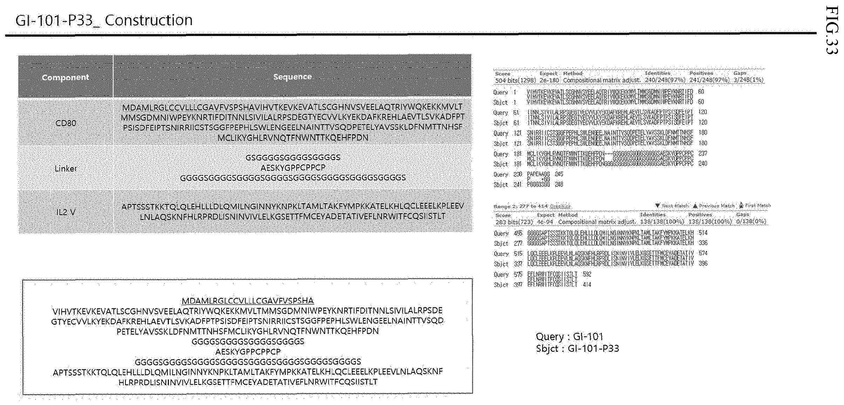

FIG. 33 illustrates components of GI-101_P33 and peptide sequences thereof (CD80: SEQ ID NO: 74, Linker: SEQ ID NO: 102, IL2V: SEQ ID NO: 6, GI101_P33: SEQ ID NO: 103).

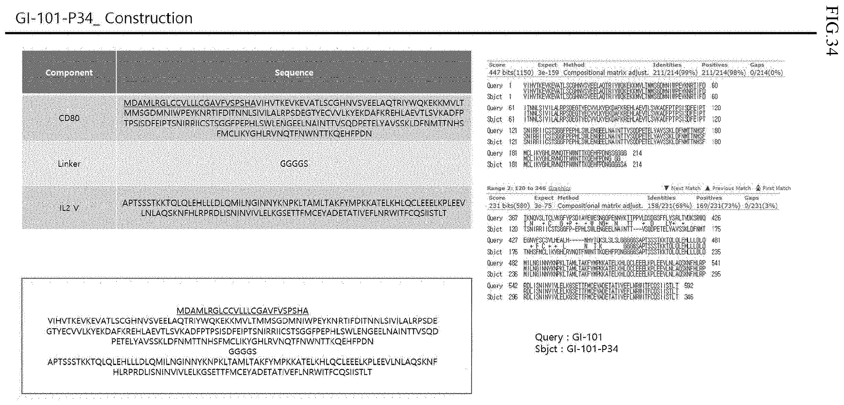

FIG. 34 illustrates components of GI-101_P34 and peptide sequences thereof (CD80: SEQ ID NO: 74, Linker: SEQ ID NO: 104, IL2V: SEQ ID NO: 6, GI101 P34: SEQ ID NO: 105).

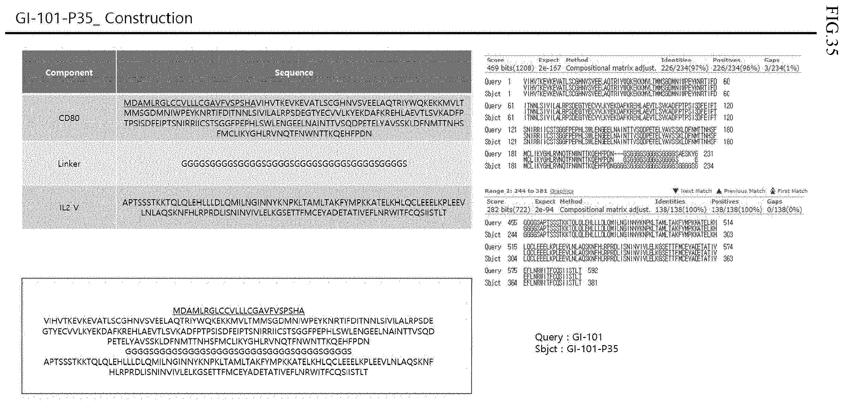

FIG. 35 illustrates components of GI-101_P35 and peptide sequences thereof (CD80: SEQ ID NO: 74, Linker: SEQ ID NO: 106, IL2V: SEQ ID NO: 6, GI101 P35: SEQ ID NO: 107).

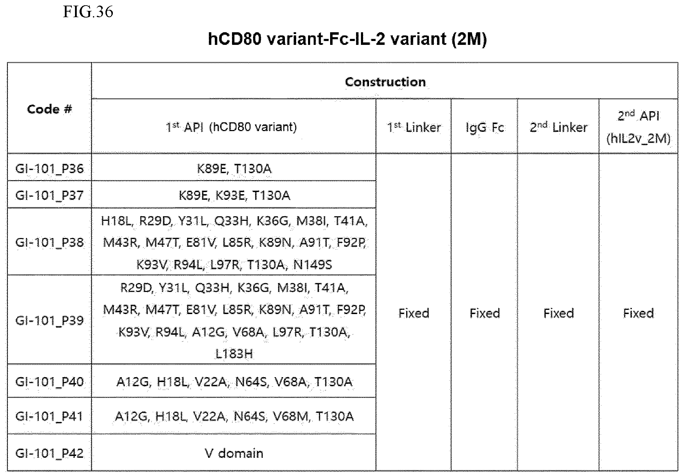

FIG. 36 illustrates GI-101_P36 to GI-101_P42 including variants of CD80 and IL-2 comprising two variations.

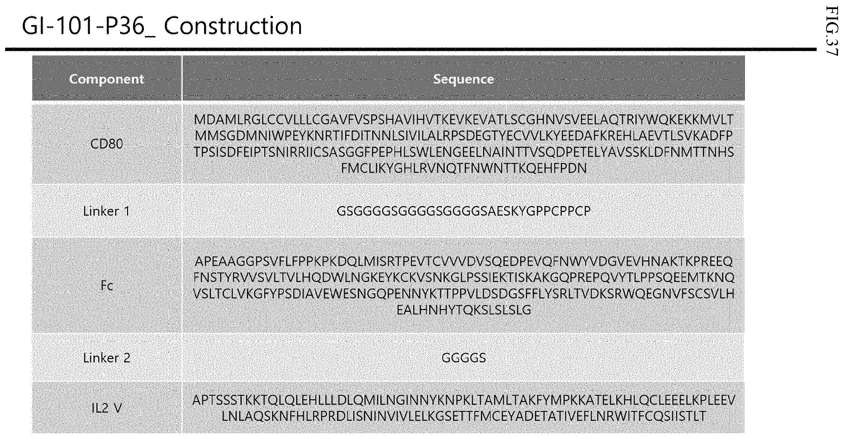

FIG. 37 illustrates components of GI-101_P36 and peptide sequences thereof (CD80: SEQ ID NO: 108, Linker 1: SEQ ID NO: 3, Fc: SEQ ID NO: 4, Linker 2: SEQ ID NO: 5, IL2V: SEQ ID NO: 6).

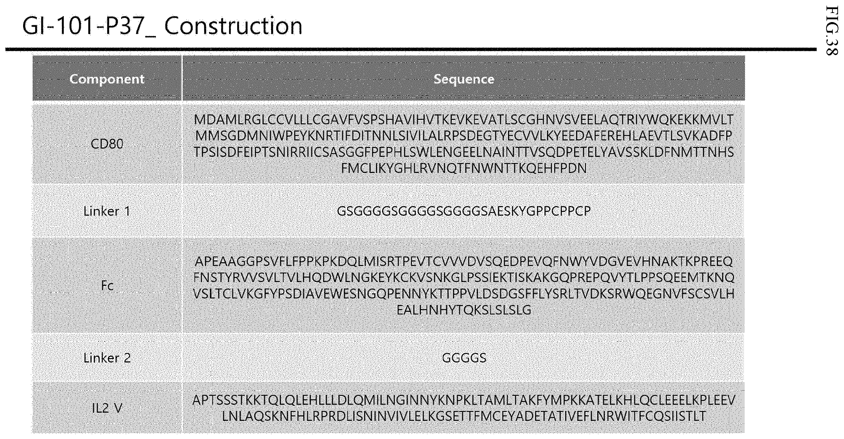

FIG. 38 illustrates components of GI-101_P37 and peptide sequences thereof (CD80: SEQ ID NO: 109, Linker 1: SEQ ID NO: 3, Fc: SEQ ID NO: 4, Linker 2: SEQ ID NO: 5, IL2V: SEQ ID NO: 6).

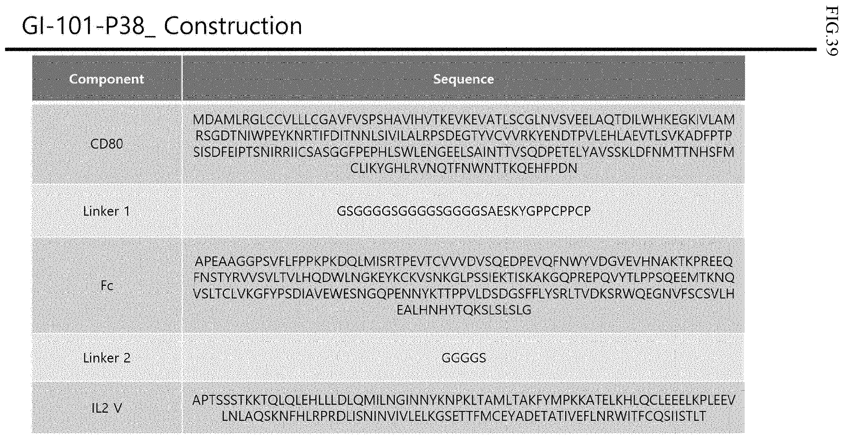

FIG. 39 illustrates components of GI-101_P38 and peptide sequences thereof (CD80: SEQ ID NO: 110, Linker 1: SEQ ID NO: 3, Fc: SEQ ID NO: 4, Linker 2: SEQ ID NO: 5, IL2V: SEQ ID NO: 6).

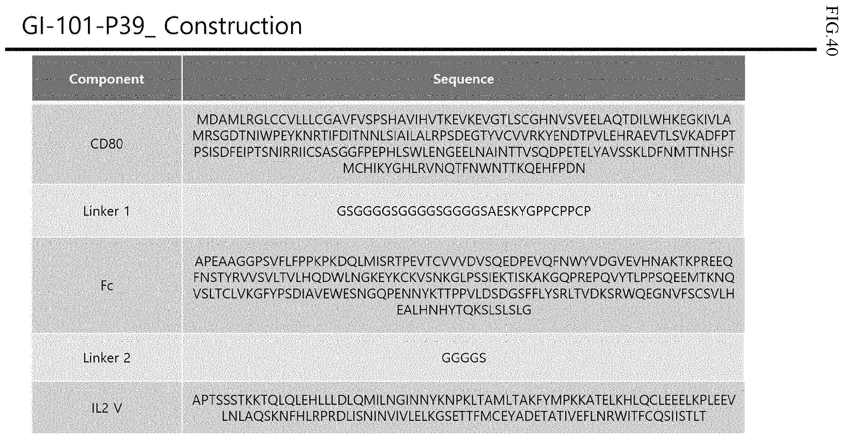

FIG. 40 illustrates components of GI-101_P39 and peptide sequences thereof (CD80: SEQ ID NO: 111, Linker 1: SEQ ID NO: 3, Fc: SEQ ID NO: 4, Linker 2: SEQ ID NO: 5, IL2V: SEQ ID NO: 6).

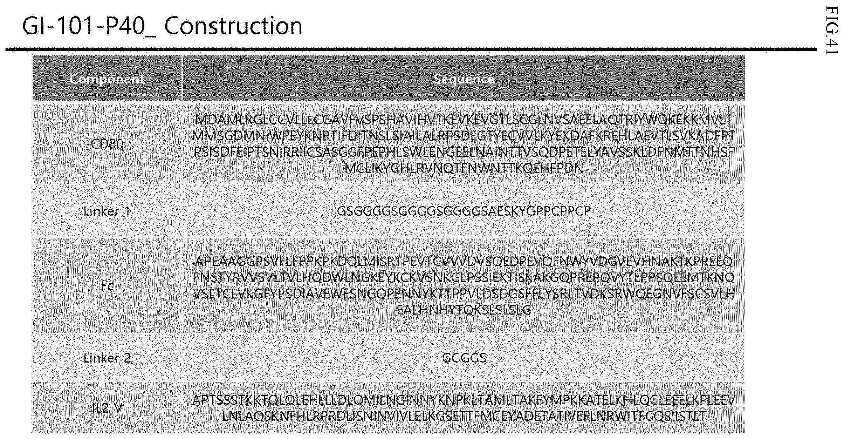

FIG. 41 illustrates components of GI-101_P40 and peptide sequences thereof (CD80: SEQ ID NO: 112, Linker 1: SEQ ID NO: 3, Fc: SEQ ID NO: 4, Linker 2: SEQ ID NO: 5, IL2V: SEQ ID NO: 6).

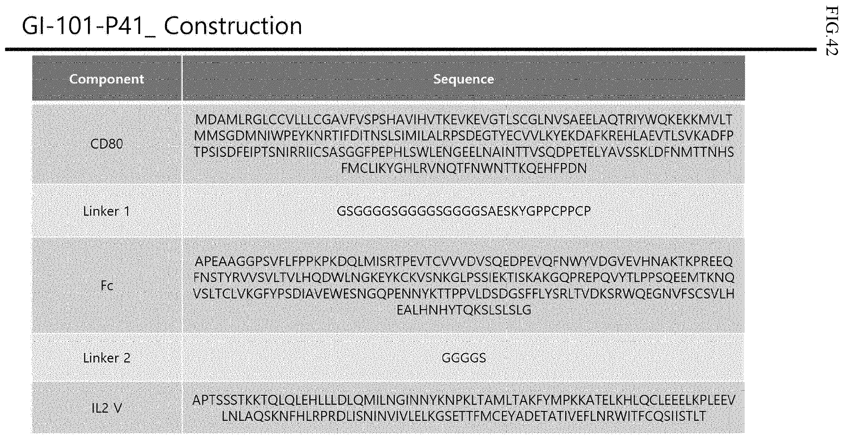

FIG. 42 illustrates components of GI-101_P41 and peptide sequences thereof (CD80: SEQ ID NO: 113, Linker 1: SEQ ID NO: 3, Fc: SEQ ID NO: 4, Linker 2: SEQ ID NO: 5, IL2V: SEQ ID NO: 6).

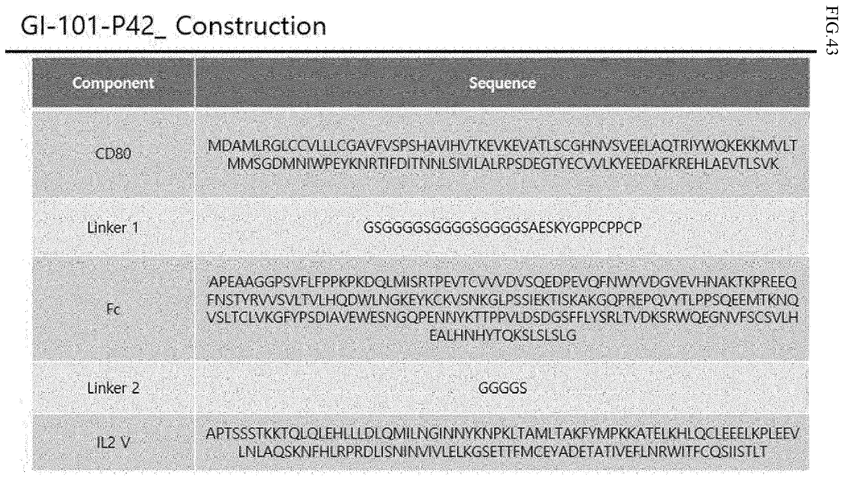

FIG. 43 illustrates components of GI-101_P42 and peptide sequences thereof (CD80: SEQ ID NO: 114, Linker 1: SEQ ID NO: 3, Fc: SEQ ID NO: 4, Linker 2: SEQ ID NO: 5, IL2V: SEQ ID NO: 6).

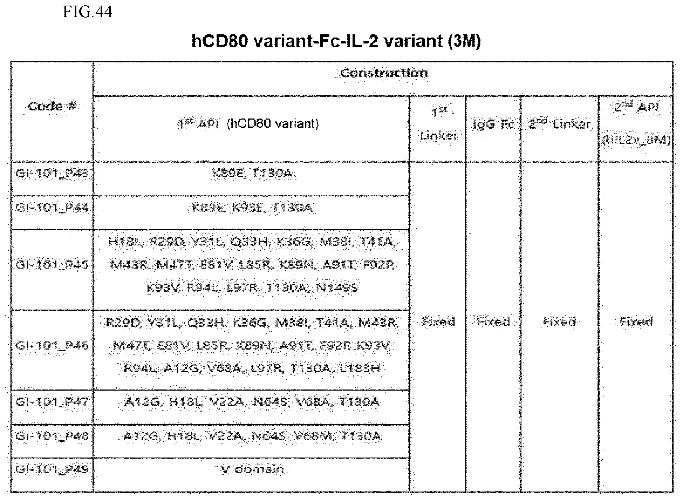

FIG. 44 illustrates GI-101_P43 to GI-101_P49 including variants of CD80 and IL-2 comprising three variations.

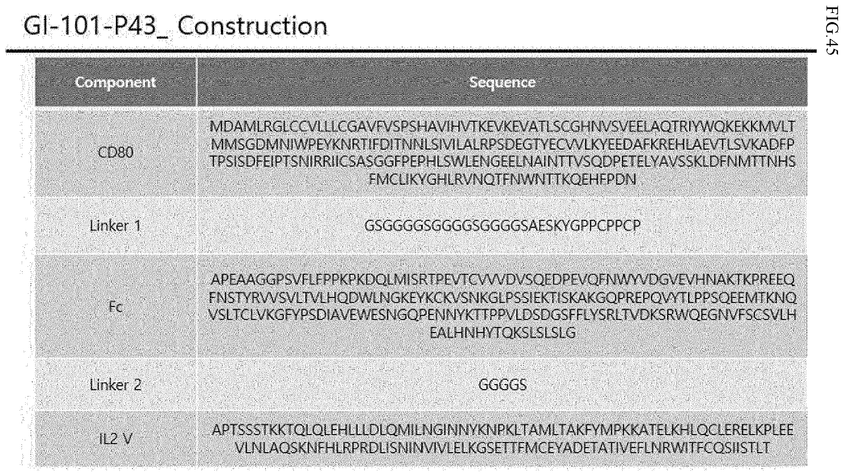

FIG. 45 illustrates components of GI-101_P43 and peptide sequences thereof (CD80: SEQ ID NO: 108, Linker 1: SEQ ID NO: 3, Fc: SEQ ID NO: 4, Linker 2: SEQ ID NO: 5, IL2V: SEQ ID NO: 115).

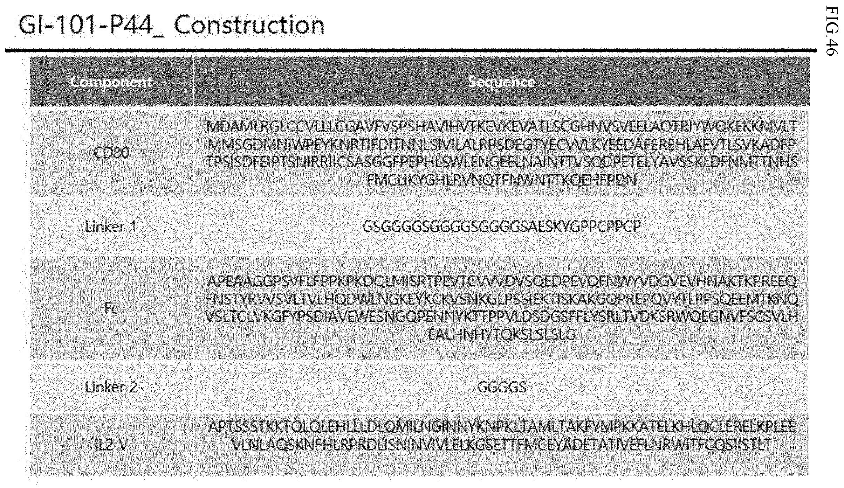

FIG. 46 illustrates components of GI-101_P44 and peptide sequences thereof (CD80: SEQ ID NO: 109, Linker 1: SEQ ID NO: 3, Fc: SEQ ID NO: 4, Linker 2: SEQ ID NO: 5, IL2V: SEQ ID NO: 115).

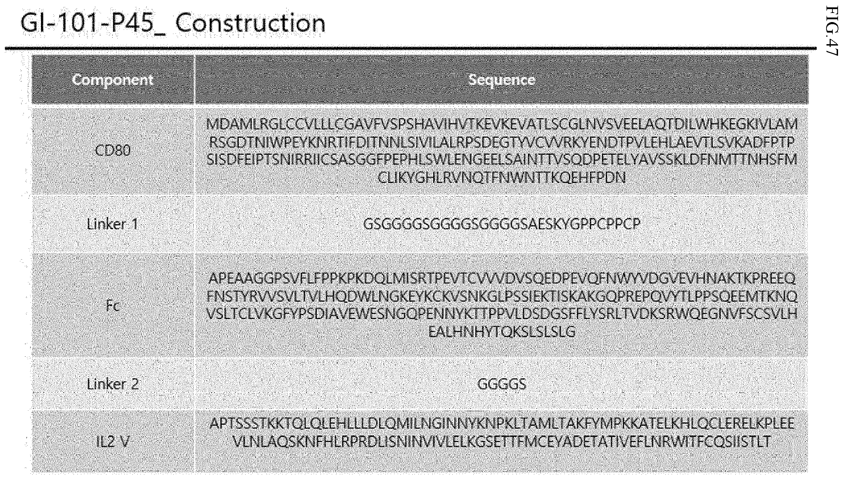

FIG. 47 illustrates components of GI-101_P45 and peptide sequences thereof (CD80: SEQ ID NO: 110, Linker 1: SEQ ID NO: 3, Fc: SEQ ID NO: 4, Linker 2: SEQ ID NO: 5, IL2V: SEQ ID NO: 115).

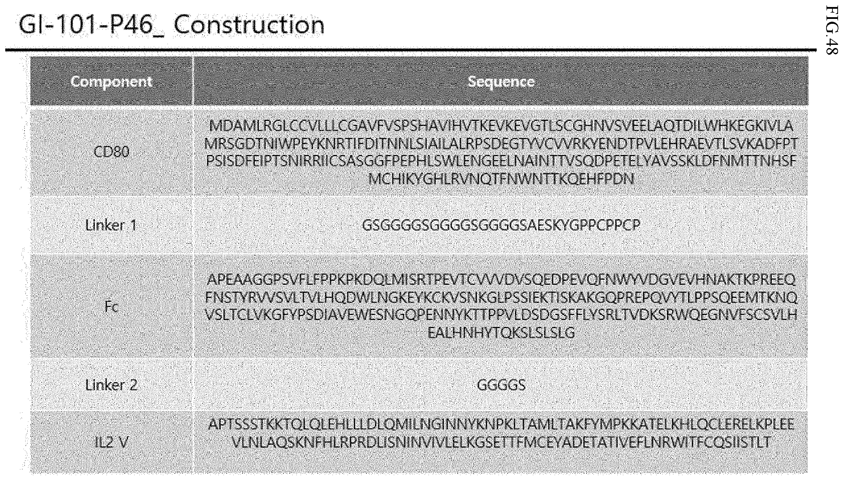

FIG. 48 illustrates components of GI-101_P46 and peptide sequences thereof (CD80: SEQ ID NO: 111, Linker 1: SEQ ID NO: 3, Fc: SEQ ID NO: 4, Linker 2: SEQ ID NO: 5, IL2V: SEQ ID NO: 115).

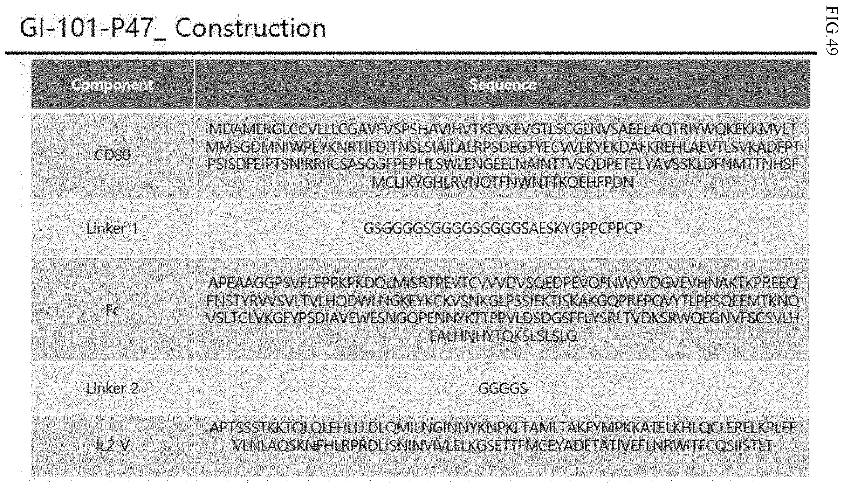

FIG. 49 illustrates components of GI-101_P47 and peptide sequences thereof (CD80: SEQ ID NO: 112, Linker 1: SEQ ID NO: 3, Fc: SEQ ID NO: 4, Linker 2: SEQ ID NO: 5, IL2V: SEQ ID NO: 115).

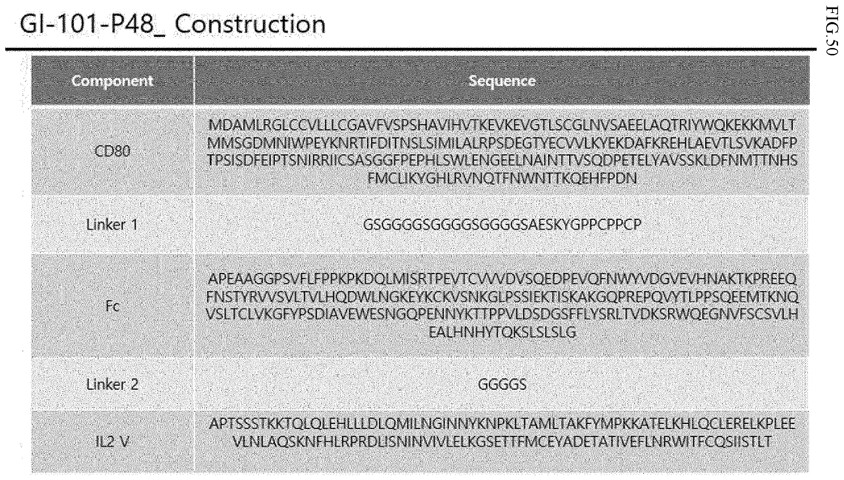

FIG. 50 illustrates components of GI-101_P48 and peptide sequences thereof (CD80: SEQ ID NO: 113, Linker 1: SEQ ID NO: 3, Fc: SEQ ID NO: 4, Linker 2: SEQ ID NO: 5, IL2V: SEQ ID NO: 115).

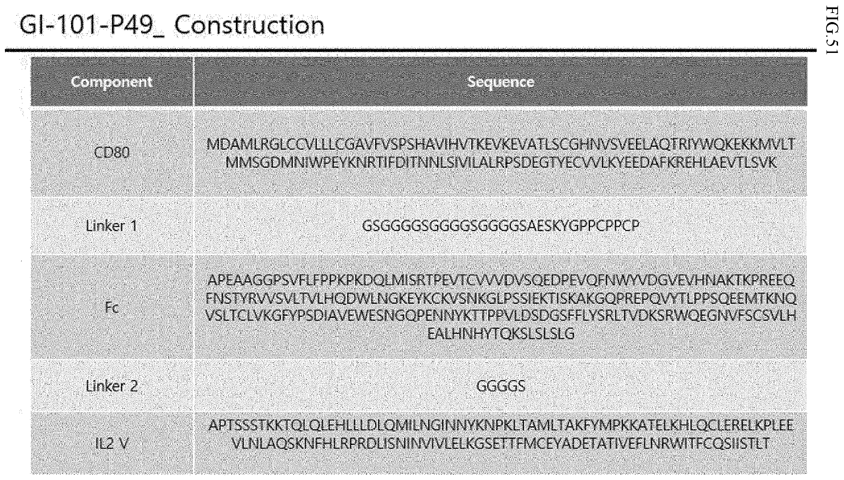

FIG. 51 illustrates components of GI-101_P49 and peptide sequences thereof (CD80: SEQ ID NO: 114, Linker 1: SEQ ID NO: 3, Fc: SEQ ID NO: 4, Linker 2: SEQ ID NO: 5, IL2V: SEQ ID NO: 115).
FIG. 52 illustrates a result obtained by identifying the GI-101_P1 fusion protein obtained according to an embodiment of the present invention with SDS-PAGE.
FIG. 53 illustrates a result obtained by identifying the GI-101_P2 fusion protein obtained according to an embodiment of the present invention with SDS-PAGE.
FIG. 54 illustrates a result obtained by identifying the GI-101_P3 fusion protein obtained according to an embodiment of the present invention with SDS-PAGE.
FIG. 55 illustrates a result obtained by identifying the GI-101_P4 fusion protein obtained according to an embodiment of the present invention with SDS-PAGE.
FIG. 56 illustrates a result obtained by identifying the GI-101_P5 fusion protein obtained according to an embodiment of the present invention with SDS-PAGE.
FIG. 57 illustrates a result obtained by identifying the GI-101_P6 fusion protein obtained according to an embodiment of the present invention with SDS-PAGE.
FIG. 58 illustrates a result obtained by identifying the GI-101_P7 fusion protein obtained according to an embodiment of the present invention with SDS-PAGE.
FIG. 59 illustrates a result obtained by identifying the GI-101_P8 fusion protein obtained according to an embodiment of the present invention with SDS-PAGE.
FIG. 60 illustrates a result obtained by identifying the GI-101_P9 fusion protein obtained according to an embodiment of the present invention with SDS-PAGE.
FIG. 61 illustrates a result obtained by identifying the GI-101_P10 fusion protein obtained according to an embodiment of the present invention with SDS-PAGE.
FIG. 62 illustrates a result obtained by identifying the GI-101_P11 fusion protein obtained according to an embodiment of the present invention with SDS-PAGE.
FIG. 63 illustrates a result obtained by identifying the GI-101_P12 fusion protein obtained according to an embodiment of the present invention with SDS-PAGE.
FIG. 64 illustrates a result obtained by identifying the GI-101_P13 fusion protein obtained according to an embodiment of the present invention with SDS-PAGE.
FIG. 65 illustrates a result obtained by identifying the GI-101_P14 fusion protein obtained according to an embodiment of the present invention with SDS-PAGE.
FIG. 66 illustrates a result obtained by identifying the GI-101_P15 fusion protein obtained according to an embodiment of the present invention with SDS-PAGE.
FIG. 67 illustrates a result obtained by identifying the GI-101_P16 fusion protein obtained according to an embodiment of the present invention with SDS-PAGE.
FIG. 68 illustrates a result obtained by identifying the GI-101_P17 fusion protein obtained according to an embodiment of the present invention with SDS-PAGE.
FIG. 69 illustrates a result obtained by identifying the GI-101_P18 fusion protein obtained according to an embodiment of the present invention with SDS-PAGE.
FIG. 70 illustrates a result obtained by identifying the GI-101_P19 fusion protein obtained according to an embodiment of the present invention with SDS-PAGE.
FIG. 71 illustrates a result obtained by identifying the GI-101_P20 fusion protein obtained according to an embodiment of the present invention with SDS-PAGE.
FIG. 72 illustrates a result obtained by identifying the GI-101_P21 fusion protein obtained according to an embodiment of the present invention with SDS-PAGE.
FIG. 73 illustrates a result obtained by identifying the GI-101_P22 fusion protein obtained according to an embodiment of the present invention with SDS-PAGE.
FIG. 74 illustrates a result obtained by identifying the GI-101_P23 fusion protein obtained according to an embodiment of the present invention with SDS-PAGE.
FIG. 75 illustrates a result obtained by identifying the GI-101_P24 fusion protein obtained according to an embodiment of the present invention with SDS-PAGE.
FIG. 76 illustrates a result obtained by identifying the GI-101_P25 fusion protein obtained according to an embodiment of the present invention with SDS-PAGE.
FIG. 77 illustrates a result obtained by identifying the GI-101_P26 fusion protein obtained according to an embodiment of the present invention with SDS-PAGE.
FIG. 78 illustrates a result obtained by identifying the GI-101_P27 fusion protein obtained according to an embodiment of the present invention with SDS-PAGE.
FIG. 79 illustrates a result obtained by identifying the GI-101_P28 fusion protein obtained according to an embodiment of the present invention with SDS-PAGE.
FIG. 80 illustrates a result obtained by identifying the GI-101_P29 fusion protein obtained according to an embodiment of the present invention with SDS-PAGE.
FIG. 81 illustrates a result obtained by identifying the GI-101_P30 fusion protein obtained according to an embodiment of the present invention with SDS-PAGE.
FIG. 82 illustrates a result obtained by identifying the GI-101_P31 fusion protein obtained according to an embodiment of the present invention with SDS-PAGE.
FIG. 83 illustrates a result obtained by identifying the GI-101_P32 fusion protein obtained according to an embodiment of the present invention with SDS-PAGE.
FIG. 84 illustrates a result obtained by identifying the GI-101_P33 fusion protein obtained according to an embodiment of the present invention with SDS-PAGE.
FIG. 85 illustrates a result obtained by identifying the GI-101_P34 fusion protein obtained according to an embodiment of the present invention with SDS-PAGE.
FIG. 86 illustrates a result obtained by identifying the GI-101_P35 fusion protein obtained according to an embodiment of the present invention with SDS-PAGE.
FIG. 87 illustrates a result obtained by analyzing the GI-101_P1 fusion protein obtained according to an embodiment of the present invention by size exclusion chromatography (SEC).
FIG. 88 illustrates a result obtained by analyzing the GI-101_P2 fusion protein obtained according to an embodiment of the present invention by size exclusion chromatography (SEC).
FIG. 89 illustrates a result obtained by analyzing the GI-101_P3 fusion protein obtained according to an embodiment of the present invention by size exclusion chromatography (SEC).
FIG. 90 illustrates a result obtained by analyzing the GI-101_P4 fusion protein obtained according to an embodiment of the present invention by size exclusion chromatography (SEC).
FIG. 91 illustrates a result obtained by analyzing the GI-101_P5 fusion protein obtained according to an embodiment of the present invention by size exclusion chromatography (SEC).
FIG. 92 illustrates a result obtained by analyzing the GI-101_P6 fusion protein obtained according to an embodiment of the present invention by size exclusion chromatography (SEC).
FIG. 93 illustrates a result obtained by analyzing the GI-101_P7 fusion protein obtained according to an embodiment of the present invention by size exclusion chromatography (SEC).
FIG. 94 illustrates a result obtained by analyzing the GI-101_P8 fusion protein obtained according to an embodiment of the present invention by size exclusion chromatography (SEC).
FIG. 95 illustrates a result obtained by analyzing the GI-101_P9 fusion protein obtained according to an embodiment of the present invention by size exclusion chromatography (SEC).
FIG. 96 illustrates a result obtained by analyzing the GI-101_P10 fusion protein obtained according to an embodiment of the present invention by size exclusion chromatography (SEC).
FIG. 97 illustrates a result obtained by analyzing the GI-101_P11 fusion protein obtained according to an embodiment of the present invention by size exclusion chromatography (SEC).
FIG. 98 illustrates a result obtained by analyzing the GI-101_P12 fusion protein obtained according to an embodiment of the present invention by size exclusion chromatography (SEC).
FIG. 99 illustrates a result obtained by analyzing the GI-101_P13 fusion protein obtained according to an embodiment of the present invention by size exclusion chromatography (SEC).
FIG. 100 illustrates a result obtained by analyzing the GI-101_P14 fusion protein obtained according to an embodiment of the present invention by size exclusion chromatography (SEC).
FIG. 101 illustrates a result obtained by analyzing the GI-101_P15 fusion protein obtained according to an embodiment of the present invention by size exclusion chromatography (SEC).
FIG. 102 illustrates a result obtained by analyzing the GI-101_P16 fusion protein obtained according to an embodiment of the present invention by size exclusion chromatography (SEC).
FIG. 103 illustrates a result obtained by analyzing the GI-101_P17 fusion protein obtained according to an embodiment of the present invention by size exclusion chromatography (SEC).
FIG. 104 illustrates a result obtained by analyzing the GI-101_P18 fusion protein obtained according to an embodiment of the present invention by size exclusion chromatography (SEC).
FIG. 105 illustrates a result obtained by analyzing the GI-101_P19 fusion protein obtained according to an embodiment of the present invention by size exclusion chromatography (SEC).
FIG. 106 illustrates a result obtained by analyzing the GI-101_P20 fusion protein obtained according to an embodiment of the present invention by size exclusion chromatography (SEC).
FIG. 107 illustrates a result obtained by analyzing the GI-101_P21 fusion protein obtained according to an embodiment of the present invention by size exclusion chromatography (SEC).
FIG. 108 illustrates a result obtained by analyzing the GI-101_P22 fusion protein obtained according to an embodiment of the present invention by size exclusion chromatography (SEC).
FIG. 109 illustrates a result obtained by analyzing the GI-101_P23 fusion protein obtained according to an embodiment of the present invention by size exclusion chromatography (SEC).
FIG. 110 illustrates a result obtained by analyzing the GI-101_P24 fusion protein obtained according to an embodiment of the present invention by size exclusion chromatography (SEC).
FIG. 111 illustrates a result obtained by analyzing the GI-101_P25 fusion protein obtained according to an embodiment of the present invention by size exclusion chromatography (SEC).
FIG. 112 illustrates a result obtained by analyzing the GI-101_P26 fusion protein obtained according to an embodiment of the present invention by size exclusion chromatography (SEC).
FIG. 113 illustrates a result obtained by analyzing the GI-101_P27 fusion protein obtained according to an embodiment of the present invention by size exclusion chromatography (SEC).
FIG. 114 illustrates a result obtained by analyzing the GI-101_P28 fusion protein obtained according to an embodiment of the present invention by size exclusion chromatography (SEC).
FIG. 115 illustrates a result obtained by analyzing the GI-101_P29 fusion protein obtained according to an embodiment of the present invention by size exclusion chromatography (SEC).
FIG. 116 illustrates a result obtained by analyzing the GI-101_P30 fusion protein obtained according to an embodiment of the present invention by size exclusion chromatography (SEC).
FIG. 117 illustrates a result obtained by analyzing the GI-101 P31 fusion protein obtained according to an embodiment of the present invention by size exclusion chromatography (SEC).
FIG. 118 illustrates a result obtained by analyzing the GI-101_P32 fusion protein obtained according to an embodiment of the present invention by size exclusion chromatography (SEC).
FIG. 119 illustrates a result obtained by analyzing the GI-101_P33 fusion protein obtained according to an embodiment of the present invention by size exclusion chromatography (SEC).
FIG. 120 illustrates a result obtained by analyzing the GI-101_P34 fusion protein obtained according to an embodiment of the present invention by size exclusion chromatography (SEC).
FIG. 121 illustrates a result obtained by analyzing the GI-101_P35 fusion protein obtained according to an embodiment of the present invention by size exclusion chromatography (SEC).
FIG. 122 illustrates binding affinity between the GI-101_P1 fusion protein obtained according to an embodiment of the present invention, and CTLA-4 or IL-2Rβ.
FIG. 123 illustrates binding affinity between the GI-101_P2 fusion protein obtained according to an embodiment of the present invention, and CTLA-4 or IL-2Rβ.
FIG. 124 illustrates binding affinity between the GI-101_P3 fusion protein obtained according to an embodiment of the present invention, and CTLA-4or IL-2Rβ.
FIG. 125 illustrates binding affinity between the GI-101_P4 fusion protein obtained according to an embodiment of the present invention, and CTLA-4 or IL-2Rβ.
FIG. 126 illustrates binding affinity between the GI-101_P5 fusion protein obtained according to an embodiment of the present invention, and CTLA-4 or IL-2Rβ.
FIG. 127 illustrates binding affinity between the GI-101_P6 fusion protein obtained according to an embodiment of the present invention, and CTLA-4 or IL-2Rβ.
FIG. 128 illustrates binding affinity between the GI-101_P7 fusion protein obtained according to an embodiment of the present invention, and CTLA-4 or IL-2Rβ.
FIG. 129 illustrates binding affinity between the GI-101_P8 fusion protein obtained according to an embodiment of the present invention, and CTLA-4 or IL-2Rβ.
FIG. 130 illustrates binding affinity between the GI-101_P9 fusion protein obtained according to an embodiment of the present invention, and CTLA-4 or IL-2Rβ.
FIG. 131 illustrates binding affinity between the GI-101_P10 fusion protein obtained according to an embodiment of the present invention, and CTLA-4 or IL-2Rβ.
FIG. 132 illustrates binding affinity between the GI-101_P11 fusion protein obtained according to an embodiment of the present invention, and CTLA-4 or IL-2Rβ.
FIG. 133 illustrates binding affinity between the GI-101_P12 fusion protein obtained according to an embodiment of the present invention, and CTLA-4 or IL-2Rβ.
FIG. 134 illustrates binding affinity between the GI-101_P13 fusion protein obtained according to an embodiment of the present invention, and CTLA-4 or IL-2Rβ.
FIG. 135 illustrates binding affinity between the GI-101_P14 fusion protein obtained according to an embodiment of the present invention, and CTLA-4 or IL-2Rβ.
FIG. 136 illustrates binding affinity between the GI-101_P15 fusion protein obtained according to an embodiment of the present invention, and CTLA-4 or IL-2Rβ.
FIG. 137 illustrates binding affinity between the GI-101_P16 fusion protein obtained according to an embodiment of the present invention, and CTLA-4 or IL-2Rβ.
FIG. 138 illustrates binding affinity between the GI-101_P17 fusion protein obtained according to an embodiment of the present invention, and CTLA-4 or IL-2Rβ.
FIG. 139 illustrates binding affinity between the GI-101_P18 fusion protein obtained according to an embodiment of the present invention, and CTLA-4 or IL-2Rβ.
FIG. 140 illustrates binding affinity between the GI-101_P19 fusion protein obtained according to an embodiment of the present invention, and CTLA-4 or IL-2Rβ.
FIG. 141 illustrates binding affinity between the GI-101_P20 fusion protein obtained according to an embodiment of the present invention, and CTLA-4 or IL-2Rβ.
FIG. 142 illustrates binding affinity between the GI-101_P21 fusion protein obtained according to an embodiment of the present invention, and CTLA-4 or IL-2Rβ.
FIG. 143 illustrates binding affinity between the GI-101_P22 fusion protein obtained according to an embodiment of the present invention, and CTLA-4 or IL-2Rβ.
FIG. 144 illustrates binding affinity between the GI-101_P23 fusion protein obtained according to an embodiment of the present invention, and CTLA-4 or IL-2Rβ.
FIG. 145 illustrates binding affinity between the GI-101_P24 fusion protein obtained according to an embodiment of the present invention, and CTLA-4 or IL-2Rβ.
FIG. 146 illustrates binding affinity between the GI-101_P25 fusion protein obtained according to an embodiment of the present invention, and CTLA-4 or IL-2Rβ.
FIG. 147 illustrates binding affinity between the GI-101_P26 fusion protein obtained according to an embodiment of the present invention, and CTLA-4 or IL-2Rβ.
FIG. 148 illustrates binding affinity between the GI-101_P27 fusion protein obtained according to an embodiment of the present invention, and CTLA-4 or IL-2Rβ.
FIG. 149 illustrates binding affinity between the GI-101_P28 fusion protein obtained according to an embodiment of the present invention, and CTLA-4 or IL-2Rβ.
FIG. 150 illustrates binding affinity between the GI-101_P29 fusion protein obtained according to an embodiment of the present invention, and CTLA-4 or IL-2Rβ.
FIG. 151 illustrates binding affinity between the GI-101_P30 fusion protein obtained according to an embodiment of the present invention, and CTLA-4 or IL-2Rβ.
FIG. 152 illustrates binding affinity between the GI-101_P31 fusion protein obtained according to an embodiment of the present invention, and CTLA-4 or IL-2Rβ.
FIG. 153 illustrates binding affinity between the GI-101_P32 fusion protein obtained according to an embodiment of the present invention, and CTLA-4 or IL-2Rβ.
FIG. 154 illustrates binding affinity between the GI-101_P33 fusion protein obtained according to an embodiment of the present invention, and CTLA-4 or IL-2Rβ.
FIG. 155 illustrates binding affinity between the GI-101_P34 fusion protein obtained according to an embodiment of the present invention, and CTLA-4 or IL-2Rβ.
FIG. 156 illustrates binding affinity between the GI-101_P35 fusion protein obtained according to an embodiment of the present invention, and CTLA-4 or IL-2Rβ.
FIG. 157 illustrates a result obtained by identifying the GI-101_P36 to GI-101_P49 fusion proteins obtained according to an embodiment of the present invention with SDS-PAGE.
FIG. 158 illustrates binding affinity between the GI-101_P36 fusion protein obtained according to an embodiment of the present invention, and IL-2Rβ, CTLA-4 or PD-L1.
FIG. 159 illustrates binding affinity between the GI-101_P37 fusion protein obtained according to an embodiment of the present invention, and IL-2Rβ, CTLA-4 or PD-L1.
FIG. 160 illustrates binding affinity between the GI-101_P38 fusion protein obtained according to an embodiment of the present invention, and IL-2Rβ, CTLA-4 or PD-L1.
FIG. 161 illustrates binding affinity between the GI-101_P39 fusion protein obtained according to an embodiment of the present invention, and IL-2Rβ, CTLA-4 or PD-L1.
FIG. 162 illustrates binding affinity between the GI-101_P40 fusion protein obtained according to an embodiment of the present invention, and IL-2Rβ, CTLA-4 or PD-L1.
FIG. 163 illustrates binding affinity between the GI-101_P41 fusion protein obtained according to an embodiment of the present invention, and IL-2Rβ, CTLA-4 or PD-L1.
FIG. 164 illustrates binding affinity between the GI-101_P42 fusion protein obtained according to an embodiment of the present invention, and IL-2Rβ, CTLA-4 or PD-L1.
FIG. 165 illustrates binding affinity between the GI-101_P43 fusion protein obtained according to an embodiment of the present invention, and IL-2Rβ, CTLA-4 or PD-L1.
FIG. 166 illustrates binding affinity between the GI-101_P44 fusion protein obtained according to an embodiment of the present invention, and IL-2Rβ, CTLA-4 or PD-L1.
FIG. 167 illustrates binding affinity between the GI-101_P45 fusion protein obtained according to an embodiment of the present invention, and IL-2Rβ, CTLA-4 or PD-L1.
FIG. 168 illustrates binding affinity between the GI-101_P46 fusion protein obtained according to an embodiment of the present invention, and IL-2Rβ, CTLA-4 or PD-L1.
FIG. 169 illustrates binding affinity between the GI-101_P47 fusion protein obtained according to an embodiment of the present invention, and IL-2Rβ, CTLA-4 or PD-L1.
FIG. 170 illustrates binding affinity between the GI-101_P49 fusion protein obtained according to an embodiment of the present invention, and IL-2Rβ, CTLA-4 or PD-L1.

### Best Mode for Carrying out the Invention

### Fusion protein comprising IL-2 protein and CD80 protein fragment or variant thereof

In an aspect of the present invention, there is provided a fusion protein comprising an IL-2 protein and a CD80 protein fragment or a variant thereof.

### IL-2 protein

As used herein, the term "IL-2" or "interleukin-2", unless otherwise stated, refers to any wild-type IL-2 obtained from any vertebrate source, including mammals, for example, primates (such as humans) and rodents (such as mice and rats). IL-2 may be obtained from animal cells, and also includes one obtained from recombinant cells capable of producing IL-2. In addition, IL-2 may be wild-type IL-2 or a variant thereof.

In the present specification, IL-2 or a variant thereof may be collectively expressed by the term "IL-2 protein" or "IL-2 polypeptide". IL-2, an IL-2 protein, an IL-2 polypeptide, and an IL-2 variant specifically bind to, for example, an IL-2 receptor. This specific binding may be identified by methods known to those skilled in the art.

An embodiment of IL-2 may have the amino acid sequence of SEQ ID NO: 35 or SEQ ID NO: 36. Here, IL-2 may also be in a mature form. Specifically, the mature IL-2 may not contain a signal sequence, and may have the amino acid sequence of SEQ ID NO: 10. Here, IL-2 may be used under a concept encompassing a fragment of wild-type IL-2 in which a portion of N-terminus or C-terminus of the wild-type IL-2 is truncated.

In addition, the fragment of IL-2 may be in a form in which 1, 2, 3, 4, 5, 6, 7, 8, 9, 10, 11, 12, 13, 14, 15, 16, 17, 18, 19, 20, 21, 22, 23, 24, or 25 contiguous amino acids are truncated from N-terminus of a protein having the amino acid sequence of SEQ ID NO: 35 or SEQ ID NO: 36. In addition, the fragment of IL-2 may be in a form in which 1, 2, 3, 4, 5, 6, 7, 8, 9, 10, 11, 12, 13, 14, 15, 16, 17, 18, 19, 20, 21, 22, 23, 24, or 25 contiguous amino acids are truncated from C-terminus of a protein having the amino acid sequence of SEQ ID NO: 35 or SEQ ID NO: 36.

As used herein, the term "IL-2 variant" refers to a form in which a portion of amino acids in the full-length IL-2 or the above-described fragment of IL-2 is substituted. That is, an IL-2 variant may have an amino acid sequence different from wild-type IL-2 or a fragment thereof. However, an IL-2 variant may have activity equivalent or similar to the wild-type IL-2. Here, "IL-2 activity" may, for example, refer to specific binding to an IL-2 receptor, which specific binding can be measured by methods known to those skilled in the art.

Specifically, an IL-2 variant may be obtained by substitution of a portion of amino acids in the wild-type IL-2. An embodiment of the IL-2 variant obtained by amino acid substitution may be obtained by substitution of at least one of the 38^{th}, 42^{nd}, 45^{th}, 61^{st}, and 72^{nd} amino acids in the amino acid sequence of SEQ ID NO: 10.

Specifically, the IL-2 variant may be obtained by substitution of at least one of the 38^{th}, 42^{nd}, 45^{th}, 61^{st}, or 72^{nd} amino acid in the amino acid sequence of SEQ ID NO: 10 with another amino acid. In addition, when IL-2 is in a form in which a portion of N-terminus in the amino acid sequence of SEQ ID NO: 35 is truncated, the amino acid at a position complementarily corresponding to that in the amino acid sequence of SEQ ID NO: 10 may be substituted with another amino acid. For example, when IL-2 has the amino acid sequence of SEQ ID NO: 35, its IL-2 variant may be obtained by substitution of at least one of 58^{th}, 62^{nd}, 65^{th}, 81^{st}, or 92^{nd} amino acid in the amino acid sequence of SEQ ID NO: 35 with another amino acid. These amino acid residues correspond to the 38^{th}, 42^{nd}, 45^{th}, 61^{st}, and 72^{nd} amino acid residues in the amino acid sequence of SEQ ID NO: 10, respectively. According to an embodiment, one, two, three, four, five, six, seven, eight, nine, or ten amino acids may be substituted as long as such IL-2 variant maintains IL-2 activity. According to another embodiment, one to five amino acids may be substituted.

In an embodiment, an IL-2 variant may be in a form in which two amino acids are substituted. Specifically, the IL-2 variant may be obtained by substitution of the 38^{th} and 42^{nd} amino acids in the amino acid sequence of SEQ ID NO: 10. In addition, in an embodiment, the IL-2 variant may be obtained by substitution of the 38^{th} and 45^{th} amino acids in the amino acid sequence of SEQ ID NO: 10. In addition, in an embodiment, the IL-2 variant may be obtained by substitution of the 38^{th} and 61^{st} amino acids in the amino acid sequence of SEQ ID NO: 10. In addition, in an embodiment, the IL-2 variant may be obtained by substitution of the 38^{th} and 72^{nd} amino acids in the amino acid sequence of SEQ ID NO: 10. In addition, in an embodiment, the IL-2 variant may be obtained by substitution of the 42^{nd} and 45^{th} amino acids in the amino acid sequence of SEQ ID NO: 10. In addition, in an embodiment, the IL-2 variant may be obtained by substitution of the 42^{nd} and 61^{st} amino acids in the amino acid sequence of SEQ ID NO: 10. In addition, in an embodiment, the IL-2 variant may be obtained by substitution of the 42^{nd} and 72^{nd} amino acids in the amino acid sequence of SEQ ID NO: 10. In addition, in an embodiment, the IL-2 variant may be obtained by substitution of the 45^{th} and 61^{st} amino acids in the amino acid sequence of SEQ ID NO: 10. In addition, in an embodiment, the IL-2 variant may be obtained by substitution of the 45^{th} and 72^{nd} amino acids in the amino acid sequence of SEQ ID NO: 10. In addition, in an embodiment, the IL-2 variant may be obtained by substitution of the 61^{st} and 72^{nd} amino acids in the amino acid sequence of SEQ ID NO: 10.

Furthermore, an IL-2 variant may be in a form in which three amino acids are substituted. Specifically, the IL-2 variant may be obtained by substitution of the 38^{th}, 42^{nd}, and 45^{th} amino acids in the amino acid sequence of SEQ ID NO: 10. In addition, in an embodiment, the IL-2 variant may be obtained by substitution of the 38^{th}, 42^{nd}, and 61^{st} amino acids in the amino acid sequence of SEQ ID NO: 10. In addition, in an embodiment, the IL-2 variant may be obtained by substitution of the 38^{th}, 42^{nd}, and 72^{nd} amino acids in the amino acid sequence of SEQ ID NO: 10. In addition, in an embodiment, the IL-2 variant may be obtained by substitution of the 38^{th}, 45^{th}, and 61^{st} amino acids in the amino acid sequence of SEQ ID NO: 10. In addition, in an embodiment, the IL-2 variant may be obtained by substitution of the 38^{th}, 45^{th}, and 72^{nd} amino acids in the amino acid sequence of SEQ ID NO: 10. In addition, in an embodiment, the IL-2 variant may be obtained by substitution of the 38^{th}, 61^{st}, and 72^{nd} amino acids in the amino acid sequence of SEQ ID NO: 10. In addition, in an embodiment, the IL-2 variant may be obtained by substitution of the 42^{nd}, 45^{th}, and 61^{st} amino acids in the amino acid sequence of SEQ ID NO: 10. In addition, in an embodiment, the IL-2 variant may be obtained by substitution of the 42^{nd}, 45^{th}, and 72^{nd} amino acids in the amino acid sequence of SEQ ID NO: 10. In addition, in an embodiment, the IL-2 variant may be obtained by substitution of the 45^{th}, 61^{st}, and 72^{nd} amino acids in the amino acid sequence of SEQ ID NO: 10.

In addition, an IL-2 variant may be in a form in which four amino acids are substituted. Specifically, the IL-2 variant may be obtained by substitution of the 38^{th}, 42^{nd}, 45^{th}, and 61^{st} amino acids in the amino acid sequence of SEQ ID NO: 10. In addition, in an embodiment, the IL-2 variant may be obtained by substitution of the 38^{th}, 42^{nd}, 45^{th}, and 72^{nd} amino acids in the amino acid sequence of SEQ ID NO: 10. In addition, in an embodiment, the IL-2 variant may be obtained by substitution of the 38^{th}, 45^{th}, 61^{st}, and 72^{nd} amino acids in the amino acid sequence of SEQ ID NO: 10. In addition, in an embodiment, the IL-2 variant may be obtained by substitution of the 38^{th}, 42^{nd}, 61^{st}, and 72^{nd} amino acids in the amino acid sequence of SEQ ID NO: 10. In addition, in an embodiment, the IL-2 variant may be obtained by substitution of 42^{nd}, 45^{th}, 61^{st}, and 72^{nd} amino acids in the amino acid sequence of SEQ ID NO: 10.

Furthermore, an IL-2 variant may be in a form in which five amino acids are substituted. Specifically, the IL-2 variant may be obtained by substitution of each of the 38^{th}, 42^{nd}, 45^{th}, 61^{st}, and 72^{nd} amino acids in the amino acid sequence of SEQ ID NO: 10 with another amino acid.

Here, the "another amino acid" introduced by the substitution may be any one selected from the group consisting of alanine, arginine, asparagine, aspartic acid, cysteine, glutamic acid, glutamine, histidine, isoleucine, leucine, lysine, methionine, phenylalanine, proline, serine, threonine, tryptophan, tyrosine, and valine. However, regarding amino acid substitution for the IL-2 variant, in the amino acid sequence of SEQ ID NO: 10, the 38^{th} amino acid cannot be substituted with arginine, the 42^{nd} amino acid cannot be substituted with phenylalanine, the 45^{th} amino acid cannot be substituted with tyrosine, the 61^{st} amino acid cannot be substituted with glutamic acid, and the 72^{nd} amino acid cannot be substituted with leucine.

Regarding amino acid substitution for an IL-2 variant, in the amino acid sequence of SEQ ID NO: 10, the 38^{th} amino acid, arginine, may be substituted with an amino acid other than arginine. Preferably, regarding amino acid substitution for an IL-2 variant, in the amino acid sequence of SEQ ID NO: 10, the 38^{th} amino acid, arginine, may be substituted with alanine (R38A).

Regarding amino acid substitution for an IL-2 variant, in the amino acid sequence of SEQ ID NO: 10, the 42^{nd} amino acid, phenylalanine, may be substituted with an amino acid other than phenylalanine. Preferably, regarding amino acid substitution for an IL-2 variant, in the amino acid sequence of SEQ ID NO: 10, the 42^{nd} amino acid, phenylalanine, may be substituted with alanine (F42A).

Regarding amino acid substitution for an IL-2 variant, in the amino acid sequence of SEQ ID NO: 10, the 45^{th} amino acid, tyrosine, may be substituted with an amino acid other than tyrosine. Preferably, regarding amino acid substitution for an IL-2 variant, in the amino acid sequence of SEQ ID NO: 10, the 45^{th} amino acid, tyrosine, may be substituted with alanine (Y45A).

Regarding amino acid substitution for an IL-2 variant, in the amino acid sequence of SEQ ID NO: 10, the 61^{st} amino acid, glutamic acid, may be substituted with an amino acid other than glutamic acid. Preferably, regarding amino acid substitution for an IL-2 variant, in the amino acid sequence of SEQ ID NO: 10, the 61^{st} amino acid, glutamic acid, may be substituted with arginine (E61R).

Regarding amino acid substitution for an IL-2 variant, in the amino acid sequence of SEQ ID NO: 10, the 72^{nd} amino acid, leucine, may be substituted with an amino acid other than leucine. Preferably, regarding amino acid substitution for an IL-2 variant, in the amino acid sequence of SEQ ID NO: 10, the 72^{nd} amino acid, leucine, may be substituted with glycine (L72G).

Specifically, an IL-2 variant may be obtained by at least one substitution selected from the group consisting of R38A, F42A, Y45A, E61R, and L72G, in the amino acid sequence of SEQ ID NO: 10.

Specifically, an IL-2 variant may be obtained by amino acid substitutions at two, three, four, or five positions among the positions selected from the group consisting of R38A, F42A, Y45A, E61R, and L72G.

In addition, an IL-2 variant may be in a form in which two amino acids are substituted. Specifically, an IL-2 variant may be obtained by the substitutions, R38A and F42A. In addition, in an embodiment, an IL-2 variant may be obtained by the substitutions, R38A and Y45A. In addition, in an embodiment, an IL-2 variant may be obtained by the substitutions, R38A and E61R. In addition, in an embodiment, an IL-2 variant may be obtained by the substitutions, R38A and L72G. In addition, in an embodiment, an IL-2 variant may be obtained by the substitutions, F42A and Y45A. In addition, in an embodiment, an IL-2 variant may be obtained by the substitutions, F42A and E61R. In addition, in an embodiment, an IL-2 variant may be obtained by the substitutions, F42A and L72G. In addition, in an embodiment, an IL-2 variant may be obtained by the substitutions, E61R and L72G.

Furthermore, an IL-2 variant may be in a form in which three amino acids are substituted. Specifically, an IL-2 variant may be obtained by the substitutions, R38A, F42A, and Y45A. In addition, in an embodiment, an IL-2 variant may be obtained by the substitutions, R38A, F42A, and E61R. In addition, in an embodiment, an IL-2 variant may be obtained by the substitutions, R38A, F42A, and L72G. In addition, in an embodiment, an IL-2 variant may be obtained by the substitutions, R38A, Y45A, and E61R. In addition, in an embodiment, an IL-2 variant may be obtained by the substitutions, R38A, Y45A, and L72G. In addition, in an embodiment, an IL-2 variant may be obtained by the substitutions, F42A, Y45A, and E61R. In addition, in an embodiment, an IL-2 variant may be obtained by the substitutions, F42A, Y45A, and L72G. In addition, in an embodiment, an IL-2 variant may be obtained by the substitutions, F42A, E61R, and L72G. In addition, in an embodiment, an IL-2 variant may be obtained by the substitutions, Y45A, E61R, and L72G.

In addition, an IL-2 variant may be in a form in which four amino acids are substituted. Specifically, an IL-2 variant may be obtained by the substitutions, R38A, F42A, Y45A, and E61R. In addition, in an embodiment, an IL-2 variant may be obtained by the substitutions, R38A, F42A, Y45A, and L72G. In addition, in an embodiment, an IL-2 variant may be obtained by the substitutions, R38A, F42A, E61R, and L72G. In addition, in an embodiment, an IL-2 variant may be obtained by the substitutions, R38A, Y45A, E61R, and L72G. In addition, in an embodiment, an IL-2 variant may be obtained by the substitutions, F42A, Y45A, E61R, and L72G.

Furthermore, an IL-2 variant may be obtained by the substitutions, R38A, F42A, Y45A, E61R, and L72G.

Preferably, an embodiment of the IL-2 variant may contain which are any one selected from the following substitution combinations (a) to (d) in the amino acid sequence of SEQ ID NO: 10:
(a) R38A/F42A
(b) R38A/F42A/Y45A
(c) R38A/F42A/E61R
(d) R38A/F42A/L72G

Here, when IL-2 has the amino acid sequence of SEQ ID NO: 35, an amino acid substitution may be present at a position complementarily corresponding to that in the amino acid sequence of SEQ ID NO: 10. In addition, even when IL-2 is a fragment of the amino acid sequence of SEQ ID NO: 35, an amino acid substitution may be present at a position complementarily corresponding to that in the amino acid sequence of SEQ ID NO: 10.

Specifically, an IL-2 variant may have the amino acid sequence of SEQ ID NO: 6, 22, 23, or 24.

In addition, an IL-2 variant may be characterized by having low *in vivo* toxicity. Here, the low *in vivo* toxicity may be a side effect caused by binding of IL-2 to the IL-2 receptor alpha chain (IL-2Rα). Various IL-2 variants have been developed to ameliorate the side effect caused by binding of IL-2 to IL-2Rα, and such IL-2 variants may be those disclosed in US Patent No. 5,229,109 and Korean Patent No. 1667096. In particular, IL-2 variants described in the present application have low binding ability for the IL-2 receptor alpha chain (IL-2Rα) and thus have lower *in vivo* toxicity than the wild-type IL-2.

### CD80 protein fragment or variant thereof

As used herein, the term "CD80", also called "B7-1", is a membrane protein present in dendritic cells, activated B cells, and monocytes. CD80 provides co-stimulatory signals essential for activation and survival of T cells. CD80 is known as a ligand for the two different proteins, CD28 and CTLA-4, present on the surface of T cells. CD80 is composed of 288 amino acids, and may specifically have the amino acid sequence of SEQ ID NO: 11. In addition, as used herein, the term "CD80 protein" refers to the full-length CD80 or a CD80 fragment.

As used herein, the term "CD80 fragment" refers to a cleaved form of CD80. In addition, the CD80 fragment may be an extracellular domain of CD80. An embodiment of the CD80 fragment may be obtained by elimination of the 1^{st} to 34^{th} amino acids from N-terminus which are a signal sequence of CD80. Specifically, an embodiment of the CD80 fragment may be a protein consisting of the 35^{th} to 288^{th} amino acids in SEQ ID NO: 11. In addition, an embodiment of the CD80 fragment may be a protein consisting of the 35^{th} to 242^{nd} amino acids in SEQ ID NO: 11. In addition, an embodiment of the CD80 fragment may be a protein consisting of the 35^{th} to 232^{nd} amino acids in SEQ ID NO: 11. In addition, an embodiment of the CD80 fragment may be a protein consisting of the 35^{th} to 139^{th} amino acids in SEQ ID NO: 11. In addition, an embodiment of the CD80 fragment may be a protein consisting of the 142^{nd} to 242^{nd} amino acids in SEQ ID NO: 11. In an embodiment, a CD80 fragment may have the amino acid sequence of SEQ ID NO: 2.

In addition, an embodiment of the CD80 protein fragment may be a protein consisting of the 35^{th} to 231^{st} amino acids in SEQ ID NO: 11. In addition, an embodiment of the CD80 protein fragment may be a protein consisting of the 35^{th} to 224^{th} amino acids in SEQ ID NO: 11. In addition, an embodiment of the CD80 protein fragment may be a protein consisting of the 35^{th} to 219^{th} amino acids in SEQ ID NO: 11. In addition, an embodiment of the CD80 protein fragment may be a protein consisting of the 35^{th} to 211^{th} amino acids in SEQ ID NO: 11. In addition, an embodiment of the CD80 protein fragment may be a protein consisting of the 35^{th} to 208^{th} amino acids in SEQ ID NO: 11. In addition, an embodiment of the CD80 protein fragment may be a protein consisting of the 35^{th} to 196^{th} amino acids in SEQ ID NO: 11. In addition, an embodiment of the CD80 protein fragment may be a protein consisting of the 35^{th} to 192^{nd} amino acids in SEQ ID NO: 11. In addition, an embodiment of the CD80 protein fragment may be a protein consisting of the 35^{th} to 184^{th} amino acids in SEQ ID NO: 11. In addition, an embodiment of the CD80 protein fragment may be a protein consisting of the 35^{th} to 145^{th} amino acids in SEQ ID NO: 11. In addition, an embodiment of the CD80 protein fragment may be a protein consisting of the 41^{st} to 145^{th} amino acids in SEQ ID NO: 11. In addition, an embodiment of the CD80 protein fragment may be a protein consisting of the 46^{th} to 145^{th} amino acids in SEQ ID NO: 11. In addition, an embodiment of the CD80 protein fragment may be a protein consisting of the 55^{th} to 145^{th} amino acids in SEQ ID NO: 11. In addition, an embodiment of the CD80 protein fragment may be a protein consisting of the 61^{st} to 145^{th} amino acids in SEQ ID NO: 11. In addition, an embodiment of the CD80 protein fragment may be a protein consisting of the 61^{st} to 138^{th} amino acids in SEQ ID NO: 11. In addition, an embodiment of the CD80 protein fragment may be a protein consisting of the 78^{th} to 145^{th} amino acids in SEQ ID NO: 11. In addition, an embodiment of the CD80 protein fragment may be a protein consisting of the 61^{st} to 84^{th} amino acids in SEQ ID NO: 11. In addition, an embodiment of the CD80 protein fragment may be a protein consisting of the 61^{st} to 77^{th} amino acids in SEQ ID NO: 11. In addition, an embodiment of the CD80 protein fragment may be a protein consisting of the 112^{th} to 138^{th} amino acids in SEQ ID NO: 11.

In addition, the variant of the CD80 protein fragment may be a variant CD80 polypeptide including an IgV domain or a fragment thereof, an IgC domain or a fragment thereof, or both. Specifically, the variant of the CD80 protein fragment means that one or more of the amino acids constituting it have been substituted with another amino acid. Specifically, among the amino acids of SEQ ID NO: 2, the amino acid at the position 4, 9, 10, 11, 12, 13, 14, 15, 16, 17, 18, 20, 21, 22, 24, 25, 27, 28, 29, 30, 31, 33, 36, 37, 38, 40, 41, 42, 43, 44, 47, 48, 50, 52, 53, 54, 59, 60, 61, 62, 63, 64, 65, 66, 67, 68, 69, 70, 72, 74, 76, 77, 80, 81, 83, 85, 86, 87, 88, 89, 90, 91, 92, 93, 94, 95, 96, 97, 99, 102, 103, 104, 107, 108, 109, 110, 114, 115, 116, 117, 118, 120, 121, 122, 126, 127, 128, 129, 130, 133, 137, 140, 142, 143, 144, 148, 149, 152, 154, 160, 162, 164, 168, 169, 174, 175, 177, 178, 183, 185, 188, 190, 192, 193, or 199 may be substituted with another amino acid.

Specifically, one or more amino acid substitutions of the variant of the CD80 protein fragment may be V4M, K9E, E10R, V11S, A12G, A12T, A12V, T13N, L14A, S15V, S15F, C16S, C16G, C16L, G17W, H18L, H18R, H18Y, V20L, S21P, V22A, E24G, L25P, Q27R, T28A, T28S, R29C, R29D, R29H, R29V, I30V, Y31F, Y31H, Y31L, Q33H, K36E, K36G, K37E, K37Q, M38I, M38L, M38T, M38V, L40M, T41A, T41G, T41D, T41I, M42T, M43I, M43Q, M43R, M43V, S44P, M47T, N48D, N48I, W50G, E52G, Y53C, K54M, F59L, F59S, D60V, I61N, T62S, N63S, N64S, L65H, S66H, I67F, I67T, V68A, V68M, I69T, L70Q, L70P, L70R, L72P, P74L, D76G, E77G, E77K, Y80N, E81A, E81R, E81V, V83A, V83I, L85I, L85R, K86E, Y87N, E88D, E88G, K89E, K89N, K89R, D90K, D90L, D90N, A91E, A91G, A91S, A91T, F92L, F92N, F92P, F92Y, K93I, K93E, K93Q, K93R, K93V, R94G, R94L, R94F, E95K, H96R, L97R, E99D, E99G, L102S, S103L, S103P, V104A, V104L, D107N, F108L, P109S, P109H, T110A, S114T, D115G, F116S, F116L, E117V, E117G, I118V, I118A, I118T, T120S, S121P, N122S, I126L, I126V, I127T, C128Y, C128R, S129L, S129P, T130A, G133D, P137L, S140T, L142S, E143G, N144D, N144S, L148S, N149D, N149S, N152T, T154I, T154A, E160G, E162G, Y164H, S168G, K169E, K169I, K169S, M174T, M174V, T175A, N177S, H178R, L183H, K185E, H188D, H188Q, R190S, N192D, Q193L, T199S, or a conservative amino acid substitution thereof.

More specifically, the substituted variant may be V4M/L70Q/A91G/T120S/T130A, A12T/H18L/M43V/F59L/E77K/P109S/I118T, A12V/S15F/Y31H/T41G/T130A/P137L/N152T, V20L/L70Q/A91S/T120S/T130A, V22A/L70Q/S121P, E24G/L25P/L70Q/T120S, T28 S/L70Q/A91G/E95K/T120 S/T13 0 A, E24G/R29D/Y31L/Q33H/K3 6G/M3 8I/T41 A/M43R/M47T/F 59L/E81 V/L85R/K89N/A91 T/F92 P/K93V/R94L/H96R, R29D/Y31L/Q33H/K3 6G/M3 8I/T41 A/M42T/M43R/M47T/E81 V/L85R/K89N/A91 T/F92P/K93 V/R94L/L148S/N149S, R29D/Y31L/Q33H/K36G/M38I/T41A/M43R/M47T/E81V/L85R/K89N/A91T/F92P/K93V/R94 L/N149S, R29D/Y31L/Q3 3H/K3 6G/M3 81/T41A/M43R/M47T/E81V/L8 5R/K89N/A91T/F92P/K93 V/R94 L/I118 T/N 149 S, R29D/Y31L/Q33H/K36G/M38I/T41A/M43R/M47T/E81V/L85R/K89N/A91T/F92P/K93V/R94 L/N144S/N149S, R29H/Y31H/T41G/Y87N/E88G/K89E/D90N/A91G/P109S, R29H/E52G/L70R/E88G/A91G/T130A, R29H/E52G/T120S/T130A, R29V/Y31F/K36G/M38L/M43Q/E81R/V83I/L85I/K89R/D90L/A91E/F92N/K93Q/R94G, R29V/M43Q/E81R/L85I/K89R/D90L/A91E/F92N/K93Q/R94G, Y31H/T41G/L70Q/A91G/T120S/T130A, K36G, K36G/K37Q/M38I/L40M, K36G/K37Q/M38I/F59L/E81V/L85R/K89N/A91T/F92P/K93V/R94L/E99G/T130A/N149S, K36E/I67T/L70Q/A91G/T120S/T130A/N152T, K37E/F59S/L70Q/A91G/T120S/T130A, M38T/L70Q/E77G/A91G/T120S/T130A/N152T, M38V/T41D/M43I/W50G/D76G/V83A/K89E/T120S/T130A, T41I/A91G, S44P/L70Q/A91G/T130A, E52G/L70Q/A91G/T120S/T130A, K54M/A91G/T120S, D60 V/A91 G/T 120 S/T 13 0 A, N63S/L70Q/A91G/T120S/T130A, S66H/D90G/T110A/F116L, I67F/L70R/E88G/A91G/T120S/T130A, I67T/L70Q/A91G/T120S, V68A/T110A, V68M/L70P/L72P/K86E, L70Q/A91G/T110A/T120S/T130A, L70Q/E81A/A91G/T120S/I127T/T130A, L70Q/Y87N/A91G/T130A, L70Q/A91G, L70Q/A91G/E117G/T120S/T130A, L70Q/A91G/T120S/T130A, L70Q/A91G/T130A, L70Q/A91G/I118A/T20S/T130A, L70R/A91G/T120S/T130A, E88D/K89R/D90K/A91G/F92Y/K93R, K89E/T130A, K89R/D90K/A91G/F92Y/K93R, E88D/K89R/D90K/A91G/F92Y/K93R/N122S/N177S, K89R/D90K/A91G/F92Y/K93R/N122S/N177S, A91G, A91G/F92L/F108L/T120S, A91G/L102S, A91G/S103P, A91G/T120S/T130A, R29D/Y31L/Q33H/K36G/M38I/T41A/M43R/M47T/E81V/L85R/K89N/A91T/F92P/K93V/R94 L/T120S/I127T/T130A/K169E, R29D/Y31L/Q33H/K36G/M38I/T41A/M43R/M47T/L70Q/E81V/L85R/K89N/A91T/F92P/K93 V/R94L/T120S/I127T/T130A, H18L/R29D/Y31L/Q33H/K36G/M38I/T41A/M43R/M47T/E81V/L85R/K89N/A91T/F92P/K93 V/R94L/T120S/I127T/T130A/K169E, R29D/Y31L/Q33H/K36G/M38I/T41A/M43R/M47T/E81V/L85R/K89N/A91T/F92P/K93V/R94 L/T120S/T130A/M174T, R29D/Y31L/Q33H/K36G/M38I/T41A/M43R/M47T/F59L/E81V/L85R/K89N/A91T/F92P/K93 V/R94L/T120S/I127T/T130A/H188D, H18R/R29D/Y31L/Q33H/K36G/K37E/M38I/T41A/M43R/M47T/L70Q/E81V/L85R/K89N/A91 T/F92P/K93V/R94L/T120S/T130A/K169E/H188D, R29D/Y31L/Q33H/K36G/M38I/T41A/M43R/M47T/L70Q/E81V/L85R/K89N/A91T/F92P/K93 V/R94L/T120S/I127T/T130A/E143G/K169E/M174V/H188D, R29D/Y31L/Q33H/K36G/M38I/T41A/M43R/M47T/L70Q/E81V/L85R/K89N/A91T/F92P/K93 V/R94L/T120S/I127T/T130A, R29D/Y31L/Q33H/K36G/M38I/T41A/M43R/M47T/E81V/L85R/K89N/A91T/F92P/K93V/R94 L/T120S/I127T/T130A/H188D, R29D/Y31L/Q33H/K36G/M38I/T41A/M43R/M47T/L70Q/E81V/L85R/K89N/A91T/F92P/K93 V/R94L/T120S/I127T/T130A/K169E, R29D/Y31L/Q33H/K36G/M38I/T41A/M43R/M47T/L70Q/E81V/L85R/K89N/A91T/F92P/K93 V/R94L/T120S/I127T/T130A, R29D/Y31L/Q33H/K36G/M38I/T41A/M43R/M47T/L85R/K89N/A91T/F92P/K93V/R94L/T12 0S/I127T/T130A/K169E/H188D, R29D/Y31L/Q33H/K36G/M38I/T41A/M43R/M47T/E81V/L85R/K89N/A91T/F92P/K93V/R94 L/F108L/T120S/T130A/K169E/H188D, R29D/Y31L/Q33H/K36G/M38I/T41A/M43R/M47T/L70Q/E81V/L85R/K89N/A91T/F92P/K93 V/R94L/T130A/H188D, R29D/Y31L/Q33H/K36G/M38I/T41A/M43R/M47T/L70Q/E81V/L85R/K89N/A91T/F92P/K93 V/R94L/T120S/I127T/T130A/K169E, H18L/R29D/Y31L/Q33H/K36G/M38I/T41A/M43R/M47T/L70Q/E81V/L85R/K89N/A91T/F92 P/K93V/R94L/T120S/T130A/K169E/H188D, R29D/Y31L/Q33H/K36G/M38I/T41A/M43R/M47T/E81V/L85R/K89N/A91T/F92P/K93V/R94 L/T120S/I127T/C128Y/T130A/H188D, R29D/Y31L/Q33H/K36G/M38I/T41A/M43R/M47T/E81V/L85R/K89N/A91T/F92P/K93V/R94 F/T130A/K169E, H18L/R29D/Y31L/Q33H/K36G/M38I/T41A/M43R/M47T/E81V/L85R/K89N/A91T/F92P/K93 V/R94L/T130A, H18L/R29D/Y31L/Q33H/K36G/M38I/T41A/M43R/M47T/L70Q/E81V/L85R/K89N/A91T/F92 P/K93V/R94L/T120S/T130A/K169E, R29D/Y31L/Q33H/K36G/M38I/T41A/M43R/M47T/L70Q/E81V/L85R/K89N/A91T/F92P/K93 I/R94L/L97R/T130A, R29D/Y31L/Q33H/K36G/M38I/T41A/M43R/M47T/L70Q/E81V/L85R/K89N/A91T/F92P/K93 I/R94L/L97R/T130A/L148S, H18L/R29D/Y31L/Q33H/K36G/M38I/T41A/M43R/M47T/E81V/L85R/K89N/A91T/F92P/K93 V/R94L/I118V/T120S/I127T/T130A/K169E, R29D/Y31L/Q33H/K36G/M38I/T41A/M43R/M47T/I61N/E81V/L85R/K89N/A91T/F92P/K93 V/R94F/V104A/T120S/T130A, R29D/Y31L/Q33H/K36G/M38I/T41A/M43R/M47T/E81V/L85R/K89N/F92P/K93V/R94F/I118 V/T130A, R29D/Y31L/Q33H/K36G/M38I/T41A/M43R/M47T/T62S/E81V/L85R/K89N/A91T/F92P/K93 V/R94L/I118V/T120S/T130A/K169E/T175A, H18L/R29D/Y31L/Q33H/K36G/M38I/T41A/M43R/M47T/E81V/L85R/K89N/A91T/F92P/K93 V/R94L/F 116S/T 130A/H188D, H18L/R29D/Y31L/Q33H/K36G/M38I/T41A/M43R/M47T/E81V/L85R/K89N/A91T/F92P/K93 V/R94L/T120S/I127T/T130A/L142S/H188D, C16S/H18L/R29D/Y31L/Q33H/K36G/M38I/T41A/M43R/M47T/E81V/L85R/K89N/A91T/F92 P/K93V/R94L/T110A/H188D, R29D/Y31L/Q33H/K36G/M38I/T41A/M43R/M47T/A91G/T120S/I127T/T130A/H188D, R29D/Y31L/Q33H/K36G/M38I/T41A/M43R/M47T/L70Q/D76G/A91G/S103L/T120S/I127T/T 130A, Q33 deleted/Y53C/L85R/K89N/A91T/F92P/K93V/R94L/T120S/I127T/T130A/K169E, T62S/E81V/L85R/K89N/A91T/F92P/K93V/R94L/T1205/T130A/K169E, R29D/Y31L/Q33H/K36G/M38I/T41A/M43R/M47T/E81V/L85R/K89N/A91T/F92P/K93V/R94 L/S129L/H188D, K9E/E10R/V11S/A12G/T13N/K14A/S15V/C16L/G17W/H18Y/Y53C/L70Q/D90G/T130A/N14 9D/N152T/H188D, H 18L/R29D/Y31L/Q33H/K3 6G/T41 A/M43R/M47T/E81 V/L85R/K89N/A91 T/F92P/K93 V/R94 L/I118V/T120S/I127T/T130A/H188D, K89E/K93E/T130A, S21P/R29D/Y31L/Q3 3H/K3 6G/M3 8 VT41A/M43R/M47T/N48I/V68 A/E81V/L85R/K89N/A91 TV/R94L/P109H/I126L/K169I, H18L/R29D/Y31L/Q33H/K36G/M38I/T41A/M43R/M47T/P74L/Y80N/E81V/L85R/K89N/A91 T/F92P/K93V/R94L/L97R, S21P/R29D/Y31L/Q33H/K3 6G/M3 8I/T41 A/M43R/M47T/P74L/Y80N/E81 V/L85R/K89N/D90 N/A91T/F92P/K93V/R94L/T130A/N149S/E162G, H 18L/R29D/Y31L/Q33H/K3 6G/M3 8I/T41 A/M43R/M47T/V68M/E81 V/L85R/K89N/A91 T/F9 2P/K93V/R94L/T130A, R29D/Y31L/Q33H/K36G/M38I/T41A/M43R/M47T/V68M/E81V/L85R/K89N/A91T/F92P/K93 V/R94L/T130A/N149S/R190S, H18L/R29D/Y31L/Q33H/K36G/M38I/T41A/M43R/M47T/P74L/Y80N/E81V/L85R/K89N/A91 T/F92P/K93V/R94L/T130A/R190S, C16G/V22A/R29D/Y31L/Q3 3H/K3 6G/M3 8I/T41A/M43R/M47T/V68M/D76G/E81V/L85R/K8 9N/A91T/F92P/K93V/R94L/I118T/T130A/S140T/N149S/K169I/H178R/N192D, R29D/Y31L/Q33H/K3 6G/M3 8I/T41 A/M43R/M47T/E81 V/L85R/K89N/A91 T/F92P/K93 V/R94 F/E117V/I118T/N149S/S168G/H188Q, V22A/R29D/Y31L/Q33H/K3 6G/M3 8I/T41 A/M43R/M47T/V68M/E81 V/L85R/K89N/A91 T/F9 2P/K93V/R94L/T130A, R29D/Y31L/Q33H/K36G/M38I/T41A/M43R/M47T/N64S/E81V/L85R/K89N/A91T/F92P/K93 V/R94F/I118T/T130A/N149S/K169I, V22A/R29D/Y31L/Q33H/K3 6G/M3 8I/T41 A/M43R/M47T/V68M/E81 V/L85R/K89N/A91 T/F9 2P/K93V/R94L/D115G/I118T/T130A/G133D/N149S, S129P, A91G/S129P, I69T/L70Q/A91G/T120S, Y31H/S129P, T28A/R29D/Y31L/Q33H/K36G/M38I/T41A/M43R/M47T/E81V/L85R/K89N/A91T/F92P/K93 V/R94L/V104L/T130A/N149S, H18L/R29D/Y31L/Q33H/K36G/M38I/T41A/M43R/M47T/E81V/L85R/K89N/A91T/F92P/K93 V/R94L/L97R/N149S/H188Q, H 18L/R29D/Y31L/Q33H/K3 6G/M3 8I/T41 A/M43R/M47T/E81 V/L85R/K89N/A91 T/F92P/K93 V/R94L/L97R/N149S, H18L/R29D/Y31L/Q33H/K36G/M38I/T41A/M43R/M47T/V68A/E81V/L85R/K89N/A91T/F92 P/K93V/R94L/T130A/N149S/T154I, A12G/R29D/Y31L/Q33H/K36G/M38I/T41A/M43R/M47T/V68A/E81V/L85R/K89N/A91T/F92 P/K93V/R94L/L97R/T130A/L183H, R29D/Y31L/Q33H/K36G/M38I/T41A/M43R/M47T/E81V/L85R/K89N/A91T/F92P/K93V/R94 L/I118T/T130A/S140T/N149S/K169S, R29D/Y31L/Q33H/K36G/M38I/T41A/M43R/M47T/E81V/L85R/K89N/A91T/F92P/K93 V/R94 L/I118T/T130A/N149S/K169I/Q193L, V22A/R29D/Y31L/Q33H/K36G/M38I/T41A/M43R/M47T/E81V/L85R/K89N/A91T/F92P/K93 V/R94L/I118T/T130A/N149S, R29D/Y31L/Q33H/K3 6G/M3 8I/T41 A/M43R/M47T/E81 V/L85R/K89N/A91 T/F92P/K93 V/R94 L/I118T/T130A/N149S, R29D/Y31L/Q33H/K36G/M38I/T41A/M43R/M47T/E81V/L85R/K89N/A91T/F92P/K93V/R94 L/I118T/T130A/N149S/K169I, R29D/Y31L/Q3 3H/K3 6G/M3 8I/T41A/M43R/M47T/E81V/L8 5R/K89N/A91T/F92P/K93 V/R94 F/T130A/N149S/K169I, I118T/C128R, Q27R/R29C/M42T/S129P/E160G, S129P/T154A, S21P/L70Q/D90G/T120S/T130A, L70Q/A91G/N144D, L70Q/A91G/I118A/T120S/T130A/K169E, V4M/L70Q/A91G/I118V/T120S/T130A/K169E, L70Q/A91G/I118V/T120S/T130A/K169E, L70Q/A91G/I118V/T120S/T130A, V20L/L70Q/A91S/I118V/T120S/T130A, L70Q/A91G/E117G/I118V/T120S/T130A, A91G/I118V/T120S/T130A, L70R/A91G/I118V/T120S/T130A/T199S, L70Q/E81A/A91G/I118V/T120S/I127T/T130A, T28S/L70Q/A91G/E95K/I118V/T120S/I126V/T130A/K169E, N63S/L70Q/A91G/S114T/I118V/T120S/T130A, K36E/I67T/L70Q/A91G/I118V/T120S/T130A/N152T, E52G/L70Q/A91G/D107N/I118V/T120S/T130A/K169E, K37E/F59S/L70Q/A91G/I118V/T120S/T130A/K185E, D60V/A91G/I118V/T120S/T130AK169E, K54M/L70Q/A91G/Y164H/T120S, M38T/L70Q/E77G/A91G/I118V/T120S/T130A/N152T, Y31H/T41G/M43L/L70Q/A91G/I118V/T120S/I126V/T130A, L65H/D90G/T110A/F116L, R29H/E52G/D90N/I118V/T120S/T130A, I67T/L70Q/A91G/I118V/T120S, L70Q/A91G/T110A/I118V/T120S/T130A, M38V/T41D/M43I/W50G/D76G/V83A/K89E/I118V/T120S/I126V/T130A, A12V/S1SF/Y31H/M38L/T41G/M43L/D90N/T130A/P137L/N149D/N152T, I67F/L70R/E88G/A91G/I118V/T120S/T130A, E24G/L25P/L70Q/A91G/I118V/T120S/N152T, A91G/F92L/F108L/I118V/T120S, E88D/K89R/D90K/A91G/F92Y/K93R/N122S/N177S, K36G/K37Q/M38I/L40M/F59L/E81V/L85R/K89N/A91T/F92P/K93V/R94L/E99G/T130A/N14 9S, K36G/L40M, R29D/Y31L/Q33H/K36G/M38I/T41A/M43R/M47T/E81V/L85R/K89N/A91T/F92P/K93V/R94 L/I118V/T120S/I127T/T130A/K169E, R29D/Y31L/Q33H/K36G/M38I/T41A/M43R/M47T/L70Q/E81V/L85R/K89N/A91T/F92P/K93 V/R94L/I118V/T120S/I127T/T130A, H18L/R29D/Y31L/Q33H/K36G/M38I/T41A/M43R/M47T/E81V/L85R/K89N/A91T/F92P/K93 V/R94L/I118V/T120S/I127T/T130A/K169E, R29D/Y31L/Q33H/K36G/M38I/T41A/M43R/M47T/E81V/L85R/K89N/A91T/F92P/K93V/R94 L/I118V/T120S/T130A/K169E/M174T, R29D/Y31L/Q33H/K36G/M38I/T41A/M43R/M47T/N48D/F59L/E81V/L85R/K89N/A91T/F92 P/K93V/R94L/I118V/T120S/I127T/T130A/H188D, H18R/R29D/Y31L/Q33H/K36G/K37E/M38I/T41A/M43R/M47T/L70Q/E81V/L85R/K89N/A91 T/F92P/K93V/R94L/I118V/T120S/T130A/K169E/H188D, R29D/Y31L/Q33H/K36G/M38I/T41A/M43R/M47T/L70Q/E81V/L85R/K89N/A91T/F92P/K93 V/R94L/I118V/T120S/I127T/T130A/E143G/K169E/M174V/H188D, R29D/I30V/Y31L/Q33H/K36G/M38I/T41A/M43R/M47T/E81V/L85R/K89N/A91T/F92P/K93 V/R94L/I118V/T120S/I127T/T130A/H188D, R29D/Y31L/Q33H/K36G/M38I/T41A/M43R/M47T/E81V/L85R/K89N/A91T/F92P/K93V/R94 L/I118V/T120S/I127T/T130A/H188D, R29D/Y31L/Q33H/K3 6G/M3 8I/T41 A/M43R/M47T/L70Q/E81 V/L85R/K89N/A91 T/F92P/K93 V/R94L/I118V/T120S/I127T/T130A/K169E, R29D/Y31L/Q33H/K36G/M38I/T41A/M43R/M47T/L70Q/E81V/K89N/A91T/F92P/K93V/R94 L/I118V/T120S/I127T/T130A, R29D/Y31L/Q33H/K36G/M38I/T41A/M43R/M47T/L85R/K89N/A91T/F92P/K93V/R94L/I118 V/T120S/I127T/T130A/K169E/H188D, R29D/Y31L/Q33H/K36G/M38I/T41A/M43R/M47T/E81V/L85R/K89N/A91T/F92P/K93V/R94 L/F108L/I118V/T1205/T130A/K169E/H188D, R29D/Y31L/Q33H/K36G/M38I/T41A/M43R/M47T/L70Q/E81V/L85R/K89N/A91T/F92P/K93 V/R94L/I118V/T120S/T130A/N149D/K169E/H188D, H18L/R29D/Y31L/Q33H/K36G/M38I/T41A/M43R/M47T/L70Q/E81V/L85R/K89N/A91T/F92 P/K93V/R94L/I118V/T120S/T130A/K169E/H188D, R29D/Y31L/Q33H/K36G/M38I/T41A/M43R/M47T/E81V/L85R/K89N/A91T/F92P/K93V/R94 L/1118V/T120S/1127T/C128Y/T130A/H188D, H18L/R29D/Y31L/Q33H/K36G/M38I/T41A/M43R/M47T/E81V/L85R/K89N/A91T/F92P/K93 V/R94L/E99D/T130A, H18L/R29D/Y31L/Q33H/K36G/M38I/T41A/M43R/M47T/L70Q/E81V/L85R/K89N/A91T/F92 P/K93V/R94L/I118V/T120S/T130A/K169E, R29D/Y31L/Q33H/K36G/M38I/T41AM43R/M47T/I61N/E81V/L85R/K89N/A91T/F92P/K93 V/R94F/V104A/I118V/T120S/I126V/T130A, R29D/Y31L/Q33H/K36G/M38I/T41A/M43R/M47T/E81V/L85R/K89N/A91T/F92P/K93V/R94 F/I118V/T120S/T130A, H18L/R29D/Y31L/Q33H/K36G/M38I/T41A/M43R/M47T/E81V/L85R/K89N/A91T/F92P/K93 V/R94L/I118V/T120S/I127T/T130A/L142S/H188D, C16S/H18L/R29D/Y31L/Q33H/K36G/M38I/T41A/M43R/M47T/E81V/L85R/K89N/A91T/F92 P/K93V/R94L/T110A/I118V/H188D, R29D/Y31L/Q33H/K36G/M38I/T41A/M43R/M47T/A91G/I118V/T120S/I127T/T130A/H188D, R29D/Y31L/Q33H/K36G/M38I/T41A/M43R/M47T/L70Q/D76G/A91G/S103L/I118V/T120S/I 127T/T130A, Y53C/L85R/K89N/A91T/F92P/K93V/R94L/I118V/T120S/I127T/T130A/K169E, T62S/E81V/L85R/K89N/A91T/F92P/K93V/R94L/I118V/T120S/T130A/K169E, Y53C/L70Q/D90G/T130A/N149D/N152T/H188D, H 18L/R29D/Y31L/Q33H/K3 6G/M3 8I/T41 A/M43R/M47T/E81 V/L85R/K89N/A91 T/F92P/K93 V/R94L/I118V/T120S/I127T/T130A/H188D, or H 18L/R29D/Y31L/Q33H/K3 6G/M3 8I/T41 A/M43R/M47T/E81 V/L85R/K89N/A91 T/F92P/K93 V/R94L/T130A/N149S.

In addition, an embodiment of the CD80 variant may be any one selected from the group consisting of A12T, H18L, V20L, R29H, Y31H, K36G, T41G, T41I, M43V, E52G, F59L, D60V, N63S, I67T, L70Q, L70R, E77K, E81A, Y87N, E88D, E88G, K89E, K89R, D90K, D90N, L40M, A91G, A91S, F92Y, K93R, D107N, P109S, S114T, E117G, I118A, I118T, I118V, T120S, I127T, T130A, N144D, K169E, N177S, and T199S, and a conservative amino acid substitution thereof.

Specifically, one or more amino acid substitutions of the variant may be A12T/H18L/M43V/F59L/E77K/P109S/I118T, V20L/L70Q/A91S/T120S/T130A, V20L/L70Q/A91S/I118V/T120S/T130A, R29H/Y31H/T41G/Y87N/E88G/K89E/D90N/A91G/P109S, K36G, K36G/L40M, T41I/A91G, E52G/L70/A91G/T120S/T130A, E52G/L70Q/A91G/D107N/I118V/T120S/T130A/K169E, D60 V/A91 G/T 120 S/T 13 0 A, D60V/A91G/I118V/T120S/T130A/K169E, N63S/L70Q/A91G/T120S/T130A, N63S/L70Q/A91G/S114T/I118V/T120S/T130A, I67T/L70Q/A91G/T120S, I67T/L70Q/A91G/I118V/T120S, L70Q/E81A/A91G/T120S/I127T/T130A, L70Q/E81A/A91G/I118V/T120S/I127T/T130A, L70Q/Y87N/A91G/T130A, L70Q/A91G, L70Q/A91G/N144D, L70Q/A91G/E117G/T120S/T130A, L70Q/A91G/E117G/I118V/T120S/T130A, L70Q/A91G/I118A/T120S/T130A, L70Q/A91G/I118A/T120S/T130A/K169E, L70Q/A91G/T120S/T130A, L70R/A91G/T120S/T130A, L70R/A91G/I118V/T120S/T130A/T199SL70Q/A91G/I118V/T120S/T130A/K169E, E88D/K89R/D90K/A91G/F92Y/K93R, K89R/D90K/A91G/F92Y/K93R, E88D/K89R/D90K/A91G/F92Y/K93R/N122S/N177S, or K89R/D90K/A91G/F92Y/K93R/N122S/N177S.

In addition, one or more amino acid substitutions of the CD80 variant may be selected from the group consisting of A12G, A12T, H18L, S21P, V22A, T28A, R29D, R29H, Y31H, Y31L, Q33H, K36G, M38I, M38L, L40M, T41A, T41G, M42T, M43R, M43V, M47T, N48I, F59L, N64S, I67T, V68A, V68M, L70Q, E77K, E81V, L85R, Y87N, E88D, E88G, K89E, K89N, K89R, D90K, D90N, A91G, A91T, F92P, F92Y, K93E, K93V, R94F, R94L, L97R, S103L, S103P, V104L, P109H, P109S, D115G, E117V, I118T, I118V, T120S, N122S, I126L, T130A, G133D, S140T, N144S, L148S, N149S, S168G, K169I, K169S, N177S, L183H, H188Q, R190S and Q193L, and a conservative amino acid substitution thereof.

Specifically, one or more amino acid substitutions of the CD80 variant may be A 12T/H 18L/M43 V/F 59L/E77K/P 109 S/I118T, R29D/Y31L/Q33H/K36G/M38I/T41A/M43R/M47T/E81V/L85R/K89N/A91T/F92P/K93V/R94 L/I 118 T/N 149 S, R29D/Y31L/Q33H/K36G/M38I/T41A/M43R/M47T/E81V/L85R/K89N/A91T/F92P/K93V/R94 L/N144S/N149S, R29D/Y31L/Q33H/K36G/M38I/T41A/M42T/M43R/M47T/E81V/L85R/K89N/A9IT/F92P/K93 V/R94L/L148S/N149S, R29D/Y31L/Q33H/K36G/M38I/T41A/M43R/M47T/E81V/L85R/K89N/A91T/F92P/K93V/R94 L/N149S, R29H/Y31H/T41G/Y87N/E88G/K89E/D90N/A91G/P109S, K36G, K36G/L40M, I67T/L70Q/A91G/T120S, I67T/L70Q/A91G/I118V/T120S, E88D/K89R/D90K/A91G/F92Y/K93R, K89R/D90K/A91G/F92Y/K93R, A91G, R29D/Y31L/Q33H/K36G/M38I/T41A/M43R/M47T/E81V/L85R/K89N/A91T/F92P/K93V/R94 L/I 118 T/N 149 S, R29D/Y31L/Q33H/K3 6G/M3 8I/T41 A/M43R/M47T/E81 V/L85R/K89N/A91 T/F92P/K93 V/R94 L/N144S/N149S, R29D/Y31L/Q33H/K36G/M38I/41A/M42T/M43R/M47T/E81V/L85R/K89N/A91T/F92P/K93 V/R94L/L148S/N149S, R29D/Y31L/Q33H/K36G/M38I/T41A/M43R/M47T/E81V/L85R/K89N/A91T/F92P/K93V/R94 L/N149S, R29H/Y31H/T41G/Y87N/E88G/K89E/D90N/A91G/P109S, K36G, K36G/L40M, I67T/L70Q/A91G/T120S, I67T/L70Q/A91G/I118V/T120S, E88D/K89R/D90K/A91G/F92Y/K93R, K89R/D90K/A91G/F92Y/K93R, A91G, H18L/R29D/Y31L/Q33H/K36G/M38I/T41A/M43R/M47T/E81V/L85R/K89N/A91T/F92P/K93 V/R94L/I118V/T120S/I127T/T130A/H188D, R29D/Y31L/Q33H/K36G/M38I/T41A/M43R/M47T/V68M/E81V/L85R/K89N/A91T/F92P/K93 V/R94L/T130A/N149S/R190S, R29D/Y31L/Q33H/K36G/M38I/T41A/M43R/M47T/E81V/L85R/K89N/A91T/F92P/K93V/R94 F/E117V/I118T/N149S/S168G/H188Q, V22A/R29D/Y31L/Q33H/K36G/M38I/T41A/M43R/M47T/V68M/E81V/L85R/K89N/A91T/F9 2P/K93V/R94L/T130A, R29D/Y31L/Q33H/K36G/M38I/T41A/M43R/M47T/N64S/E81V/L85R/K89N/A91T/F92P/K93 V/R94F/I118T/T130A/N149S/K1691, V22A/R29D/Y31L/Q33H/K36G/M38I/T41A/M43R/M47T/V68M/E81V/L85R/K89N/A91T/F9 2P/K93V/R94L/D115G/I118T/T130A/G133D/N149S, T28A/R29D/Y31L/Q33H/K3 6G/M3 8I/T41 A/M43R/M47T/E81 V/L85R/K89N/A91 T/F92P/K93 V/R94L/V104L/T130A/N149S, H 18L/R29D/Y31L/Q33H/K3 6G/M3 8I/T41 A/M43R/M47T/E81 V/L85R/K89N/A91 T/F92P/K93 V/R94L/L97R/N149S/H188Q, K89E/T130A, K89E/K93E/T130A, S21P/R29D/Y31L/Q3 3H/K3 6G/M3 8I/T41A/M43R/M47T/N48I/V68 A/E81V/L85R/K89N/A91 T/F92P/K93 V/R94L/S21P/N48I/V68A/P 109H/I126L/K 169I, H 18L/R29D/Y31L/Q33H/K3 6G/M3 8I/T41 A/M43R/M47T/V68M/E81 V/L85R/K89N/A91 T/F9 2P/K93V/R94L/T130A, H 18L/R29D/Y31L/Q33H/K3 6G/M3 8I/T41 A/M43R/M47T/E81 V/L85R/K89N/A91 T/F92P/K93 V/R94L/T130A/N149S, A 12G/R29D/Y31L/Q33H/K3 6G/M3 8I/T41 A/M43R/M47T/V68A/E81 V/L85R/K89N/A91 T/F92 P/K93V/R94L/L97R/T130A/L183H, R29D/Y31L/Q33H/K36G/M38I/T41A/M43R/M47T/E81V/L85R/K89N/A91T/F92P/K93V/R94 L/I118T/T130A/S140T/N149S/K169S, R29D/Y31L/Q33H/K3 6G/M3 8I/T41 A/M43R/M47T/E81 V/L85R/K89N/A91 T/F92P/K93 V/R94 L/I118T/T130A/N149 S/K169I/Q193L, V22A/R29D/Y31L/Q33H/K3 6G/M3 8I/T41 A/M43R/M47T/E81 V/L85R/K89N/A91 T/F92P/K93 V/R94L/I118T/T130A/N149S, R29D/Y31L/Q33H/K36G/M38I/T41A/M43R/M47T/E81V/L85R/K89N/A91T/F92P/K93V/R94 L/I118T/T130A/N149S, R29D/Y31L/Q33H/K36G/M38I/T41A/M43R/M47T/E81V/L85R/K89N/A91T/F92P/K93V/R94 L/I118T/T130A/N149S/K169I, or R29D/Y31L/Q33H/K36G/M38I/T41A/M43R/M47T/E81V/L85R/K89N/A91T/F92P/K93V/R94 F/T130A/N149S/K169I.

In addition, one or more amino acid substitutions of the CD80 variant may be selected from the group consisting of A12T, H18L, K36G, L40M, M43V, F59L, E77K, E88D, K89R, D90K, A91G, F92Y, K93R, P109S, I118T, N112S, N177S, and a conservative amino acid substitution thereof.

Specifically, one or more amino acid substitutions of the CD80 variant may be A12T/H18L/M43V/F59L/E77K/P109S/I118T, K36G, K36G/L40M, E88D/K89R/D90K/A91G/F92Y/K93R, K89R/D90K/A91G/F92Y/K93R, E88D/K89R/D90K/A91G/F92Y/K93R/N122S/N177S, or K89R/D90K/A91G/F92Y/K93R/N122S/N177S.

In addition, one or more amino acid substitutions of the CD80 variant may be selected from the group consisting of V4M, R29H, Y31H, K36G, L40M, T41G, E52G, I67T, V68A, L70Q, Y87N, E88D, E88G, K89E, K89R, D90K, D90N, A91G, F92Y, K93R, D107N, P109S, T110A, 1118V, T120S, T130A, N144D, and K169E, and a conservative amino acid substitution thereof.

Specifically, one or more amino acid substitutions of the CD80 variant may be V4M/L70Q/A91G/T120S/T130A, V4M/L70Q/A91G/I118V/T120S/T130A/K169E, R29H/Y31H/T41G/Y87N/E88G/K89E/D90N/A91G/P109S, K36G, K36G/L40M, E52G/L70Q/A91G/T120S/T130A, E52G/L70Q/A91G/D107N/I118V/T120S/T130A/K169E, I67T/L70Q/A91G/T120S, I67T/L70Q/A91G/I118V/T120S,V68A/T110A, L70Q/A91G, L70Q/A91G/N144D, L70Q/A91G/T120S/T130A, L70Q/A91G/I118V/T120S/T130A/K169E, L70Q/A91G/T130A, K89R/D90K/A91G/F92Y/K93R, E88D/K89R/D90K/A91G/F92Y/K93R, A91G/I118V/T120S/T130A, A91G/T120S/T130A, or I69T/L70Q/A91G/T120S.

In addition, one or more amino acid substitutions of the CD80 variant may be selected from the group consisting of K36G, L40M, E52G, L70Q, E88D, K89R, D90K, A91G, F92Y, K93R, D107N, I118V, T120S, T130A, N144D, and K169E, and a conservative amino acid substitution thereof.

Specifically, one or more amino acid substitutions of the CD80 variant may be K36G, K36G/L40M, E52G/L70Q/A91G/T120S/T130A, E52G/L70Q/A91G/D107N/I118V/T120S/T130A/K169E, L70Q/A91G, L70Q/A91G/N144D, L70Q/A91G/T120S/T130A, L70Q/A91G/I118V/T120S/T130A/K169E, E88D/K89R/D90K/A91G/F92Y/K93R, or K89R/D90K/A91G/F92Y/K93R.

In addition, one or more amino acid substitutions of the CD80 variant may be selected from the group consisting of R29H, Y31H, K36G, L40M, T41G, I67T, L70Q, Y87N, E88D, E88G, K89E, K89R, D90N, D90K, A91G, F92Y, K93R, P109S, I118V, T120S, and a conservative amino acid substitution thereof.

Specifically, one or more amino acid substitutions of the CD80 variant may be R29H/Y31H/T41G/Y87N/E88G/K89E/D90N/A91G/P109S, K36G, K36G/L40M, I67T/L70Q/A91G/T120S, I67T/L70Q/A91G/I118V/T120S, E88D/K89R/D90K/A91G/F92Y/K93R, or K89R/D90K/A91G/F92Y/K93R.

In addition, one or more amino acid substitutions of the CD80 variant may be selected from the group consisting of K36G, L40M, E88D, K89R, D90K, A91G, F92Y, K93R, and a conservative amino acid substitution thereof.

Specifically, one or more amino acid substitutions of the CD80 variant may be K36G, K36G/L40ME88D/K89R/D90K/A91G/F92Y/K93R, or K89R/D90K/A91G/F92Y/K93R.

In addition, one or more amino acid substitutions of the CD80 variant may be selected from the group consisting of R29D, R29H, Y31H, Y31L, Q33H, K36G, M38I, T41A, T41G, M42T, M43R, M47T, N63S, I67T, L70Q, E81A, E81V, L85R, Y87N, E88G, K89E, K89N, D90N, A91G, A91T, F92P, K93V, R94L, P109S, S114T, I118T, I118V, T120S, I127T, T130A, N144S, L148S, and N149S, and a conservative amino acid substitution thereof.

Specifically, one or more amino acid substitutions of the CD80 variant may be N63S/L70Q/A91G/T120S/T130A, N63S/L70Q/A91G/S114T/I118V/T120S/T130A, or L70Q/Y87N/A91G/T120S/I127T/T130A.

In addition, one or more amino acid substitutions of the CD80 variant may be selected from the group consisting of N63S, L70Q, E81A, Y87N, A91G, S114T, I118V, T120S, I127T, and T130A, and a conservative amino acid substitution thereof.

Specifically, one or more amino acid substitutions of the CD80 variant may be R29D/Y31L/Q33H/K3 6G/M3 8I/T41 A/M42T/M43R/M47T/E81 V/L85R/K89N/A91 T/F92P/K93 V/R94L/L148S/N149S, R29D/Y31L/Q33H/K36G/M38I/T41A/M43R/M47T/E81V/L85R/K89N/A91T/F92P/K93V/R94 L/I 118 T/N 149 S, R29D/Y31L/Q33H/K3 6G/M3 8I/T41 A/M43R/M47T/E81 V/L85R/K89N/A91 T/F92P/K93 V/R94 L/N144S/N149S, R29D/Y31L/Q33H/K36G/M38I/T41A/M43R/M47T/E81V/L85R/K89N/A91T/F92P/K93V/R94 L/N149S, R29H/Y31 H/T41 G/Y87N/E8 8 G/K 8 9E/D 90N/A91 G/P 109 S, N63S/L70Q/A91G/T120S/T130A, N63S/L70Q/A91G/S114T/I118V/T120S/T130A, I67T/L70Q/A91G/T120S, I67T/L70Q/A91G/I118V/T120S, or L70Q/Y87N/A91G/T120S/I127T/T130A.

In addition, one or more amino acid substitutions of the CD80 variant may be selected from the group consisting of R29D, R29H, Y31H, Y31L, Q33H, K36G, M38I, T41A, T41G, M42T, M43R, M47T, I67T, L70Q, E81V, L85R, Y87N, E88G, K89E, K89N, D90N, A91G, A91T, F92P, K93V, R94L, P109S, I118T, I118V, T120S, N144S, L148S, andN149S, and a conservative amino acid substitution thereof.

Specifically, one or more amino acid substitutions of the CD80 variant may be R29D/Y31L/Q33H/K36G/M38I/T41A/M42T/M43R/M47T/E81V/L85R/K89N/A91T/F92P/K93 V/R94L/L148S/N149S, R29D/Y31L/Q33H/K36G/M38I/T41A/M43R/M47T/E81V/L85R/K89N/A91T/F92P/K93V/R94 L/I 118 T/N 149 S, R29D/Y31L/Q33H/K36G/M38I/T41A/M43R/M47T/E81V/L85R/K89N/A91T/F92P/K93V/R94 L/N144S/N149S, R29D/Y31L/Q33H/K36G/M38I/T41A/M43R/M47T/E81V/L85R/K89N/A91T/F92P/K93V/R94 L/N149S, R29H/Y31 H/T41 G/Y87N/E8 8 G/K 8 9E/D 90N/A91 G/P 109 S, I67T/L70Q/A91G/I118V/T120S or I67T/L70Q/A91G/T120S.

In addition, one or more amino acid substitutions of the CD80 variant may be selected from the group consisting of L70Q, Y87N, A91G, and T120S, and a conservative amino acid substitution thereof.

In an embodiment of the CD80 variant, the variant of the CD80 protein fragment is one in which at least one amino acid has been substituted with another amino acid. Specifically, the variant of the CD80 protein fragment may be any one selected from the group consisting of K89E/T130A, K89E/K93E/T130A, H 18L/R29D/Y31L/Q33H/K3 6G/M3 8I/T41 A/M43R/M47T/E81 V/L85R/K89N/A91 T/F92P/K93 V/R94L/L97R/T130A/N149S, R29D/Y31L/Q33H/K36G/M38I/T41A/M43R/M47T/E81V/L85R/K89N/A91T/F92P/K93V/R94 L/A12G/V68A/L97R/T130A/L183H, A12G/H18L/V22A/N64S/V68M/T130A, and A12G/H18L/V22A/N64S/V68M/T130A.

In addition, the IL-2 protein and the CD80 protein may be attached to each other via a linker or a carrier. Specifically, the IL-2 or a variant thereof and the CD80 (B7-1) or a fragment thereof may be attached to each other via a linker or a carrier. In the present description, the linker and the carrier may be used interchangeably.

The linker links two proteins. An embodiment of the linker may include 1 to 50 amino acids, albumin or a fragment thereof, an Fc domain of an immunoglobulin, or the like. Here, the Fc domain of immunoglobulin refers to a protein that contains heavy chain constant region 2 (CH2) and heavy chain constant region 3 (CH3) of an immunoglobulin, and does not contain heavy and light chain variable regions and light chain constant region 1 (CH1) of an immunoglobulin. The immunoglobulin may be IgG, IgA, IgE, IgD, or IgM, and may preferably be IgG4. Here, Fc domain of wild-type immunoglobulin G4 may have the amino acid sequence of SEQ ID NO: 4.

In addition, the Fc domain of an immunoglobulin may be an Fc domain variant as well as wild-type Fc domain. In addition, as used herein, the term "Fc domain variant" may refer to a form which is different from the wild-type Fc domain in terms of glycosylation pattern, has a high glycosylation as compared with the wild-type Fc domain, or has a low glycosylation as compared with the wild-type Fc domain, or a deglycosylated form. In addition, an aglycosylated Fc domain is included therein. The Fc domain or a variant thereof may be adapted to have an adjusted number of sialic acids, fucosylations, or glycosylations, through culture conditions or genetic manipulation of a host.

In addition, glycosylation of the Fc domain of an immunoglobulin may be modified by conventional methods such as chemical methods, enzymatic methods, and genetic engineering methods using microorganisms. In addition, the Fc domain variant may be in a mixed form of respective Fc regions of immunoglobulins, IgG, IgA, IgE, IgD, and IgM. In addition, the Fc domain variant may be in a form in which some amino acids of the Fc domain are substituted with other amino acids. An embodiment of the Fc domain variant may have the amino acid sequence of SEQ ID NO: 12.

The fusion protein may have a structure in which, using an Fc domain as a linker (or carrier), a CD80 protein and an IL-2 protein, or an IL-2 protein and a CD80 protein are linked to N-terminus and C-terminus of the linker or carrier, respectively. Linkage between N-terminus or C-terminus of the Fc domain and CD80 or IL-2 may optionally be achieved by a linker peptide.

Specifically, a fusion protein may consist of the following structural formula (I) or (II):
N'-X-[linker (1)]ₙ-Fc domain-[linker (2)]ₘ-Y-C' (I)
N'-Y-[linker (1)]ₙ-Fc domain-[linker (2)]ₘ-X-C' (II)
Here, in the structural formulas (I) and (II),
N' is the N-terminus of the fusion protein,
C' is the C-terminus of the fusion protein,
X is a CD80 protein,
Y is an IL-2 protein,
the linkers (1) and (2) are peptide linkers, and
n and m are each independently 0 or 1.

Preferably, the fusion protein may consist of the structural formula (I). The IL-2 protein is as described above. In addition, a CD80 fragment or a variant thereof is as described above. According to an embodiment, the IL-2 protein may be an IL-2 variant with one to five amino acid substitutions as compared with the wild-type IL-2. The CD80 fragment or a variant thereof may be a fragment obtained by truncation of up to about 34 contiguous amino acid residues from the N-terminus or C-terminus of the wild-type CD80. Alternatively, the CD80 protein may be an extracellular immunoglobulin-like domain having the activity of binding to the T cell surface receptors CTLA-4 and CD28. In addition, a variant of the CD80 fragment is as described above.

Specifically, the fusion protein may have the amino acid sequence of SEQ ID NO: 9, 26, 28, or 30. According to another embodiment, the fusion protein includes a polypeptide having a sequence identity of 85%, 86%, 87%, 88%, 89%, 90%, 91%, 92%, 93%, 94%, 95%, 96%, 97%, 98%, 99%, or 100% to the amino acid sequence of SEQ ID NO: 9, 26, 28, or 30. Here, the identity is, for example, percent homology, and may be determined through homology comparison software such as BlastN software of the National Center of Biotechnology Information (NCBI).

The peptide linker (1) may be included between the CD80 protein and the Fc domain. The peptide linker (1) may consist of 5 to 80 contiguous amino acids, 20 to 60 contiguous amino acids, 25 to 50 contiguous amino acids, or 30 to 40 contiguous amino acids. In an embodiment, the peptide linker (1) may consist of 30 amino acids. In addition, the peptide linker (1) may contain at least one cysteine. Specifically, the peptide linker (1) may contain one, two, or three cysteines. In addition, the peptide linker (1) may be derived from the hinge of an immunoglobulin. In an embodiment, the peptide linker (1) may be a peptide linker consisting of the amino acid sequence of SEQ ID NO: 3.

The peptide linker (2) may consist of 1 to 50 contiguous amino acids, 3 to 30 contiguous amino acids, or 5 to 15 contiguous amino acids. In an embodiment, the peptide linker (2) may be (G4S)ₙ (where n is an integer of 1 to 10). Here, in (G4S)ₙ, n may be 1, 2, 3, 4, 5, 6, 7, 8, 9, or 10. In an embodiment, the peptide linker (2) may be a peptide linker consisting of the amino acid sequence of SEQ ID NO: 5.

In another aspect of the present invention, there is provided a dimer obtained by binding of two fusion proteins, each of which comprises an IL-2 protein and a CD80 protein fragment or a variant thereof. The fusion protein comprising an IL-2 or a variant thereof, and a CD80 protein fragment or a variant thereof is as described above.

Here, the binding between the fusion proteins constituting the dimer may be achieved by, but is not limited to, a disulfide bond formed by cysteines present in the linker. The fusion proteins constituting the dimer may be the same or different fusion proteins from each other. Preferably, the dimer may be a homodimer. An embodiment of the fusion protein constituting the dimer may be a protein having the amino acid sequence of SEQ ID NO: 9.

### Polynucleotide encoding fusion protein

In yet another aspect of the present invention, there is provided a polynucleotide encoding a fusion protein comprising an IL-2 protein and a CD80 protein fragment or a variant thereof. The fusion protein comprising an IL-2 protein and a CD80 protein fragment or a variant thereof is as described above. In the polynucleotide, one or more nucleotides may be altered by substitution, deletion, insertion, or a combination thereof. When a nucleotide sequence is prepared by chemical synthesis, synthetic methods well known in the art may be used, such as those described in Engels and Uhlmann (Angew Chem IntEd Eng., 37: 73-127, 1988). Such methods may include triester, phosphite, phosphoramidite and H-phosphate methods, PCR and other autoprimer methods, oligonucleotide syntheses on solid supports, and the like.

The polynucleotide may further contain a nucleic acid encoding a signal sequence or a leader sequence. As used herein, the term "signal sequence" refers to a signal peptide that directs secretion of a target protein. The signal peptide is translated and then cleaved in a host cell. Specifically, the signal sequence is an amino acid sequence that initiates migration of a protein across the endoplasmic reticulum (ER) membrane. In an embodiment, the signal sequence may have the amino acid sequence of SEQ ID NO: 1.

Signal sequences are well known in the art for their characteristics. Such signal sequences typically contain 16 to 30 amino acid residues, and may contain more or fewer amino acid residues than such amino acid residues. A typical signal peptide is composed of three regions, that is, a basic N-terminal region, a central hydrophobic region, and a more polar C-terminal region. The central hydrophobic region contains 4 to 12 hydrophobic residues that cause the signal sequence to be immobilized during migration of an immature polypeptide through the membrane lipid bilayer.

After initiation, signal sequences are cleaved in the lumen of ER by cellular enzymes, commonly known as signal peptidases. Here, the signal sequence may be a secretory signal sequence of tPa (tissue plasminogen activator), HSV gDs (signal sequence of Herpes simplex virus glycoprotein D), or a growth hormone. Preferably, a secretory signal sequence used in higher eukaryotic cells including mammals, and the like may be used. In addition, a signal sequence included in the wild-type IL-2 and/or CD80 may be used, or a signal sequence that has been substituted with a codon having high expression frequency in a host cell may be used.

### Vector with polynucleotide encoding fusion protein

In still yet another aspect of the present invention, there is provided a vector comprising the polynucleotide.

The vector may be introduced into a host cell to be recombined with and inserted into the genome of the host cell. Also, the vector is understood as nucleic acid means containing a polynucleotide sequence which is autonomously replicable as an episome. The vectors include linear nucleic acids, plasmids, phagemids, cosmids, RNA vectors, viral vectors, and analogs thereof. Examples of the viral vector include, but are not limited to, retroviruses, adenoviruses, and adeno-associated viruses.

Specifically, the vector may include plasmid DNA, phage DNA, and the like; and commercially developed plasmids (pUC18, pBAD, pIDTSAMRT-AMP, and the like), *E. coli-*derived plasmids (pYG601BR322, pBR325, pUC118, pUC119, and the like), *Bacillus subtilis-*derived plasmids (pUB110, pTP5, and the like), yeast-derived plasmids (YEp13, YEp24, YCp50, and the like), phage DNA (Charon4A, Charon21A, EMBL3, EMBL4, λ gt10, λ gt11, λ ZAP, and the like), animal viral vectors (retroviruses, adenoviruses, vaccinia viruses, and the like), insect viral vectors (baculoviruses and the like). Since the vector exhibits different expression levels and modification of a protein depending on a host cell, it is preferred to select and use a host cell which is most suitable for the purpose.

As used herein, the term "gene expression" or "expression" of a target protein is understood to mean transcription of DNA sequences, translation of mRNA transcripts, and secretion of fusion protein products or fragments thereof. A useful expression vector may be RcCMV (Invitrogen, Carlsbad) or a variant thereof. Expression vectors may further contain human cytomegalovirus (CMV) promoter for promoting continuous transcription of a target gene in mammalian cells, and a bovine growth hormone polyadenylation signal sequence for increasing the stability level of RNA after transcription.

### Transformed cell expressing fusion protein

In still yet another aspect of the present invention, there is provided a transformed cell into which the vector has been introduced. Host cells for the transformed cell may include, but are not limited to, prokaryotic cells, eukaryotic cells, and cells of mammalian, plant, insect, fungal, or bacterial origin. As an example of the prokaryotic cells, *E. coli* may be used. In addition, as an example of the eukaryotic cells, yeast may be used. In addition, for the mammalian cells, CHO cells, F2N cells, CSO cells, BHK cells, Bowes melanoma cells, HeLa cells, 911 cells, AT1080 cells, A549 cells, HEK 293 cells, HEK293T cells, or the like may be used. However, the mammalian cells are not limited thereto, and any cells which are known to those skilled in the art to be usable as mammalian host cells may be used.

In addition, for the introduction of an expression vector into the host cell, CaCl₂ precipitation, Hanahan method whose efficiency has been increased efficiency by using a reducing agent such as dimethyl sulfoxide (DMSO) in CaCl₂ precipitation, electroporation, calcium phosphate precipitation, protoplast fusion, agitation using silicon carbide fiber, Agrobacterium-mediated transformation, transformation using PEG, dextran sulfate-, Lipofectamine-, or dry/inhibition-mediated transformation, or the like may be used.

As described above, for optimization of properties of a fusion protein as a therapeutic agent or for any other purpose, glycosylation pattern of the fusion protein (for example, sialic acids, fucosylations, glycosylations) may be adjusted by manipulating, through methods known to those skilled in the art, glycosylation-related genes possessed by host cells.

### Method for producing a fusion protein

In still yet another aspect of the present invention, there is provided a method for producing a fusion protein comprising an IL-2 protein and a CD80 protein fragment or a variant thereof, the method comprising culturing the transformed cells. Specifically, the production method may comprise i) culturing the transformed cells to obtain a culture; and ii) collecting the fusion protein from the culture.

Culturing the transformed cells may be carried out using methods well known in the art. Specifically, the culture may be carried out in a batch process, or carried out continuously in a fed batch or repeated fed batch process.

### Use of fusion protein or dimer thereof

In still yet another aspect of the present invention, there is provided a pharmaceutical composition for treating or preventing cancer or an infectious disease, the composition comprising, as an active ingredient, a fusion protein comprising an IL-2 protein and a CD80 protein fragment or a variant thereof, or a fusion protein dimer where the two fusion proteins are attached.

The fusion protein comprising an IL-2 protein and a CD80 protein fragment or a variant thereof, or the fusion protein dimer where the two fusion proteins are attached is as described above.

The cancer may be selected from the group consisting of gastric cancer, liver cancer, lung cancer, colorectal cancer, breast cancer, prostate cancer, ovarian cancer, pancreatic cancer, cervical cancer, thyroid cancer, laryngeal cancer, acute myeloid leukemia, brain tumor, neuroblastoma, retinoblastoma, head and neck cancer, salivary gland cancer, and lymphoma. In addition, the infectious disease may be any one selected from the group consisting of hepatitis B, hepatitis C, human papilloma virus (HPV) infection, cytomegalovirus infection, viral respiratory disease, and influenza.

A preferred dose of the pharmaceutical composition varies depending on the patient's condition and body weight, severity of disease, form of drug, route and duration of administration and may be appropriately selected by those skilled in the art. In the pharmaceutical composition for treating or preventing cancer or an infectious disease of the present invention, the active ingredient may be contained in any amount (effective amount) depending on application, dosage form, blending purpose, and the like, as long as the active ingredient can exhibit anticancer activity or a therapeutic effect on an infectious disease. A conventional effective amount thereof will be determined within a range of 0.001 wt% to 20.0 wt% by weight, based on the total weight of the composition. Here, the term "effective amount" refers to an amount of an active ingredient capable of inducing an anticancer effect or an infectious disease-treating effect. Such an effective amount can be experimentally determined within the scope of common knowledge of those skilled in the art.

As used herein, the term "treatment" may be used to mean both therapeutic and prophylactic treatment. Here, prophylaxis may be used to mean that a pathological condition or disease of an individual is alleviated or mitigated. In an embodiment, the term "treatment" includes both application and any form of administration for treating a disease in a mammal, including a human. In addition, the term includes inhibiting or slowing down a disease or disease progression; and includes meanings of restoring or repairing impaired or lost function so that a disease is partially or completely alleviated; stimulating inefficient processes; or alleviating a serious disease.

As used herein, the term "efficacy" refers to capacity that can be determined by one or parameters, for example, survival or disease-free survival over a certain period of time such as one year, five years, or ten years. In addition, the parameter may include inhibition of size of at least one tumor in an individual.

Pharmacokinetic parameters such as bioavailability and underlying parameters such as clearance rate may also affect efficacy. Thus, "enhanced efficacy" (for example, improvement in efficacy) may be due to enhanced pharmacokinetic parameters and improved efficacy, which may be measured by comparing clearance rate and tumor growth in test animals or human subjects, or by comparing parameters such as survival, recurrence, or disease-free survival.

As used herein, the term "therapeutically effective amount" or "pharmaceutically effective amount" refers to an amount of a compound or composition effective to prevent or treat the disease in question, which is sufficient to treat the disease at a reasonable benefit/risk ratio applicable to medical treatment and does not cause adverse effects. A level of the effective amount may be determined depending on factors including the patient's health condition, type and severity of disease, activity of drug, the patient's sensitivity to drug, mode of administration, time of administration, route of administration and excretion rate, duration of treatment, formulation or simultaneously used drugs, and other factors well known in the medical field. In an embodiment, the therapeutically effective amount means an amount of drug effective to treat cancer.

Here, the pharmaceutical composition may further comprise a pharmaceutically acceptable carrier. The pharmaceutically acceptable carrier may be any carrier as long as the carrier is a non-toxic substance suitable for delivery to a patient. Distilled water, alcohol, fat, wax, and inert solid may be contained as the carrier. A pharmaceutically acceptable adjuvant (buffer, dispersant) may also be contained in the pharmaceutical composition.

Specifically, by including a pharmaceutically acceptable carrier in addition to the active ingredient, the pharmaceutical composition may be prepared into a parenteral formulation depending on its route of administration using conventional methods known in the art. Here, the term "pharmaceutically acceptable" means that the carrier does not have more toxicity than the subject to be applied (prescribed) can adapt while not inhibiting activity of the active ingredient.

When the pharmaceutical composition is prepared into a parenteral formulation, it may be made into preparations in the form of injections, transdermal patches, nasal inhalants, or suppositories with suitable carriers according to methods known in the art. In a case of being made into injections, sterile water, ethanol, polyol such as glycerol or propylene glycol, or a mixture thereof may be used as a suitable carrier; and an isotonic solution, such as Ringer's solution, phosphate buffered saline (PBS) containing triethanol amine or sterile water for injection, and 5% dextrose, or the like may preferably be used. Formulation of pharmaceutical compositions is known in the art, and reference may specifically be made to Remington's Pharmaceutical Sciences (19th ed., 1995) and the like. This document is considered part of the present description.

A preferred dose of the pharmaceutical composition may range from 0.01 µg/kg to 10 g/kg, or 0.01 mg/kg to 1 g/kg, per day, depending on the patient's condition, body weight, sex, age, severity of the patient, and route of administration. The dose may be administered once a day or may be divided into several times a day. Such a dose should not be construed as limiting the scope of the present invention in any aspect.

Subjects to which the pharmaceutical composition can be applied (prescribed) are mammals and humans, with humans being particularly preferred. In addition to the active ingredient, the pharmaceutical composition of the present application may further contain any compound or natural extract, which has already been validated for safety and is known to have anticancer activity or a therapeutic effect on an infectious disease, so as to boost or reinforce anticancer activity.

In still yet another aspect of the present invention, there is provided a use of a fusion protein comprising an IL-2 protein and a CD80 protein fragment or a variant thereof for treating cancer or an infectious disease.

In still yet another aspect of the present invention, there is provided a use of a fusion protein comprising an IL-2 protein and a CD80 protein fragment or a variant thereof for enhancing a therapeutic effect on cancer or an infectious disease.

In still yet another aspect of the present invention, there is provided a use of a fusion protein comprising an IL-2 protein and a CD80 protein fragment or a variant thereof for manufacture of a medicament for treating cancer or an infectious disease.

In still yet another aspect of the present invention, there is provided a method for treating cancer or an infectious disease, and/or a method for enhancing a therapeutic effect on cancer or an infectious disease, comprising administering, to a subject, a fusion protein comprising an IL-2 protein and a CD80 protein fragment or a variant thereof, or a fusion protein dimer where the two fusion proteins are attached.

The subject may be an individual suffering from cancer or an infectious disease. In addition, the subject may be a mammal, preferably a human. The fusion protein comprising an IL-2 protein and a CD80 protein fragment or a variant thereof, or the fusion protein dimer where the two fusion proteins are attached is as described above.

Route of administration, dose, and frequency of administration of the fusion protein or fusion protein dimer may vary depending on the patient's condition and the presence or absence of side effects, and thus the fusion protein or fusion protein dimer may be administered to a subject in various ways and amounts. The optimal administration method, dose, and frequency of administration can be selected in an appropriate range by those skilled in the art. In addition, the fusion protein or fusion protein dimer may be administered in combination with other drugs or physiologically active substances whose therapeutic effect is known with respect to a disease to be treated, or may be formulated in the form of combination preparations with other drugs.

Due to IL-2 activity, the fusion protein in an embodiment of the present invention can activate immune cells such as natural killer cells. Thus, the fusion protein can be effectively used for cancer and infectious diseases. In particular, it was identified that as compared with the wild type, an IL-2 variant with two to five amino acid substitutions, in particular, an IL-2 variant that contains amino acid substitutions at two, three, four, or five positions among the positions selected from the group consisting of R38A, F42A, Y45A, E61R, and L72G in an amino acid sequence of SEQ ID NO: 10, has low binding ability for the IL-2 receptor alpha chain and thus exhibits improved characteristics with respect to pharmacological side effects of conventional IL-2. Thus, such an IL-2 variant, when used alone or in the form of a fusion protein, can decrease incidence of vascular (or capillary) leakage syndrome (VLS), a problem with IL-2 conventionally known.

### Mode for Carrying out the Invention

Hereinafter, the present invention will be described in more detail by way of the following examples. However, the following examples are only for illustrating the present invention, and the scope of the present invention is not limited thereto.

### Preparation Example 1. Preparation of hCD80 variant-Fc-IL-2 variant: GI-101_P1 to GI-101_P35

### Preparation Example 1.1. Construction of hCD80 variant-Fc-IL-2 variant expression vector

In order to produce a fusion protein comprising a human CD80 variant, an Fc domain, and an IL-2 variant, a total of 35 polynucleotides were synthesized through the Invitrogen GeneArt Gene Synthesis service of ThermoFisher Scientific. Specifically, the polynucleotide contains a nucleotide sequence which encodes a fusion protein that contains a signal peptide, a CD80 protein fragment, an Ig hinge, an Fc domain, a linker, and an IL-2 variant (2M) (R38A, F42A) having two amino acid substitutions, in this order, from the N-terminus. The polynucleotide was inserted into pcDNA3_4 vector.

The constructions of the hCD80 variant-Fc-IL-2 variant (GI-101_P1 to GI-101_P35) fusion proteins are summarized in Table 1.

**[Table 1]**

| **Code** # | **Construction** | | | | |
|---|---|---|---|---|---|
| | 1^{st} API (hCD80 variant) | 1^{st} Linker | IgG Fc | 2^{nd} Linker | 2^{nd} API (hIL2v_2M) |
| GI-101_P1 | V domain - Loop - C domain (A-B-C-D-E-F-G) | Fixed | Fixed | Fixed | Fixed |
| GI-101_P2 | V domain - Loop - C domain (A-B-C-D-E-F-) | | | | |
| GI-101 P3 | V domain - Loop - C domain (A-B-C-D-E-F) | | | | |
| GI-101 P4 | V domain - Loop - C domain (A-B-C-D-E-) | | | | |
| GI-101 P5 | V domain - Loop - C domain (A-B-C-D-E) | | | | |
| GI-101 P6 | V domain - Loop - C domain (A-B-C-D-) | | | | |
| GI-101_P7 | V domain - Loop - C domain (A-B-C-D) | | | | |
| GI-101 P8 | V domain - Loop - C domain (A-B-C-) | | | | |
| GI-101_P9 | V domain (A-B-a₁-C-C'-C"-a₂D-E-a₃E-F-G-) | | | | |
| GI-101_P10 | V domain (-B-a₁-C-C'-C"-a₂D-E-a₃E-F-G-) | | | | |
| GI-101_P11 | V domain (B-a₁-C-C'-C"-a₂D-E-a₃E-F-G-) | | | | |
| GI-101_P12 | V domain (a₁-C-C'-C"-a₂D-E-a₃E-F-G-) | | | | |
| GI-101_P13 | V domain (C-C'-C"-a₂D-E-a₃E-F-G-) | | | | |
| GI-101_P14 | V domain (C-C'-C"-a₂D-E-a₃E-F-G) | | | | |
| GI-101_P15 | V domain (C'-C"-a₂D-E-a₃E-F-G-) | | | | |
| GI-101_P16 | V domain (C-C'-C") | | | | |
| GI-101_P17 | V domain (C-C') | | | | |
| GI-101_P18 | V domain (F-G) | | | | |
| GI-101_P19 | Fixed | Fixed | Fixed | - | Fixed |
| GI-101_P20 | | | | EAAAK | |
| GI-101_P21 | | | | (EAAAK)₂ | |
| GI-101_P22 | | | | (EAAAK)₄ | |
| GI-101_P23 | | | | (GGGGS)₂ | |
| GI-101_P24 | | | | (GGGGS)₄ | |
| GI-101_P25 | | - | | Fixed | |
| GI-101_P26 | | EAAAK | | | |
| GI-101_P27 | | (EAAAK)₂ | | | |
| GI-101_P28 | | (EAAAK)₄ | | | |
| GI-101_P29 | | GGGGS | | | |
| GI-101_P30 | | (GGGGS)₂ | | | |
| GI-101_P31 | | (GGGGS)₄ | | | |
| GI-101_P32 | | Fixed | Deletion | (GGGGS)₁ | |
| GI-101_P33 | | | | (GGGGS)₈ | |
| GI-101_P34 | | (GGGGS)₁ | Deletion | | |
| GI-101_P35 | | (GGGGS)₈ | | | |

### Preparation Example 1.2. Purification of hCD80 variant-Fc-IL-2 variant

The vector prepared in Preparation Example 1.1. above was introduced into CHO cells (Expi-CHO^{™}) to express each fusion protein. After the vector was introduced, culture was performed for 7 days in an environment of 37°C, 125 RPM, and 5% CO₂ concentration. Then, the culture was harvested, and the fusion protein was purified therefrom. The purified fusion proteins were designated "GI-101_P1 to GI-101_P35".

### Preparation Example 1.2.1. Purification of GI-101_P1 to GI-101_P31

GI-101_P1 to GI-101_P31 were purified using chromatography containing BAKEROND^{™} PROchievA^{™}. The fusion protein was bound thereto under a condition of 50 mM NaPi, 50 mM NaCl, pH 7. Thereafter, washing was performed under a condition of 50 mM NaPi, 500 mM NaCl, pH 7 (or 200 mM Glycine, 300 mM NaCl, pH 5). Then, elution was performed under a condition of 100 or 200 mM Glycine, 300 mM NaCl, pH 3 (or 100 mM Glycine, 300 mM NaCl, 500 mM Arginine, pH 4). 0.3 N NaOH was placed in a collection tube, and then the fusion protein was collected.

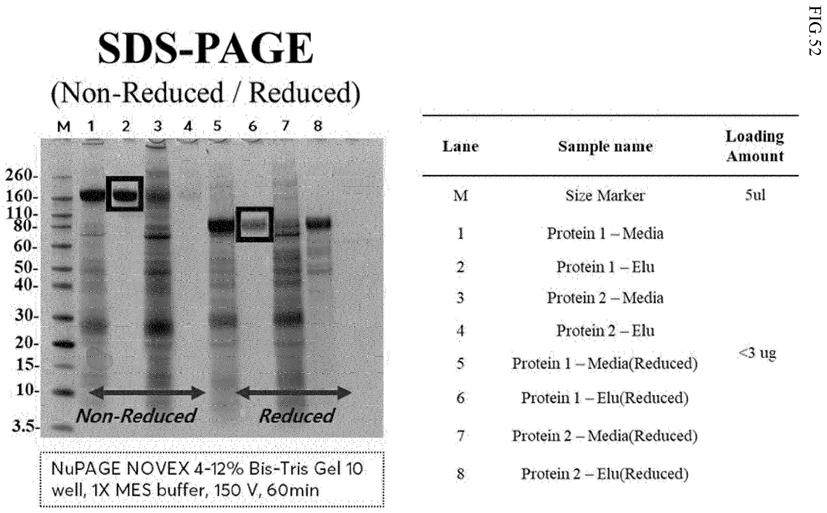

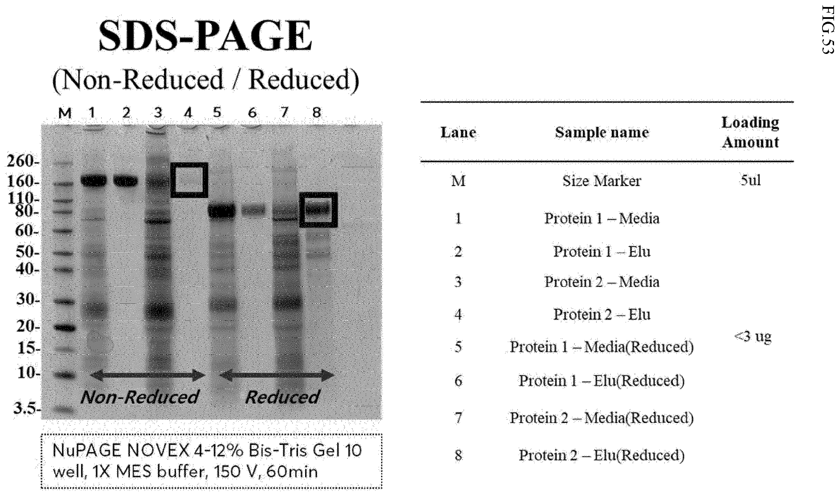

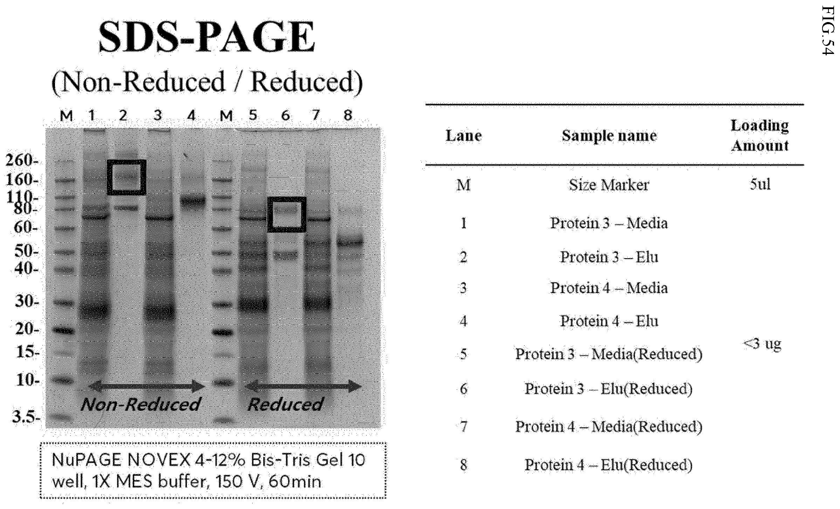

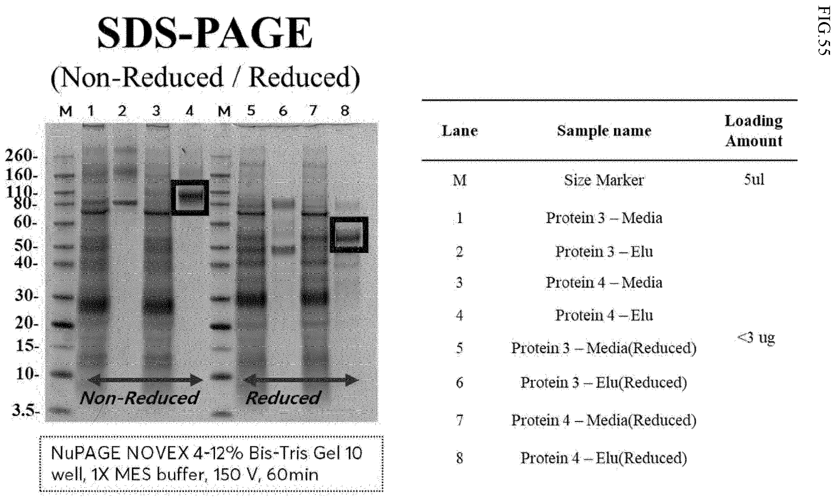

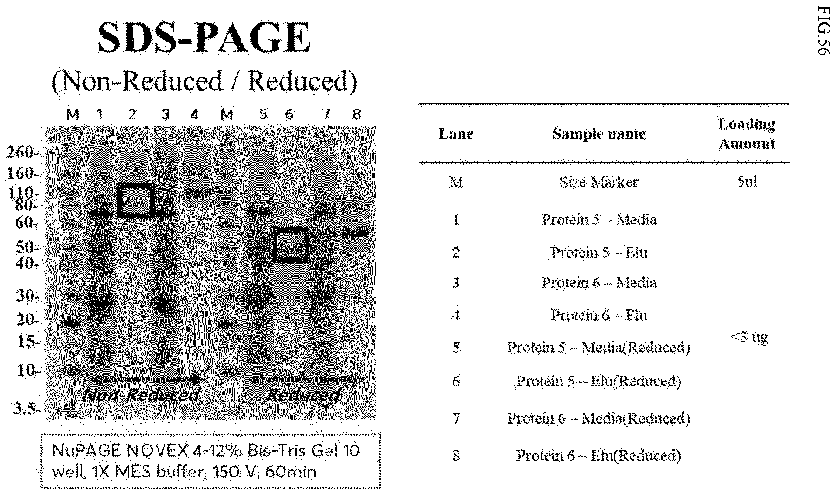

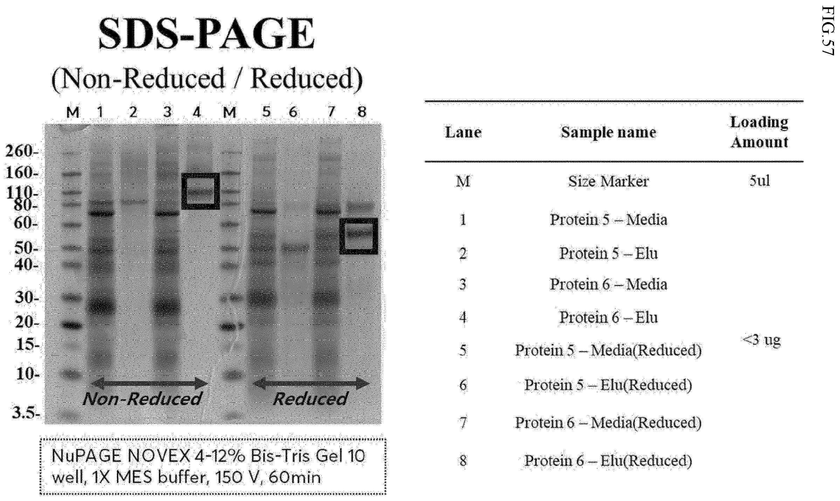

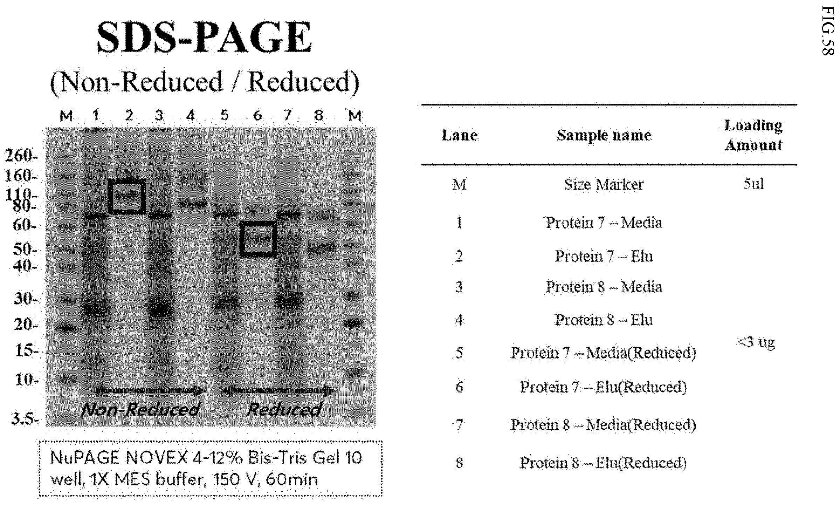

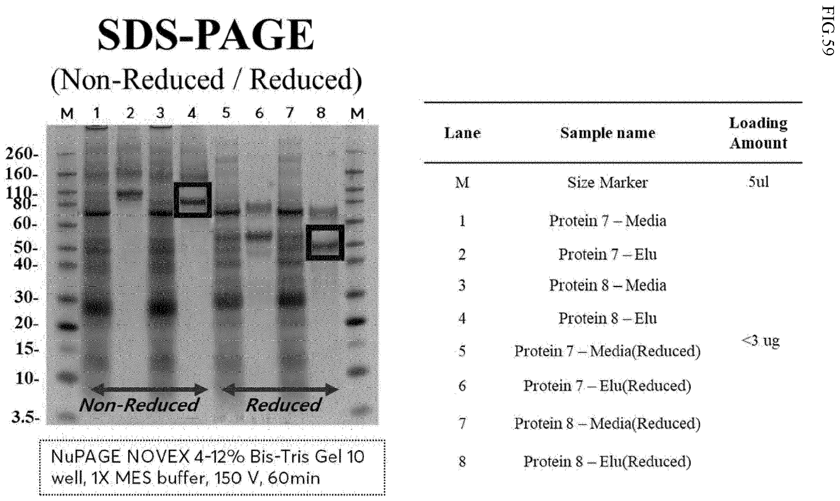

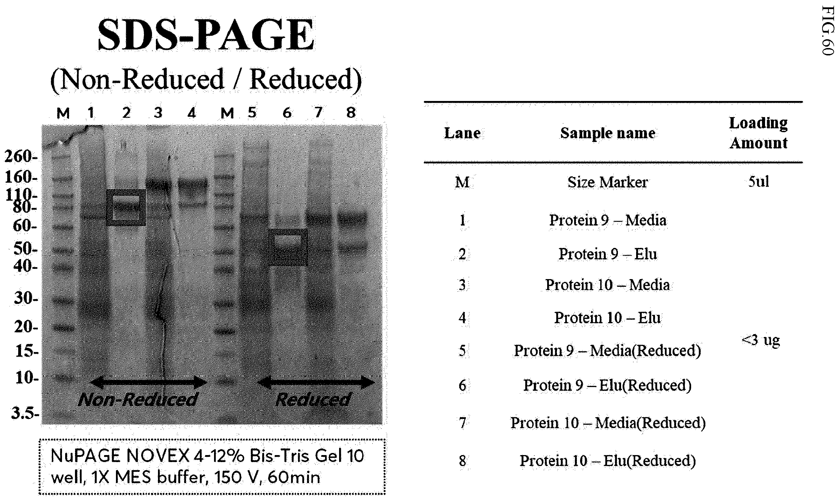

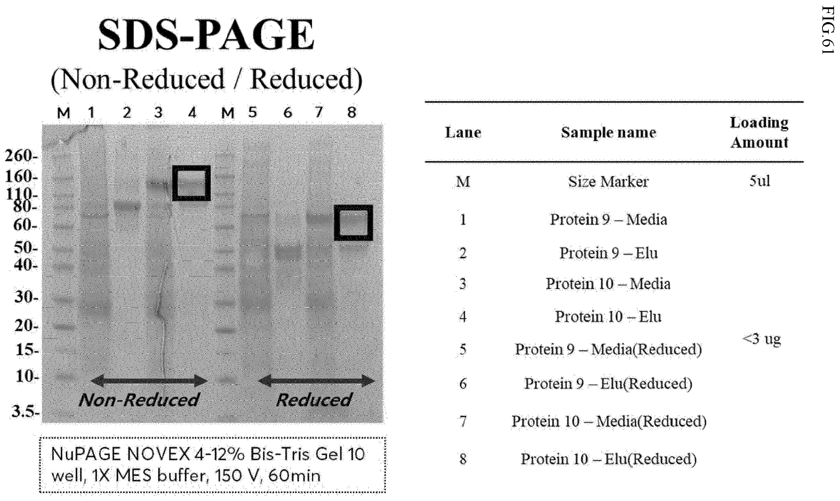

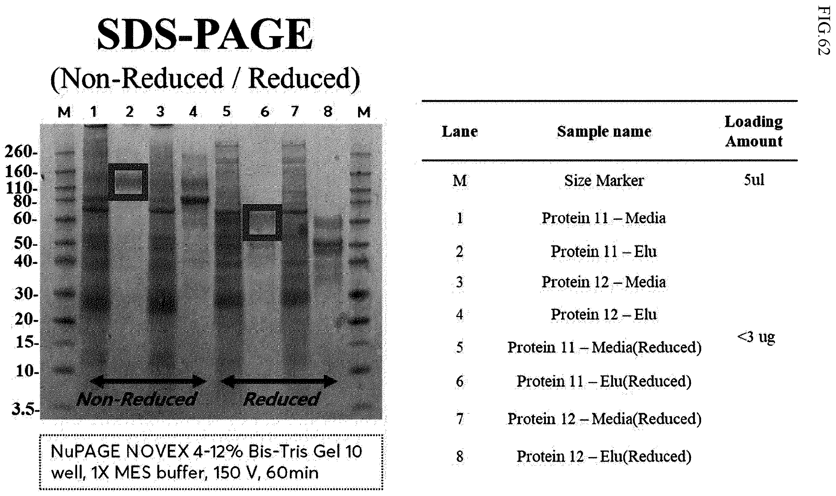

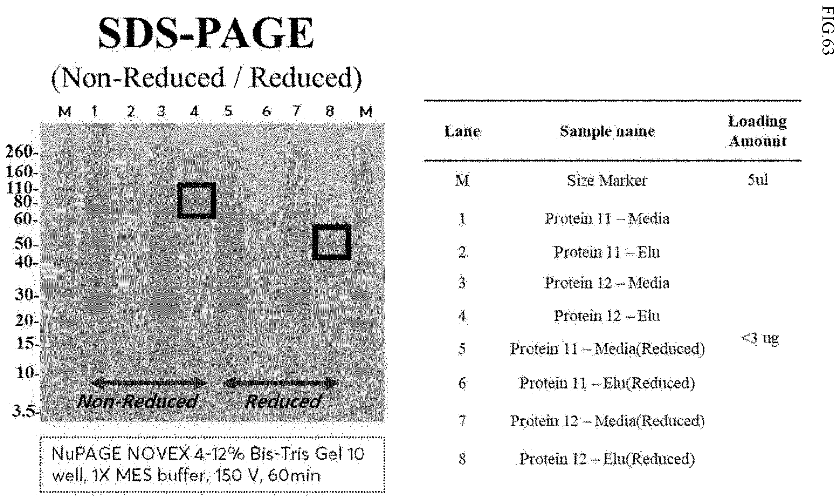

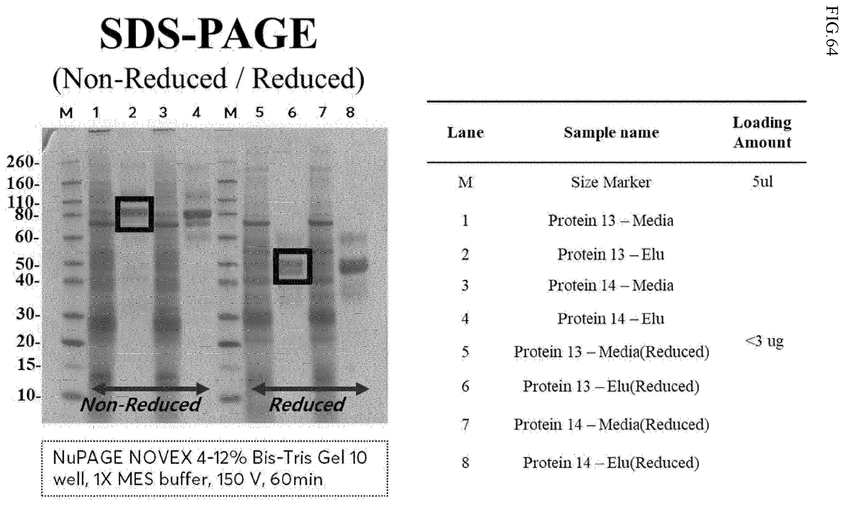

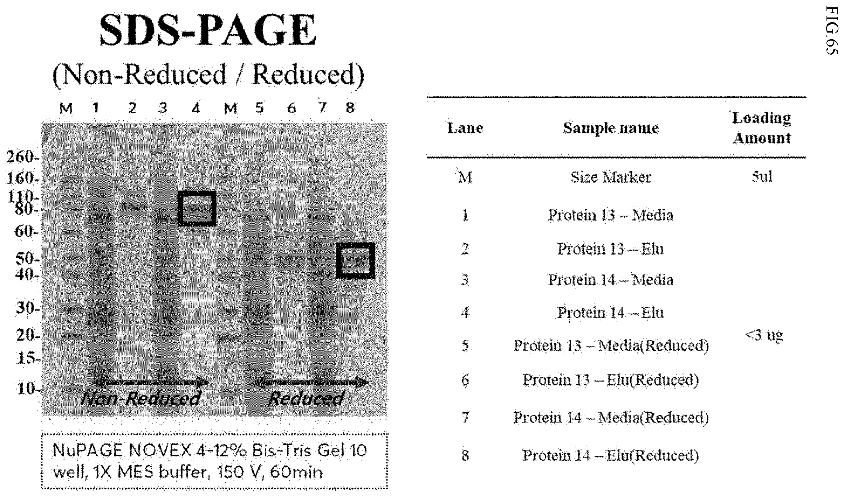

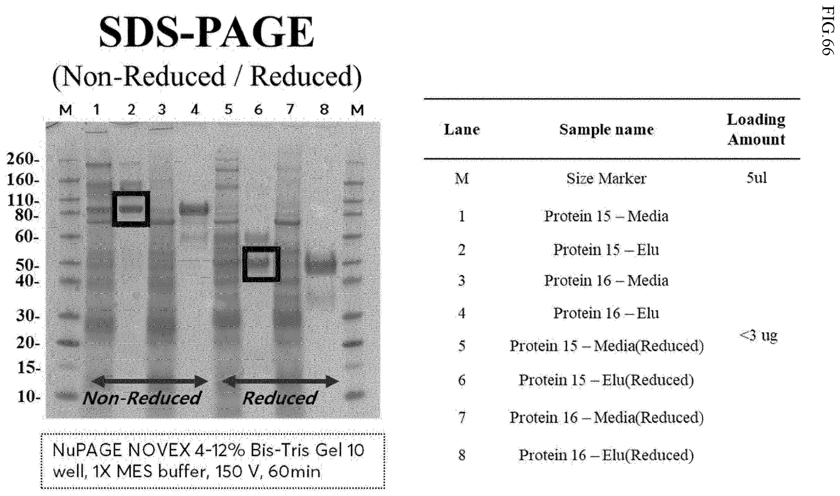

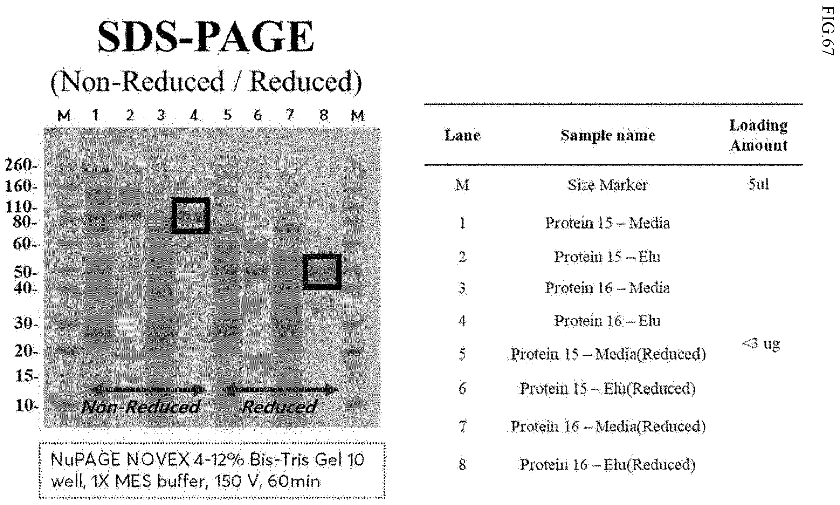

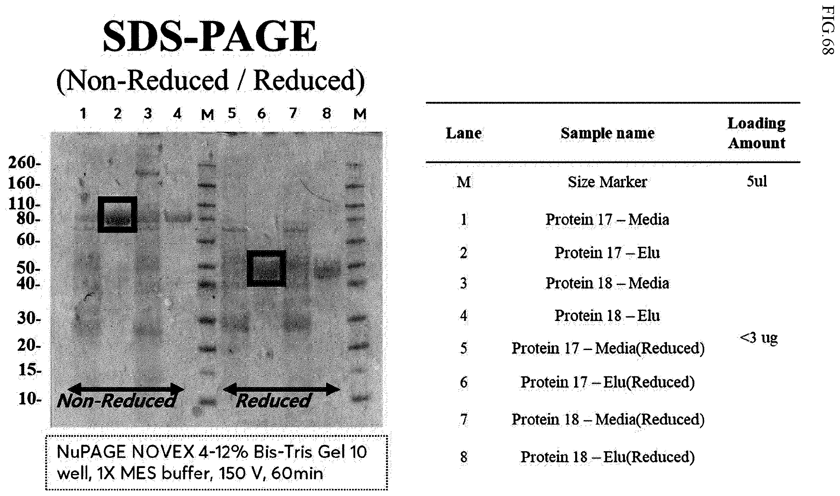

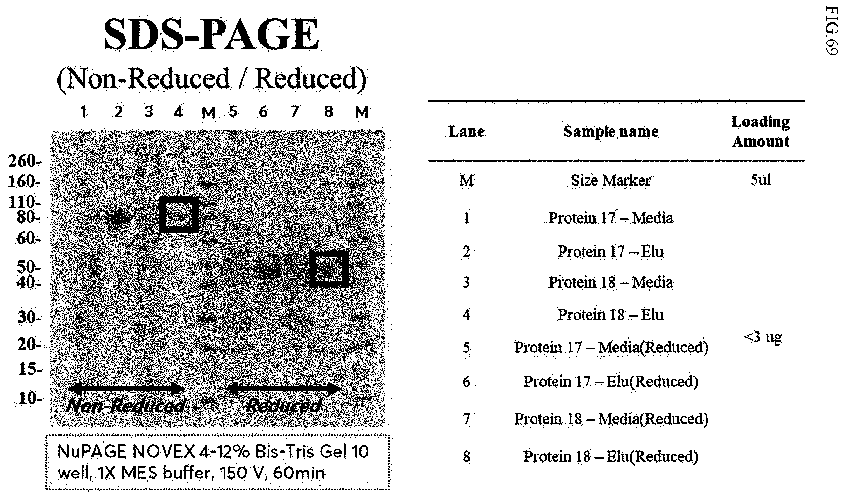

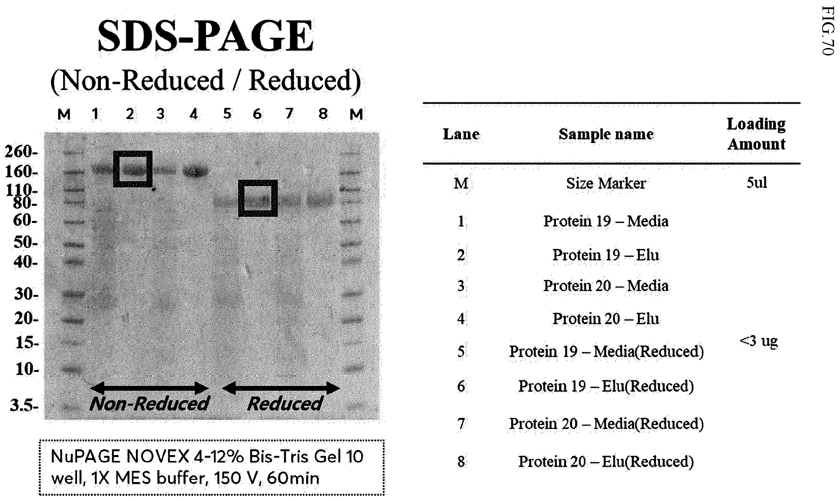

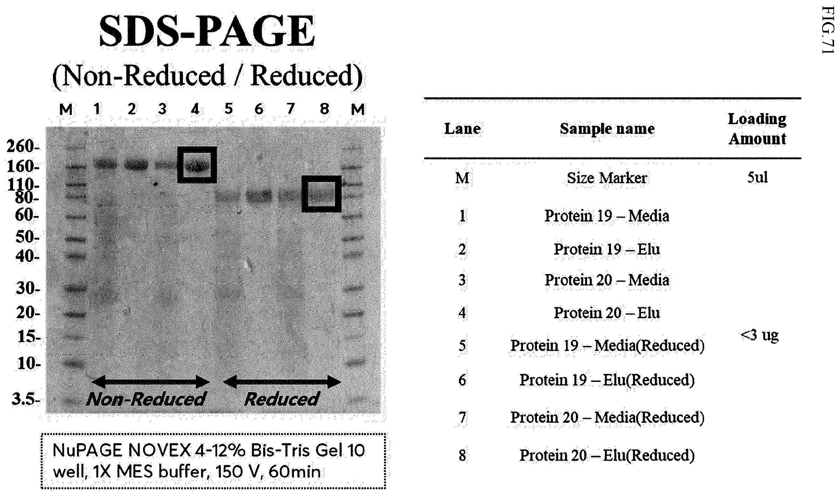

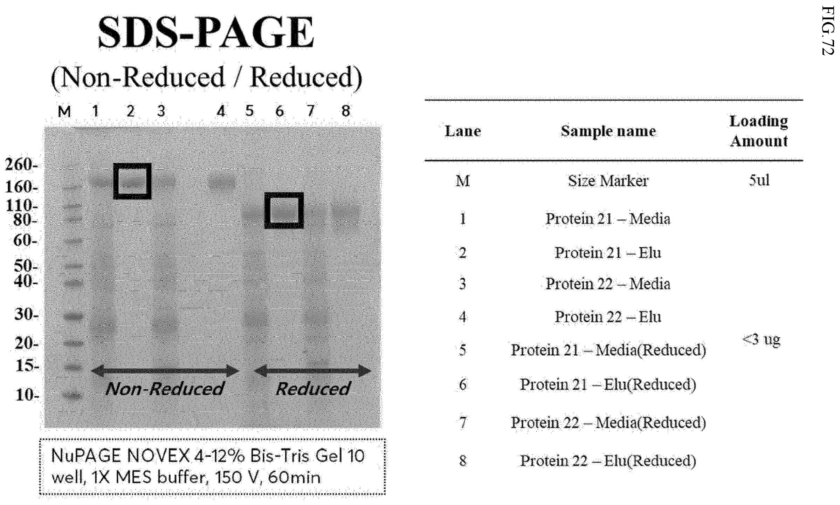

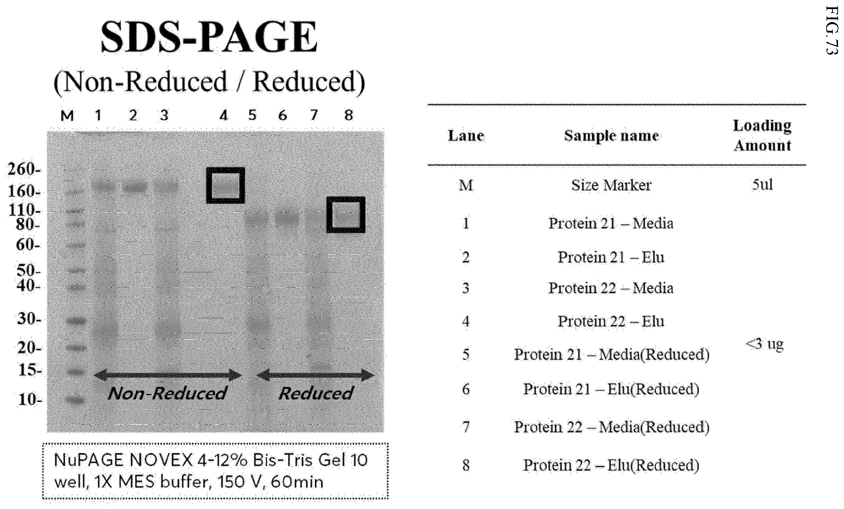

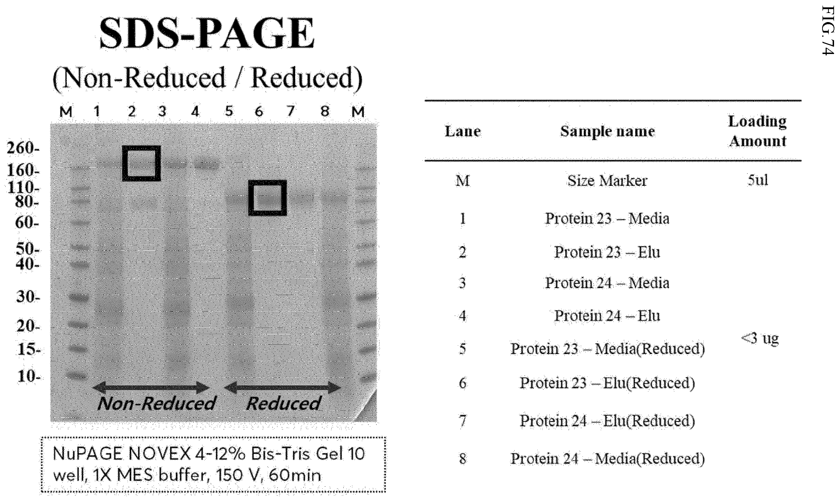

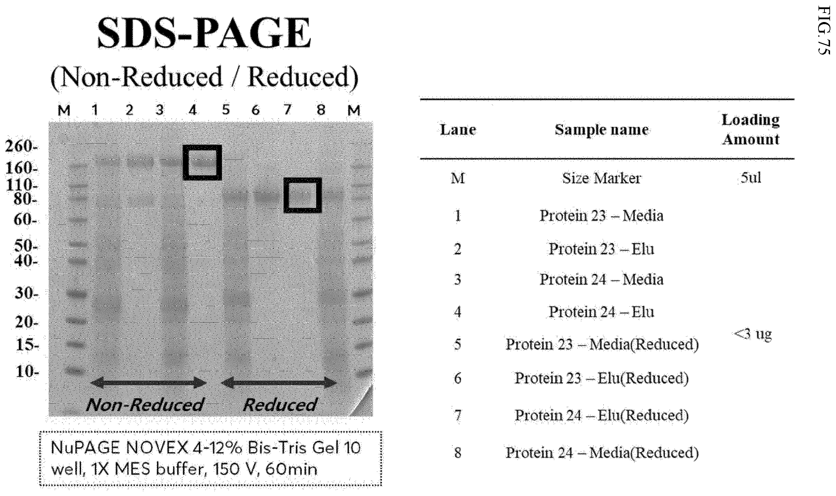

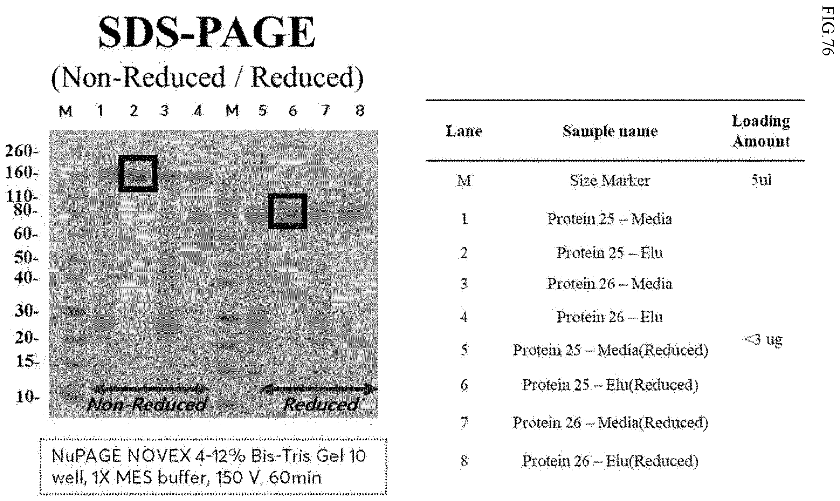

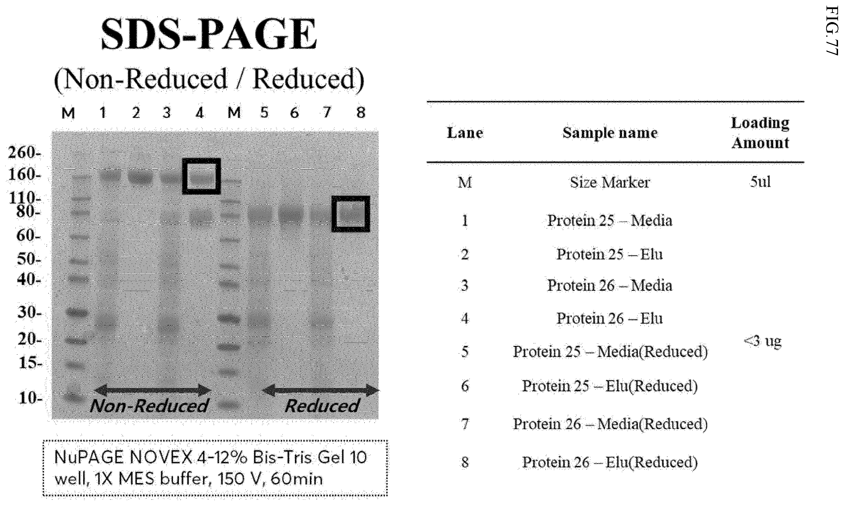

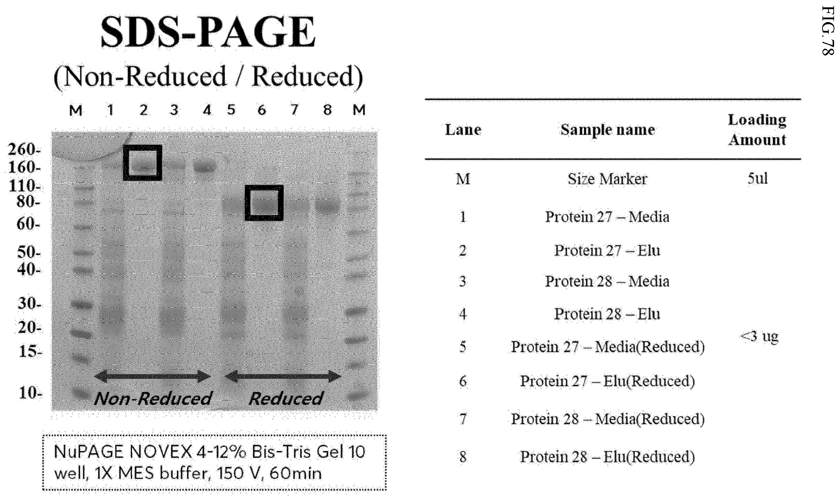

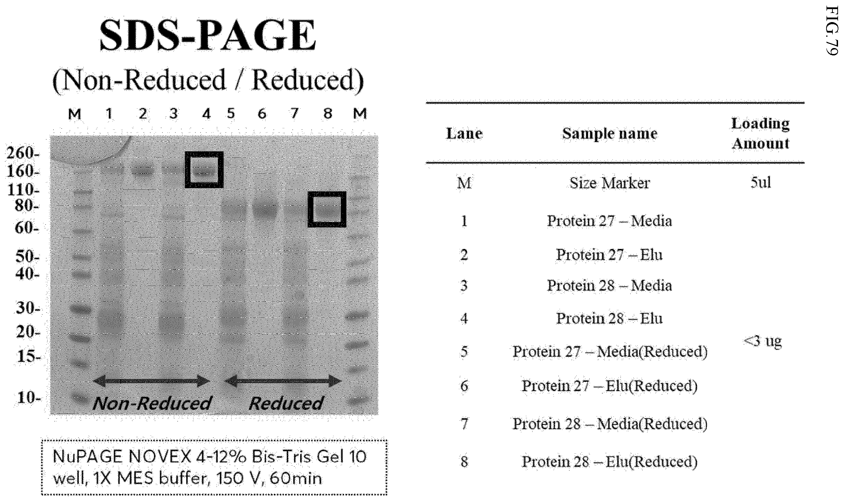

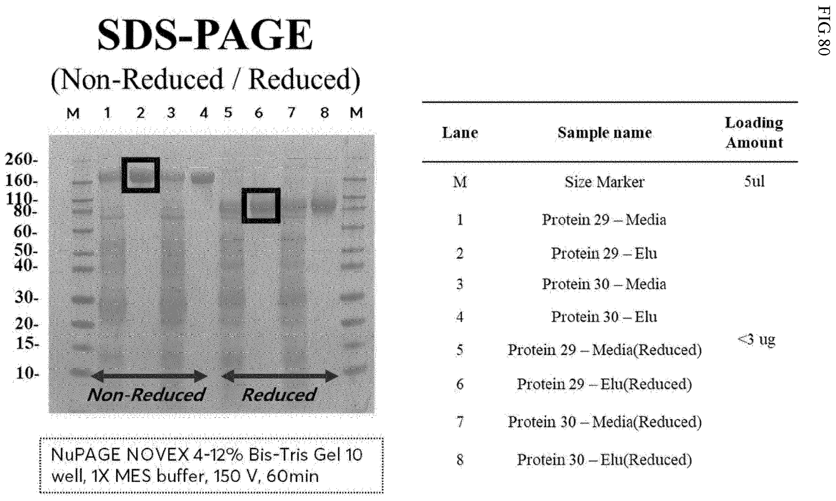

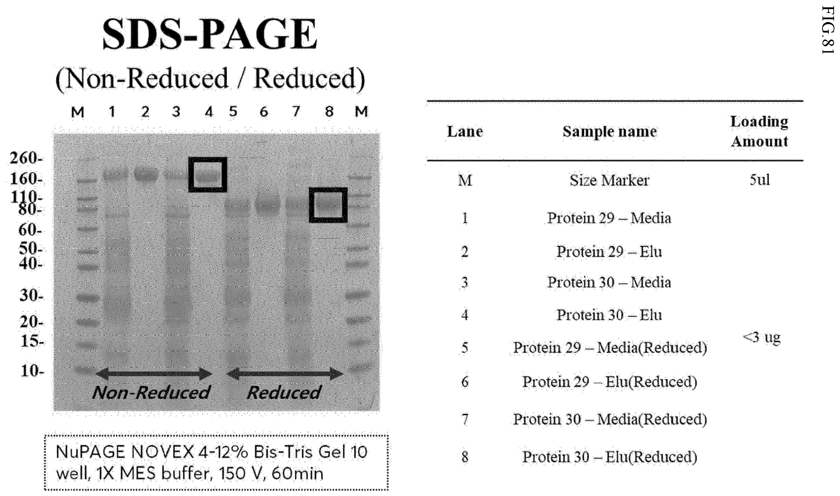

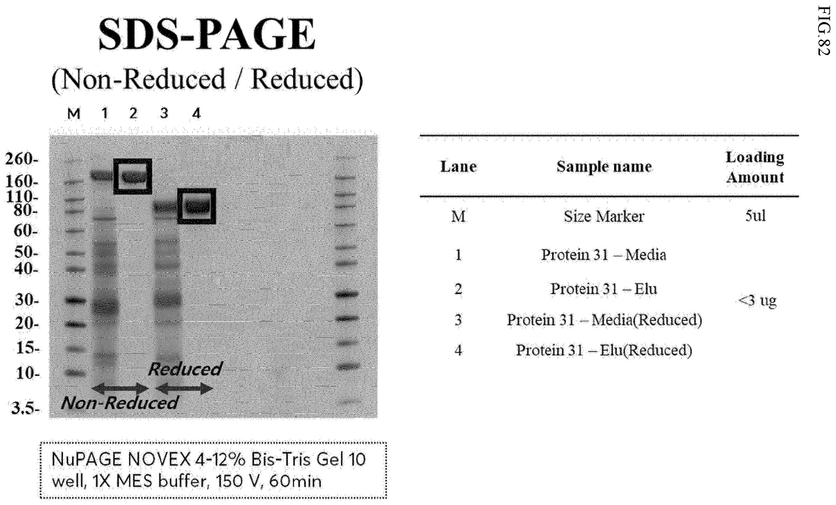

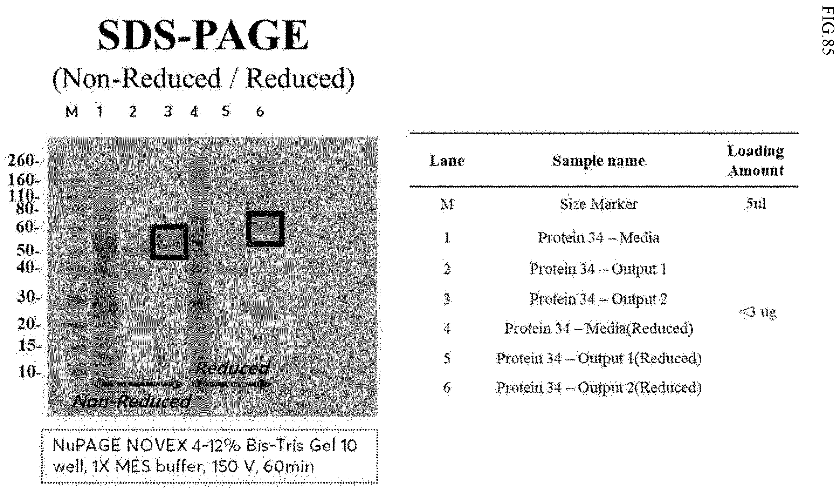

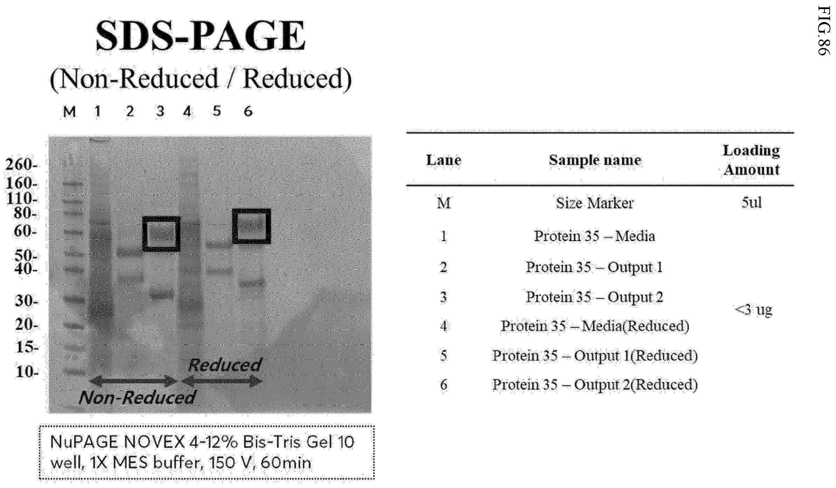

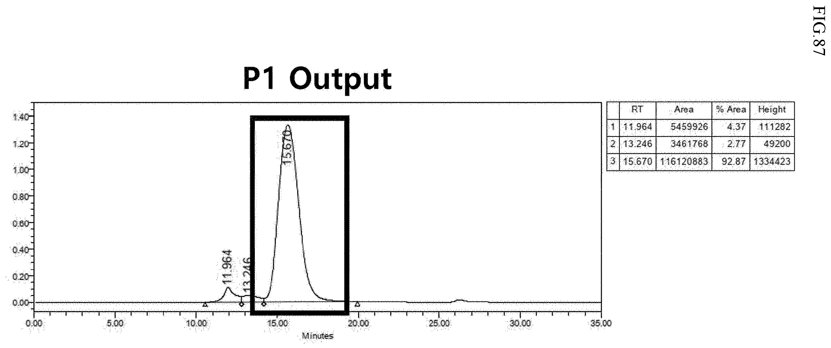

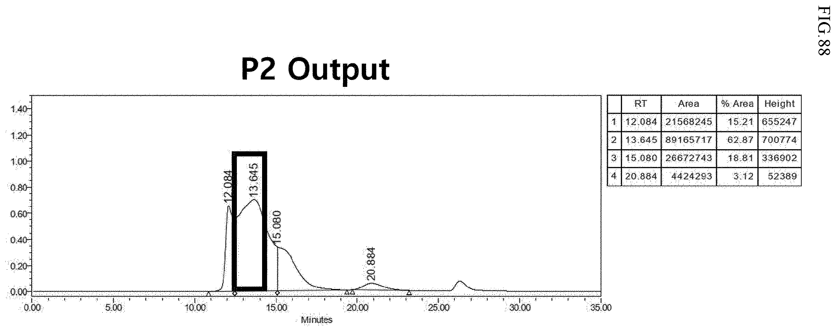

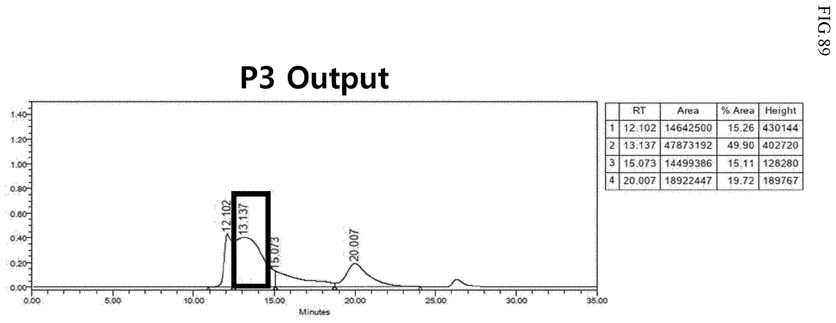

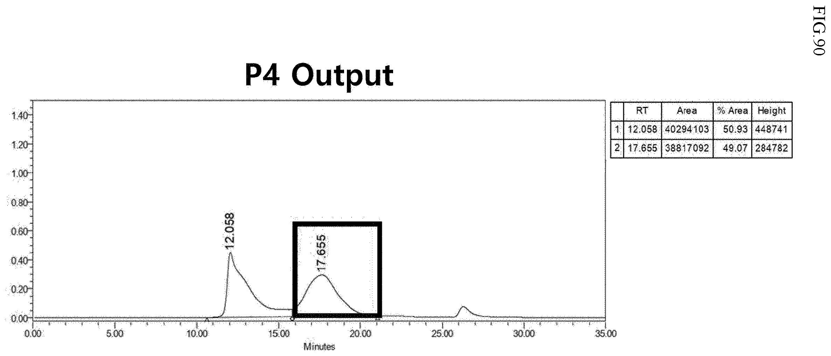

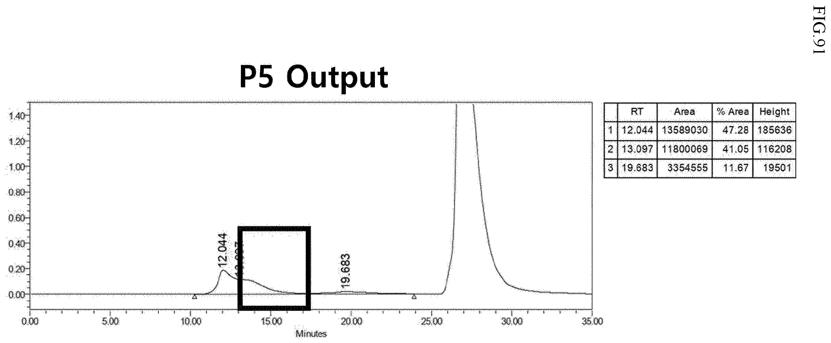

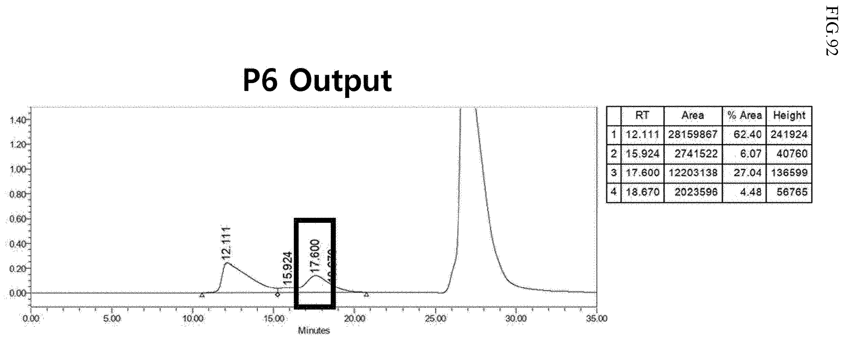

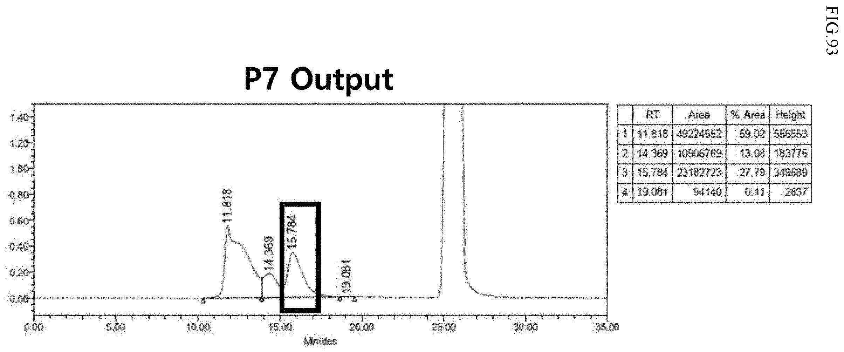

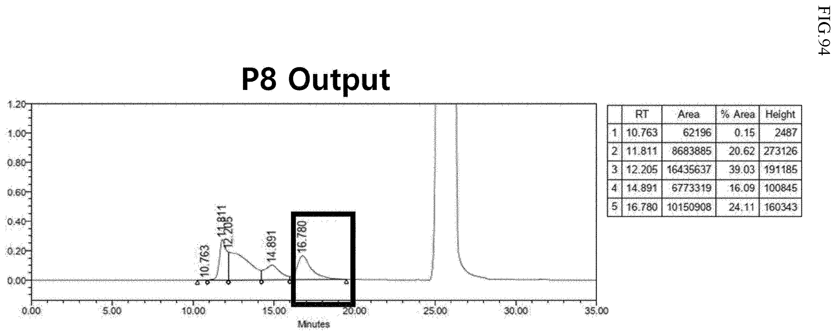

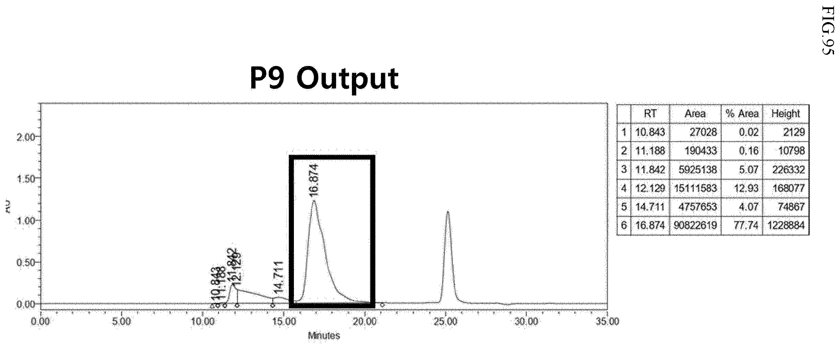

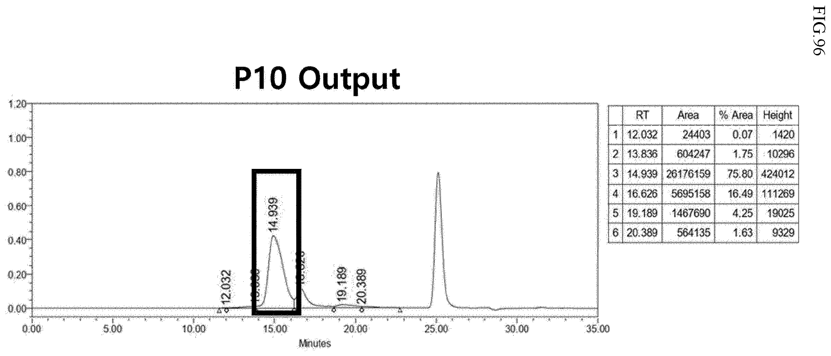

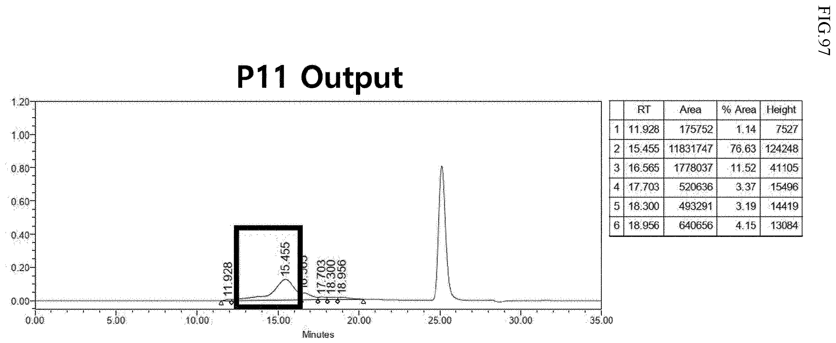

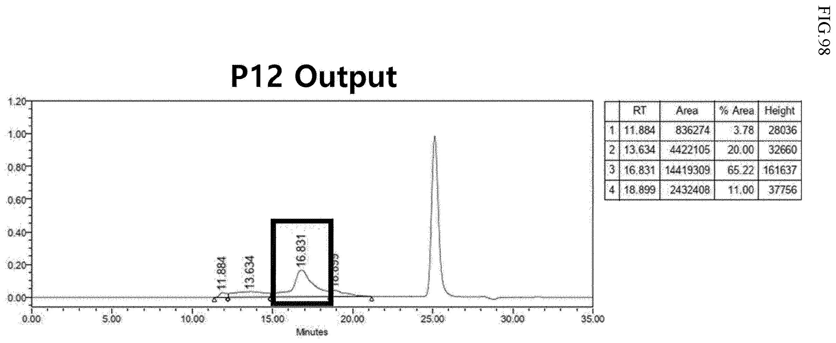

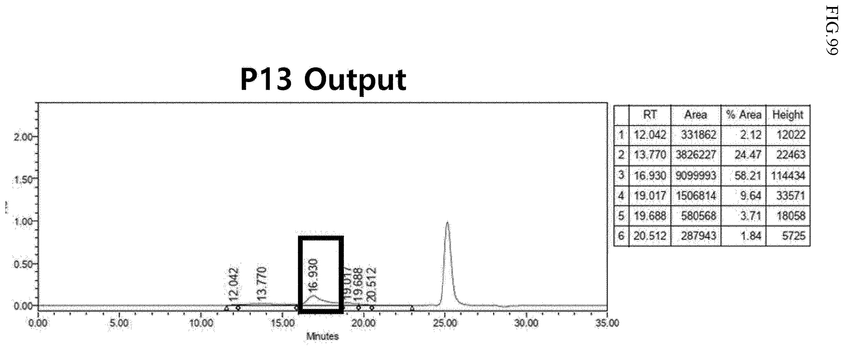

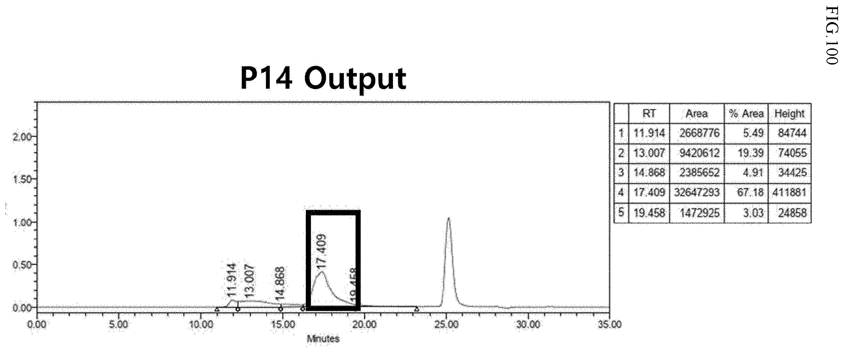

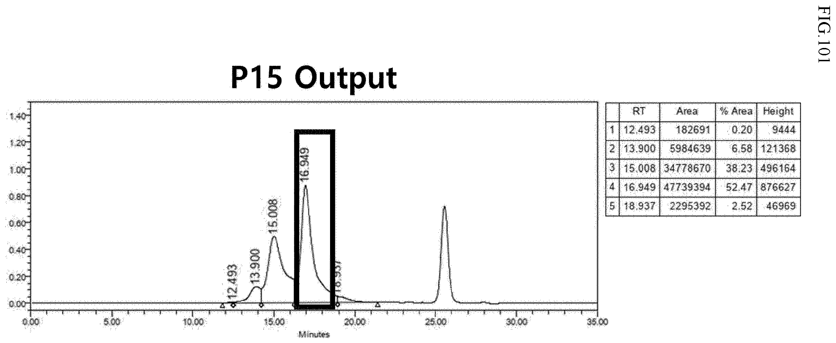

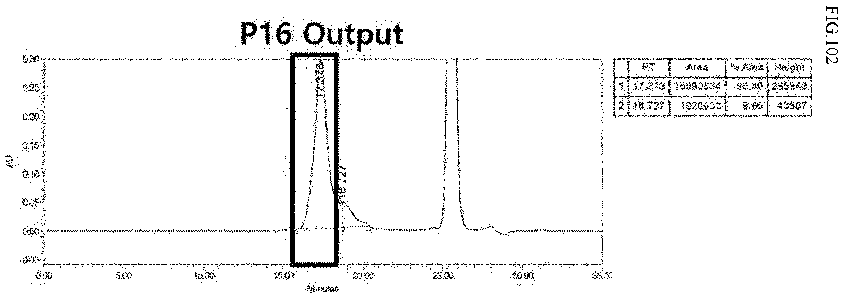

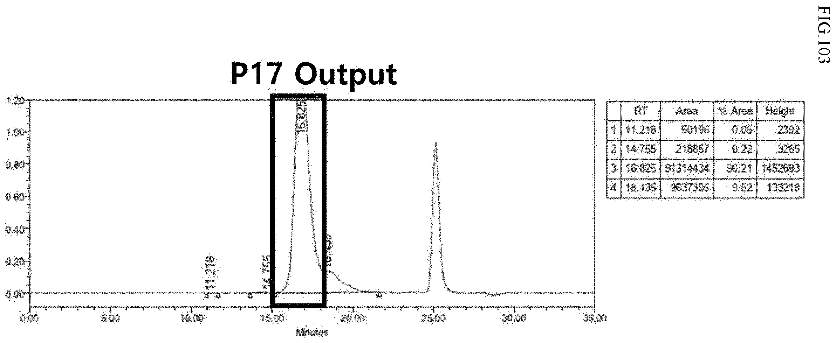

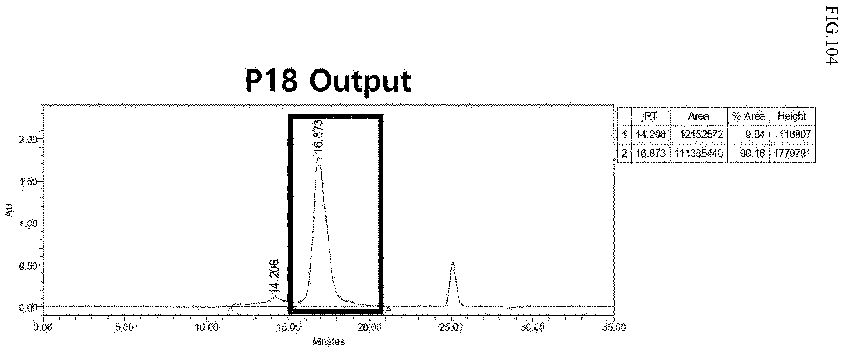

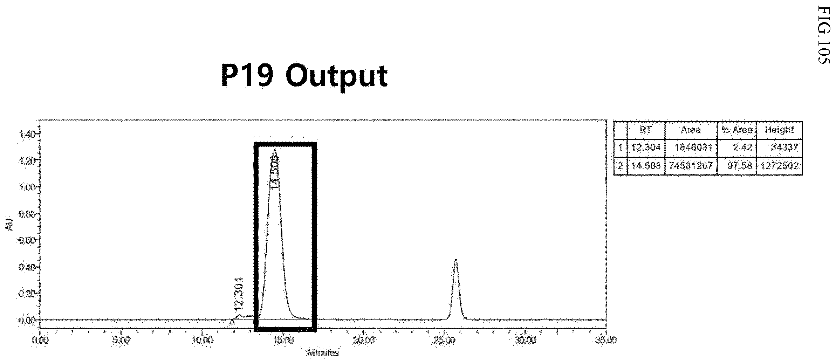

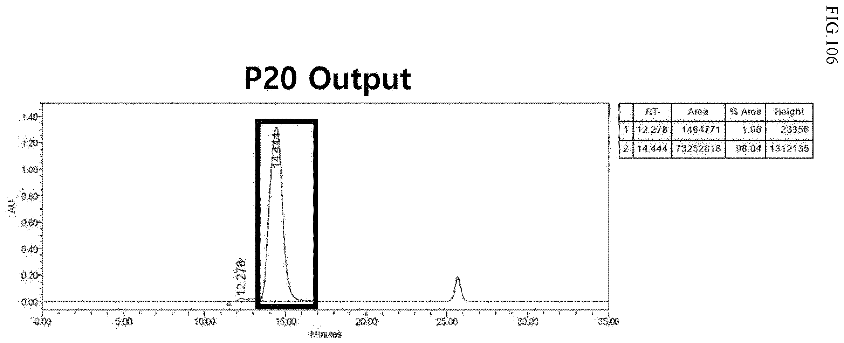

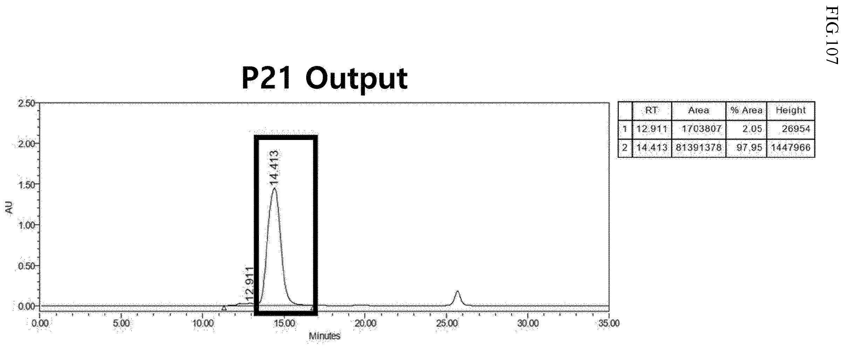

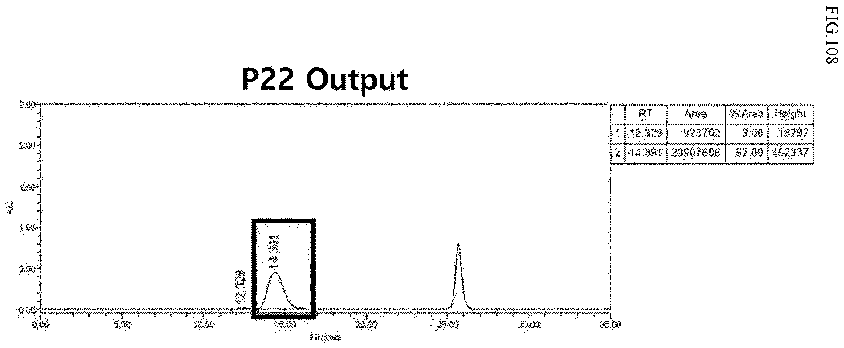

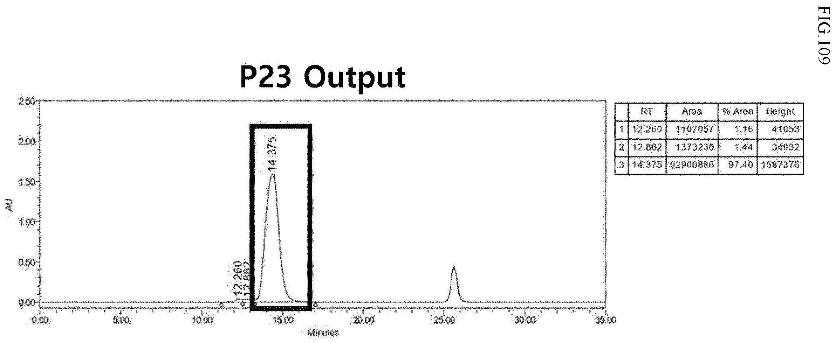

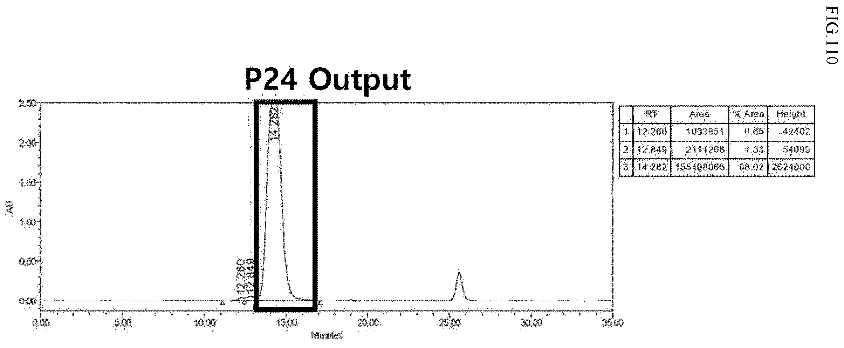

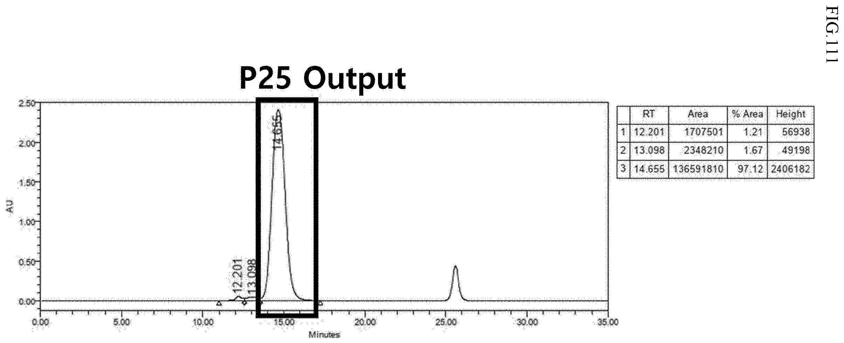

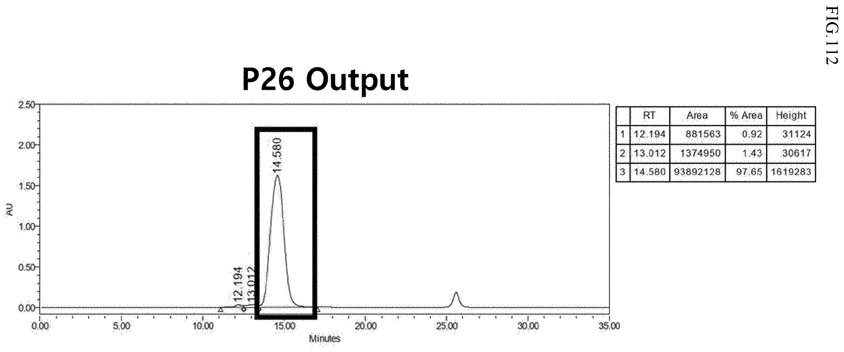

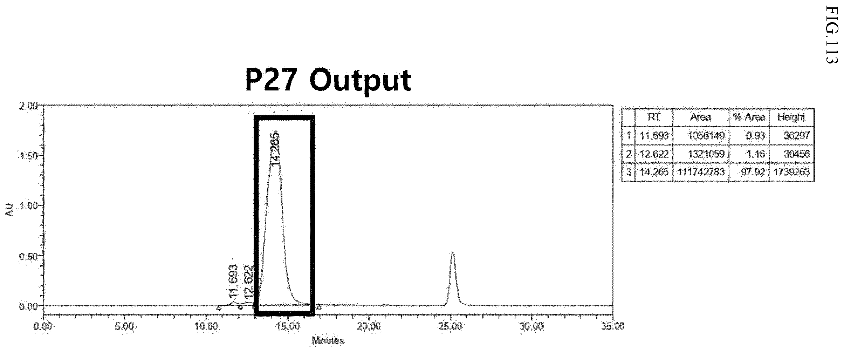

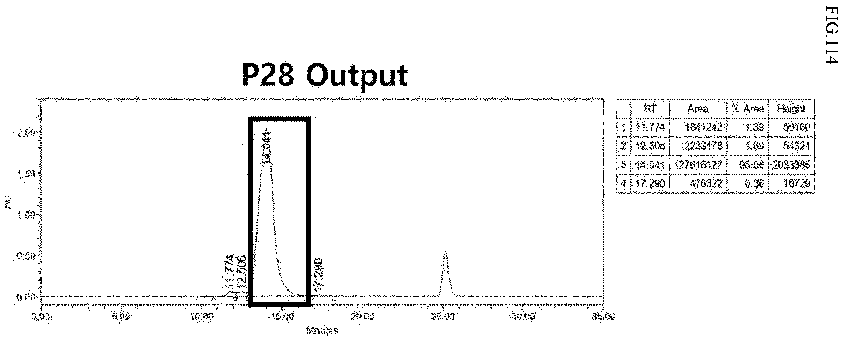

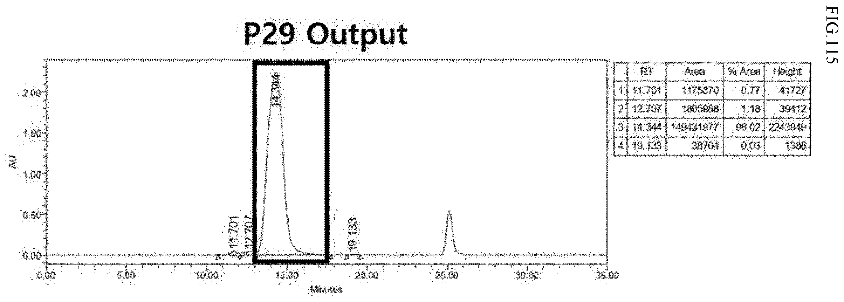

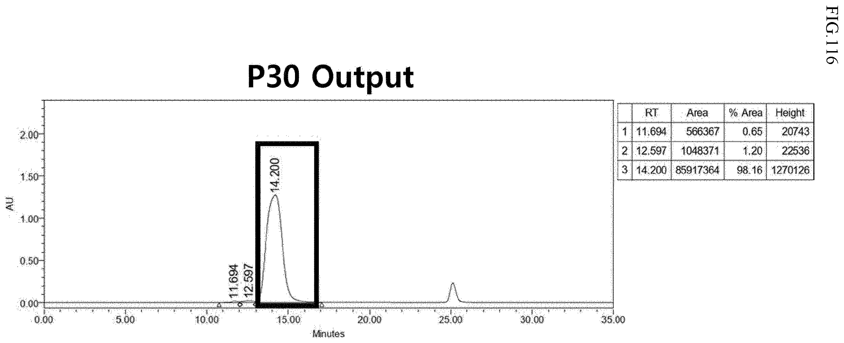

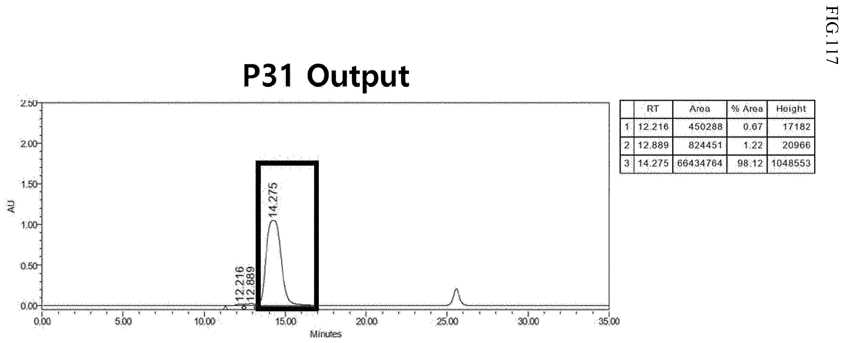

Thereafter, absorbance at 214 nm wavelength was measured with size exclusion chromatography using a TSKgel G3000SWXI, column (TOSOH Bioscience), to obtain a highly concentrated fusion protein. The isolated and purified fusion protein was subjected to SDS-PAGE under reduced (R) or non-reduced (NR) condition, and it was identified by staining with Coomassie Blue (FIGs. 52 to 82). In addition, the results obtained by analysis using size exclusion chromatography are illustrated in FIGs. 85 to 117.

### Preparation Example 1.2.2. Purification of GI-101_P32 to GI-101_P35

Primary purification was carried out using chromatography containing BAKEROND^{™}XWP 500 PolyQuat-35. The fusion protein was bound thereto under a condition of 50 mM Tris, pH 8. Thereafter, elution was performed under a condition of 50 mM Tris, 500 mM NaCl, pH 8. 0.5 N NaOH was placed in a collection tube, and then the fusion protein was collected. Thereafter, secondary purification was carried out using chromatography containing BAKEROND^{™}XWP 500 PolyHi-Propyl-35. The fusion protein was bound thereto under a condition of 50 mM Tris, 1.0 or 1.2 M AmSO₄, pH 7.5 (or 50 mM Tris, 1.0 M AmSO₄, 500 mM Arginine, pH 7.5).

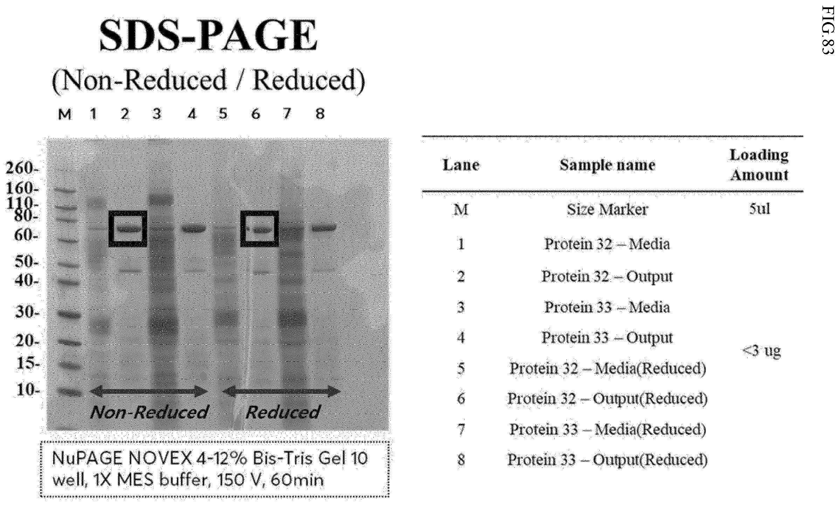

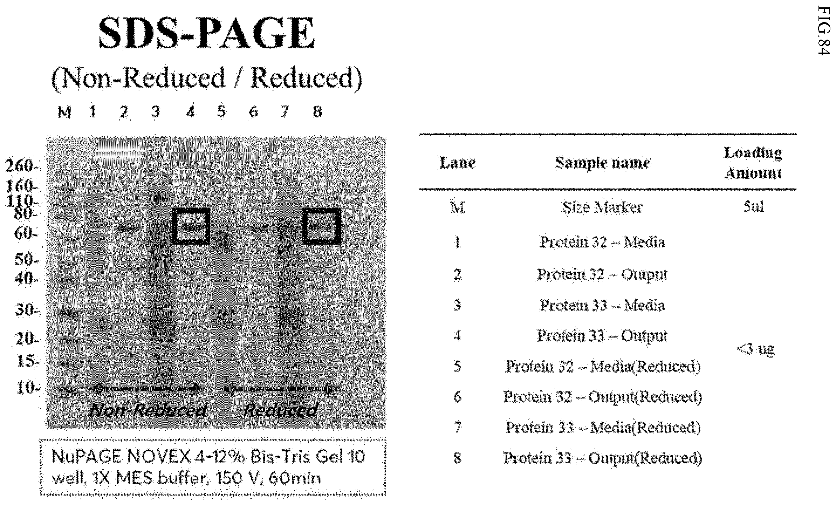

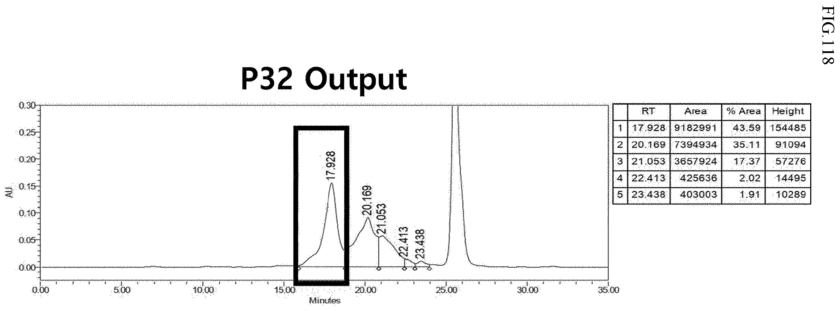

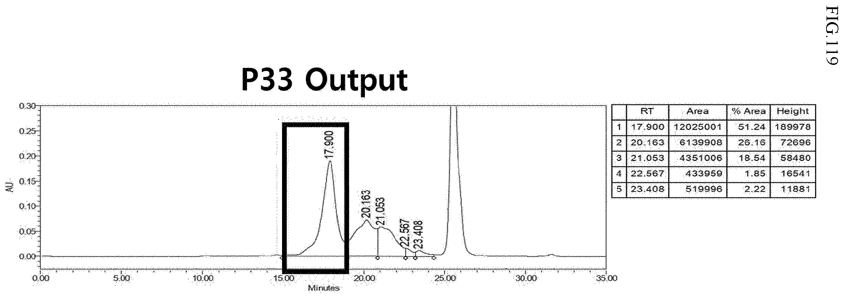

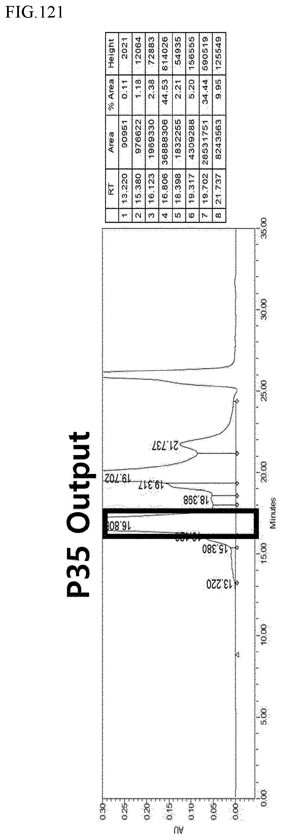

Thereafter, elution was performed under a condition of 50 mM Tris, pH 7.5 (or 50 mM Tris, 500 mM Arginine, pH 7.5). 0.5 N NaOH was placed in a collection tube, and then the fusion protein was collected. Thereafter, absorbance at 214 nm wavelength was measured with size exclusion chromatography using a TSKgel G3000SWXI, column (TOSOH Bioscience), to obtain a highly concentrated fusion protein. Here, the isolated and purified fusion protein was subjected to SDS-PAGE under reduced (R) or non-reduced (NR) condition, and it was identified by staining with Coomassie Blue (FIGs. 83 to 86). In addition, the results obtained by analysis using size exclusion chromatography are illustrated in FIGs. 118 to 121.

### Preparation Example 2. Preparation of hCD80 variant-Fc-IL-2 variant (2M): GI-101_P36 to GI-101_P42

### Preparation Example 2.1. Construction of hCD80 variant-Fc-IL-2 variant (2M) expression vector

In order to produce a fusion protein comprising a human CD80 variant, an Fc domain, and an IL-2 variant, a total of 6 polynucleotides were synthesized through the Invitrogen GeneArt Gene Synthesis service of ThermoFisher Scientific. Specifically, the polynucleotide contains a nucleotide sequence which encodes a fusion protein that contains a signal peptide, a CD80 protein fragment, an Ig hinge, an Fc domain, a linker, and an IL-2 variant (2M) (R38A and F42A) having two amino acid substitutions, in this order, from the N-terminus. The polynucleotide was inserted into pcDNA3_4 vector.

The constructions of the hCD80 variant-Fc-IL-2 variant (2M) (GI-101_P36 to GI-101_P42) fusion proteins are summarized in Table 2.

**[Table 2]**

| **Code** # | **Construction** | | | | |
|---|---|---|---|---|---|
| | 1^{st} API (hCD80 variant) | 1^{st} Linker | IgG Fc | 2^{nd} Linker | 2^{nd} API (hIL2v_2M) |
| GI-101_P36 | K89E, T130A | Fixed | Fixed | Fixed | Fixed |
| GI-101_P37 | K89E, K93E, T130A | | | | |
| GI-101_P38 | H18L, R29D, Y31L, Q33H, K36G, M38I, T41A, M43R, M47T, E81V, L85R, K89N, A91T, F92P, K93V, R94L, L97R, T130A, N149S | | | | |
| GI-101_P39 | R29D, Y31L, Q33H, K36G, M38I, T41A, M43R, M47T, E81V, L85R, K89N, A91T, F92P, K93V, R94L, A12G, V68A, L97R, T130A, L183H | | | | |
| GI-101_P40 | A12G, H18L, V22A, N64S, V68A, T130A | | | | |
| GI-101_P41 | A12G, H18L, V22A, N64S, V68M, T130A | | | | |
| GI-101_P42 | V domain | | | | |

### Preparation Example 2.2. Purification of hCD80 variant-Fc-IL-2 variant (2M)

The vector prepared in Preparation Example 2.1. above was introduced into CHO cells (Expi-CHO^{™}) to express each fusion protein. After the vector was introduced, culture was performed for 7 days in an environment of 37°C, 125 RPM, and 5% CO₂ concentration. Then, the culture was harvested, and the fusion protein was purified therefrom. The purified fusion proteins were designated "GI-101_P36 to GI-101_P42".

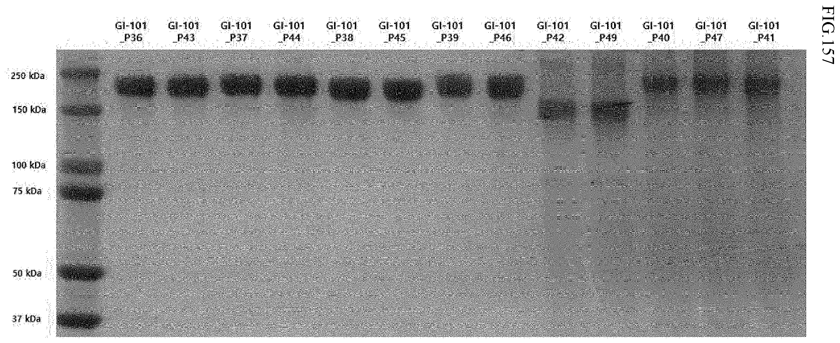

GI-101_P36 to GI-101_P42 were purified using chromatography containing BAKEROND^{™}PROchievA^{™}. The fusion protein was bound thereto under a condition of 50 mM NaPi, 50 mM NaCl, pH 7. Thereafter, washing was performed under a condition of 50 mM NaPi, 500 mM NaCl, pH 7 (or 200 mM Glycine, 300 mM NaCl, pH 5). Then, elution was performed under a condition of 100 or 200 mM Glycine, 300 mM NaCl, pH 3 (or 100 mM Glycine, 300 mM NaCl, 500 mM Arginine, pH 4). 0.3 N NaOH was placed in a collection tube, and then the fusion protein was collected. Thereafter, absorbance at 214 nm wavelength was measured with size exclusion chromatography using a TSKgel G3000SWXI, column (TOSOH Bioscience), to obtain a highly concentrated fusion protein. Here, the isolated and purified fusion protein was subjected to SDS-PAGE under reduced (R) or non-reduced (NR) condition, and it was identified by staining with Coomassie Blue (FIG. 157).

### Preparation Example 3. Preparation of hCD80 variant-Fc-IL-2 variant (3M): GI-101_P43 to GI-101_P49

### Preparation Example 3.1. Construction of hCD80 variant-Fc-IL-2 variant (3M) expression vector

In order to produce a fusion protein comprising a human CD80 variant, an Fc domain, and an IL-2 variant, a total of 6 polynucleotides were synthesized through the Invitrogen GeneArt Gene

Synthesis service of ThermoFisher Scientific. Specifically, the polynucleotide contains a nucleotide sequence which encodes a fusion protein that contains a signal peptide, a CD80 protein fragment, an Ig hinge, an Fc domain, a linker, and an IL-2 variant (3M) (R38A, F42A, and Y45A) having three amino acid substitutions, in this order, from the N-terminus. The polynucleotide was inserted into pcDNA3_4 vector.

The constructions of the hCD80-Fc-IL-2 variant (GI-101_P43 to GI-101_P49) fusion proteins are summarized in Table 3.

**[Table 3]**

| **Code** # | **Construction** | | | | |
|---|---|---|---|---|---|
| | 1^{st} API (hCD80 variant) | 1^{st} Linker | IgG Fc | 2^{nd} Linker | 2^{nd} API (hIL2v_3M) |
| GI-101_P43 | K89E, T130A | Fixed | Fixed | Fixed | Fixed |
| GI-101_P44 | K89E, K93E, T130A | | | | |
| GI-101_P45 | H18L, R29D, Y31L, Q33H, K36G, M38I, T41A, M43R, M47T, E81V, L85R, K89N, A91T, F92P, K93V, R94L, L97R, T130A, N149S | | | | |
| GI-101_P46 | R29D, Y31L, Q33H, K36G, M38I, T41A, M43R, M47T, E81V, L85R, K89N, A91T, F92P, K93V, R94L, A12G, V68A, L97R, T130A, L183H | | | | |
| GI-101_P47 | A12G, H18L, V22A, N64S, V68A, T130A | | | | |
| GI-101_P48 | A12G, H18L, V22A, N64S, V68M, T130A | | | | |
| GI-101_P49 | V domain | | | | |

### Preparation Example 3.2. Purification of hCD80 variant-Fc-IL-2 variant (3M)

The vector prepared in Preparation Example 3.1. above was introduced into CHO cells (Expi-CHO^{™}) to express each fusion protein. After the vector was introduced, culture was performed for 7 days in an environment of 37°C, 125 RPM, and 5% CO₂ concentration. Then, the culture was harvested, and the fusion protein was purified therefrom. The purified fusion proteins were designated "GI-101_P43 to GI-101_P49".

GI-101_P43 to GI-101_P49 were purified using chromatography containing BAKEROND^{™}PROchievA^{™}. The fusion protein was bound thereto under a condition of 50 mM NaPi, 50 mM NaCl, pH 7. Thereafter, washing was performed under a condition of 50 mM NaPi, 500 mM NaCl, pH 7 (or 200 mM Glycine, 300 mM NaCl, pH 5). Then, elution was performed under a condition of 100 or 200 mM Glycine, 300 mM NaCl, pH 3 (or 100 mM Glycine, 300 mM NaCl, 500 mM Arginine, pH 4). 0.3 N NaOH was placed in a collection tube, and then the fusion protein was collected. Thereafter, absorbance at 214 nm wavelength was measured with size exclusion chromatography using a TSKgel G3000SWXI, column (TOSOH Bioscience), to obtain a highly concentrated fusion protein. Here, the isolated and purified fusion protein was subjected to SDS-PAGE under reduced (R) or non-reduced (NR) condition, and it was identified by staining with Coomassie Blue (FIG. 157).

### Experimental Example 1. Identification of binding affinity between hCTLA-4 and GI-101_P1 to GI-101_P35

Ni-NTA biosensor (Nickel charged Tris-NTA, ForteBio, 18-5101) was previously hydrated with 200 µl of 1X Ni-NTA kinetic buffer (10X Kinetics buffer, ForteBio, 18-1042) in a 96-well microplate (GreinerBio-one, Cat:655209). A ligand (CTLA-4 protein, Human CTLA-4/CD152, His-Tag, Aero, CT4-H5229) to be attached to the biosensor was diluted with 1X Ni-NTA kinetic buffer to a concentration of 1 µg/ml. The fusion protein (GI-101_P1 to GI-101_P35) to be attached to the ligand was diluted with 1X kinetic buffer to a concentration of 100 nM. 80 µl of each reagent was placed in a 384-well microplate (GreinerBio-one, Cat:781209), and the program was set up.

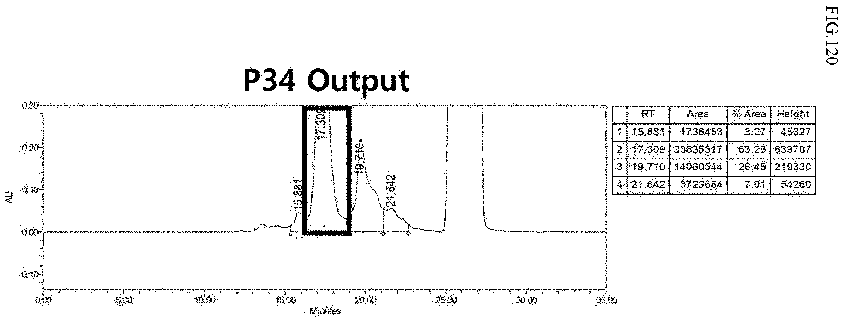

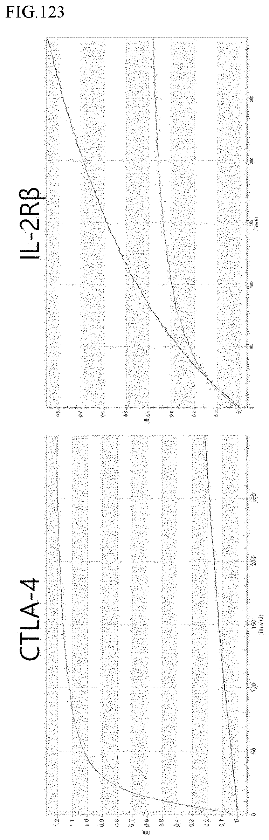

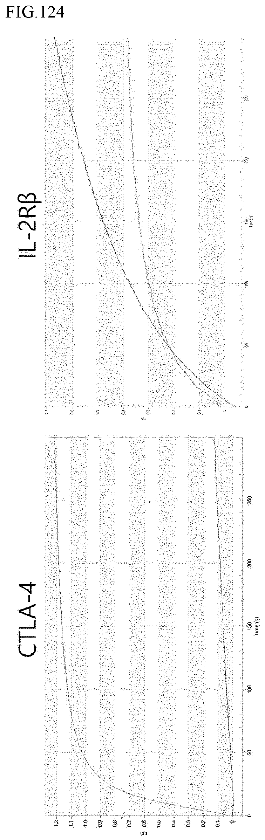

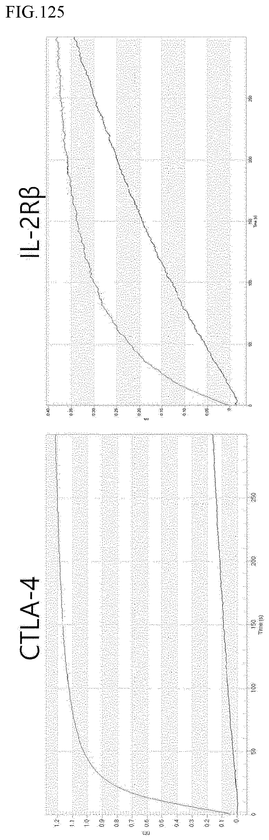

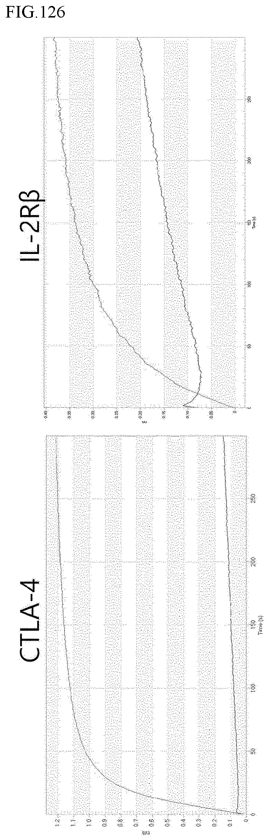

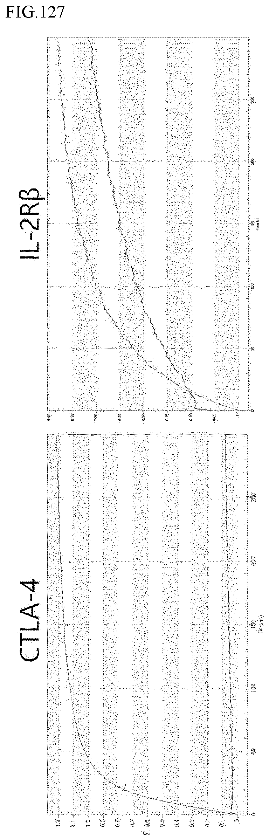

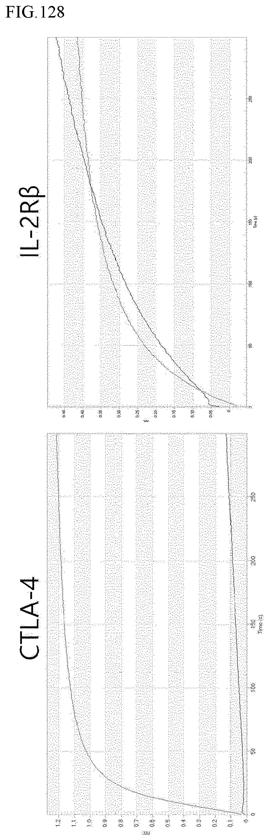

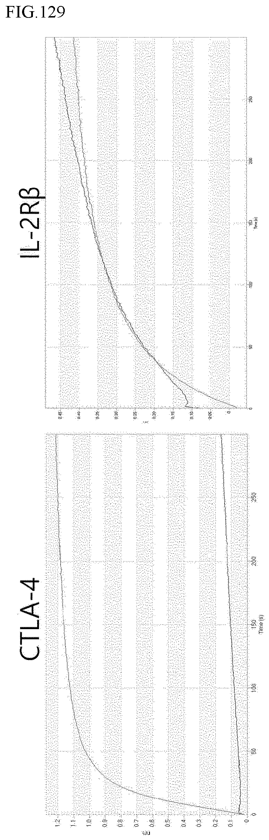

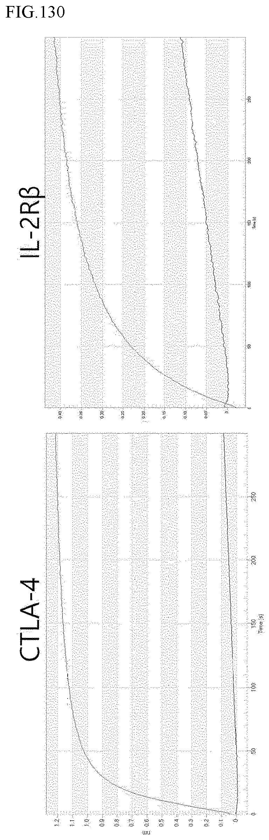

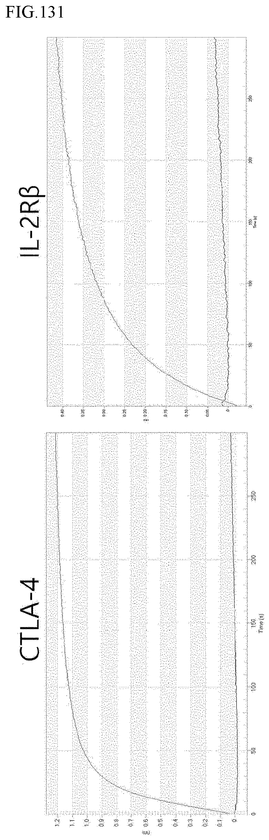

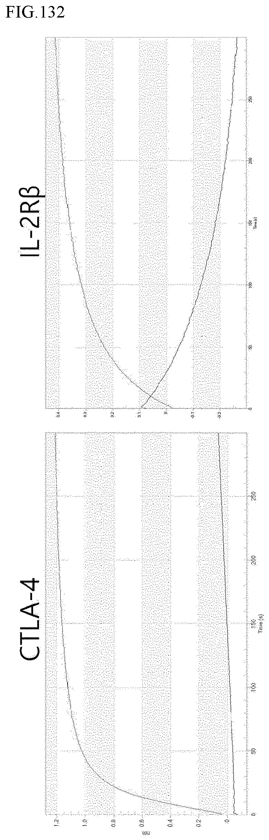

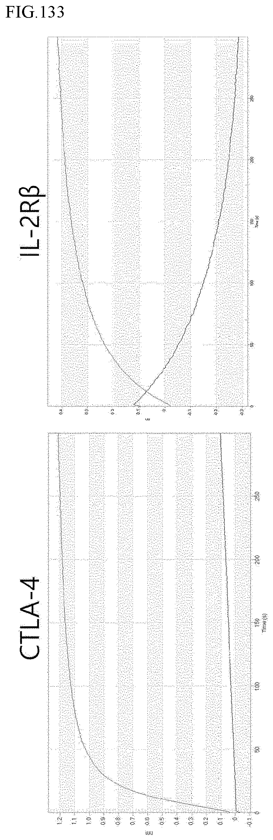

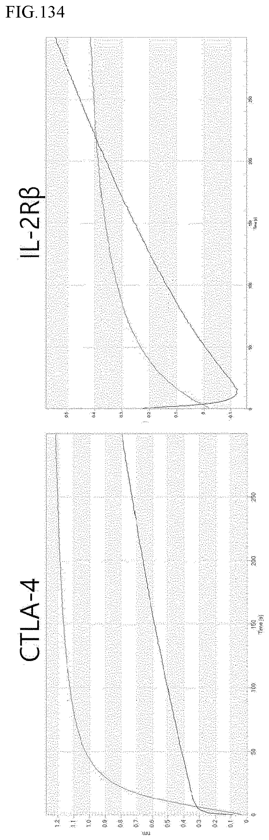

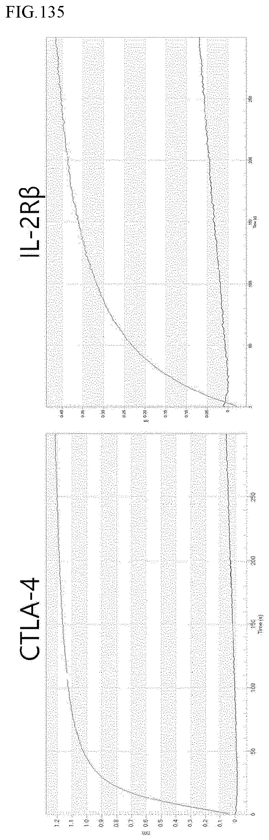

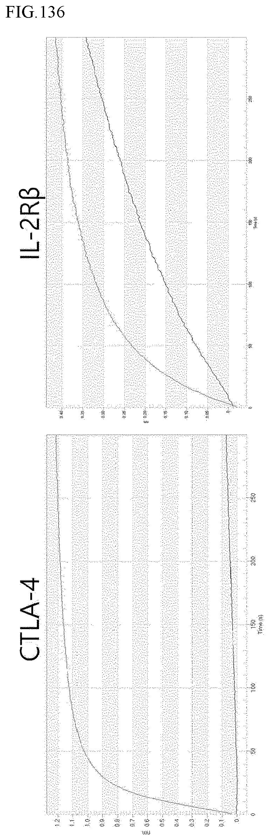

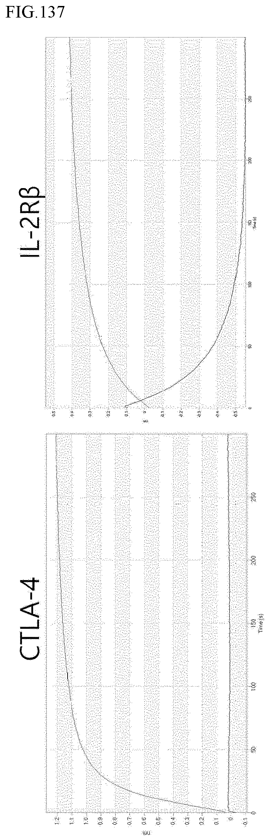

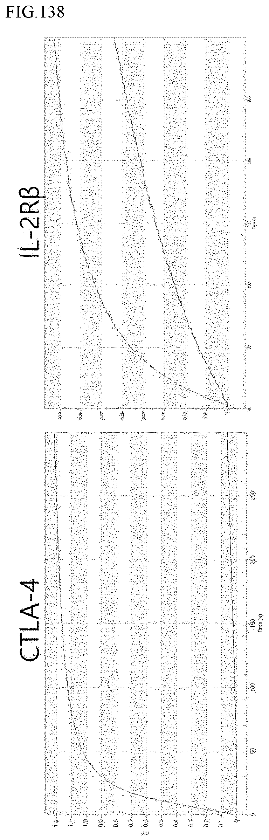

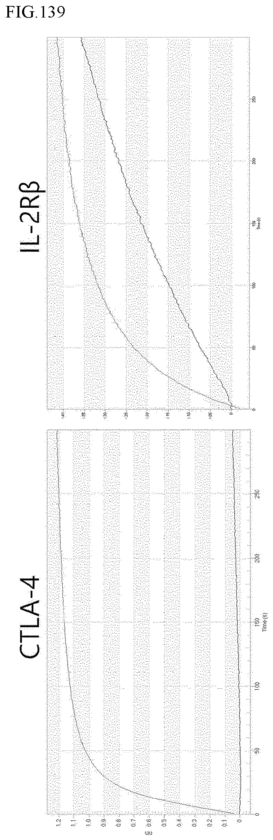

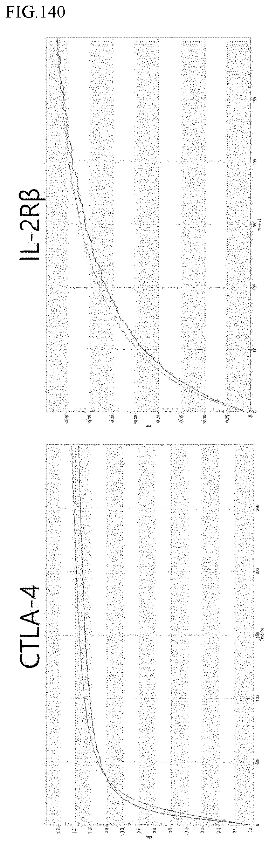

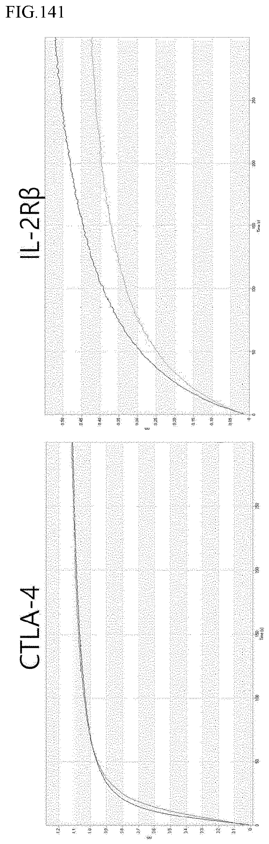

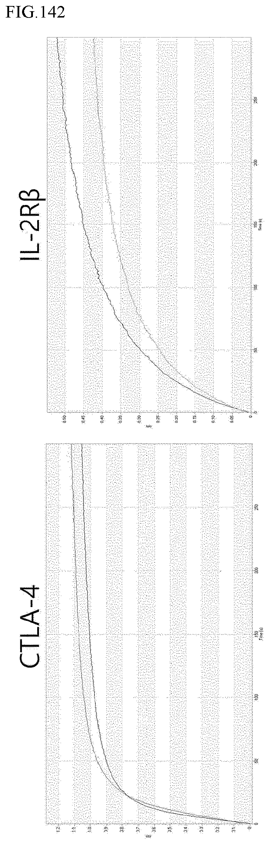

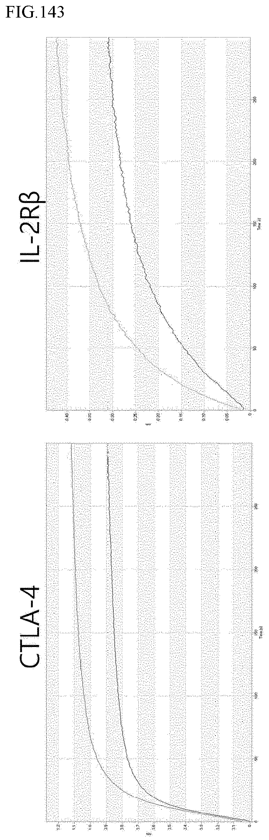

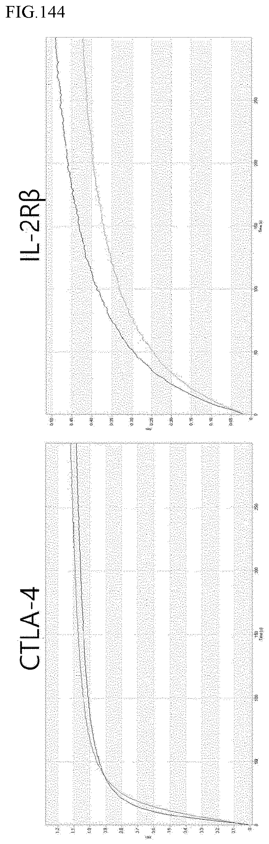

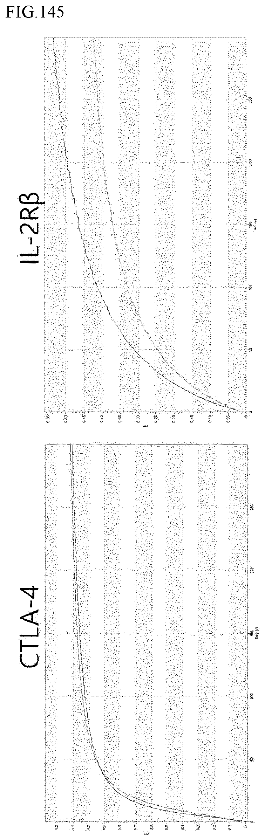

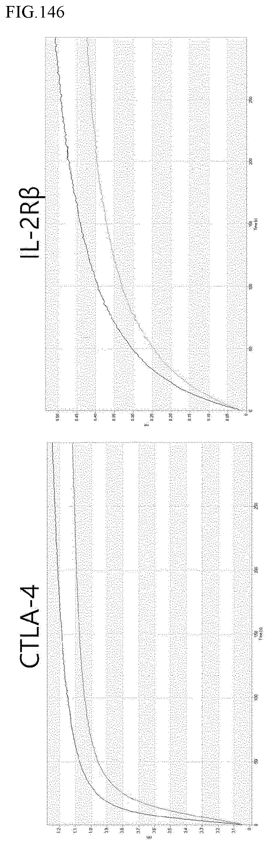

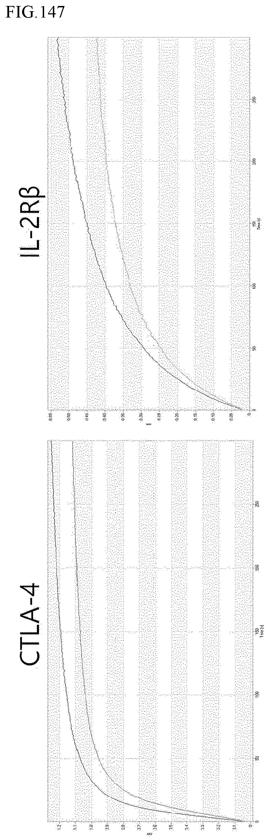

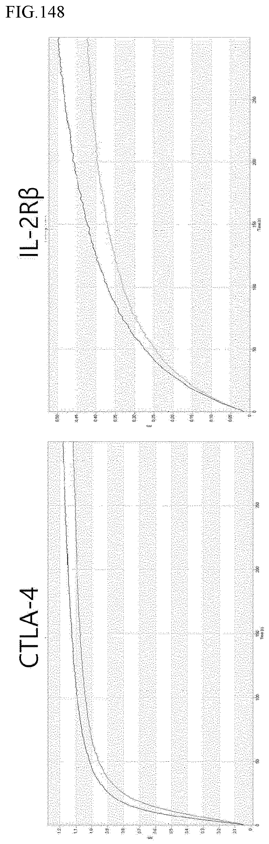

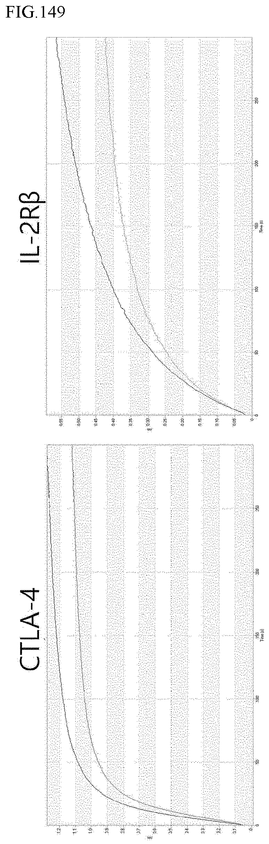

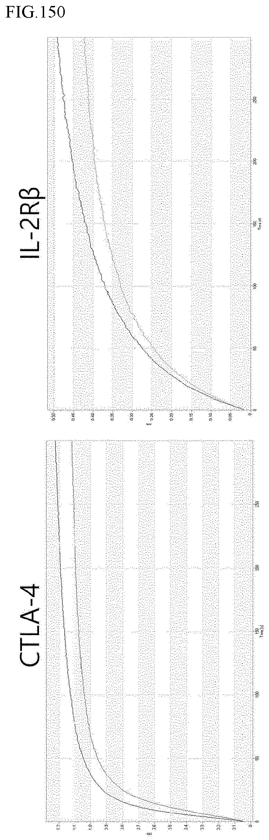

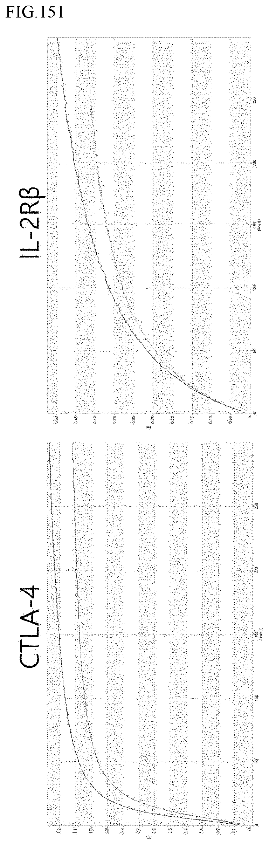

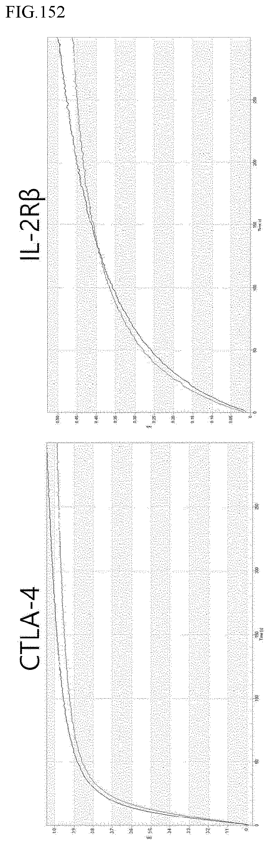

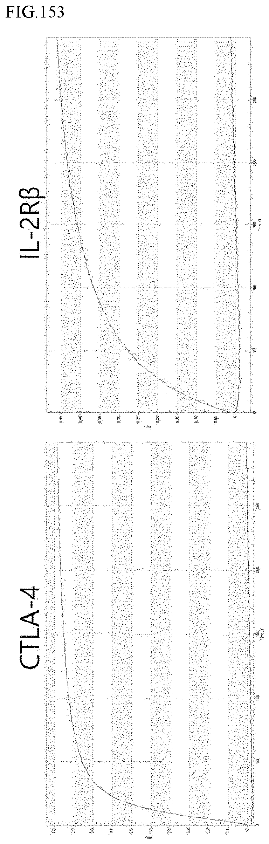

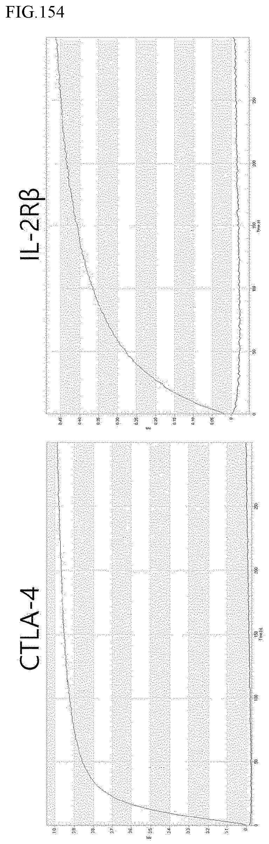

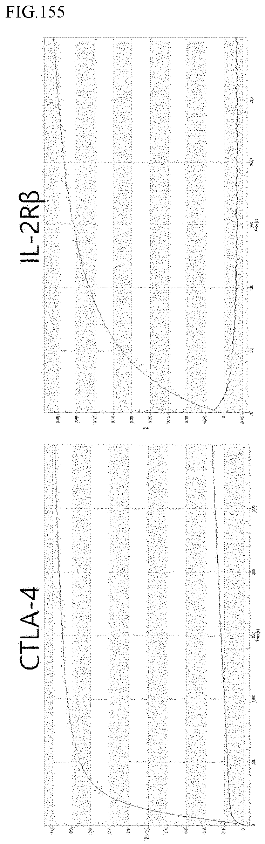

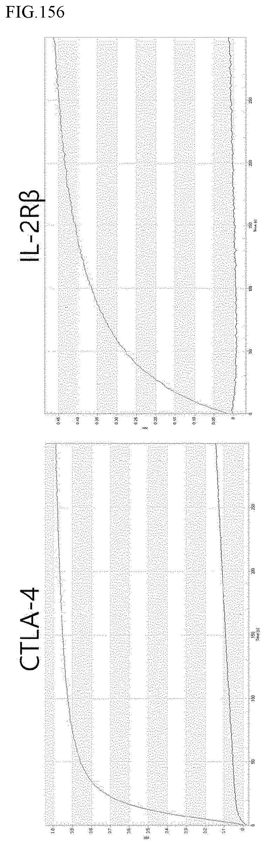

As a result, the binding affinity between hCTLA-4 and GI-101_P1 to GI-101_P35 was measured as illustrated in FIGs. 120 to 156, respectively.

### Experimental Example 2. Identification of binding affinity between hIL-2 beta receptor and GI-101_P1 to GI-101_P35

Ni-NTA biosensor (Nickel charged Tris-NTA, ForteBio, 18-5101) was previously hydrated with 200 µl of 1X Ni-NTA kinetic buffer (10X Kinetics buffer, ForteBio, 18-1042) in a 96-well microplate (GreinerBio-one, Cat:655209). A ligand (Human IL-2 R beta/CD122 protein, His-Tag, Acro, CD2-H5221) to be attached to the biosensor was diluted with 1X Ni-NTA kinetic buffer to a concentration of 2 µg/ml. The fusion protein (GI-101_P1 to GI-101_P35) to be attached to the ligand was diluted with 1X kinetic buffer to a concentration of 100 nM. 80 µl of each reagent was placed in a 384-well microplate (GreinerBio-one, Cat:781209), and the program was set up.

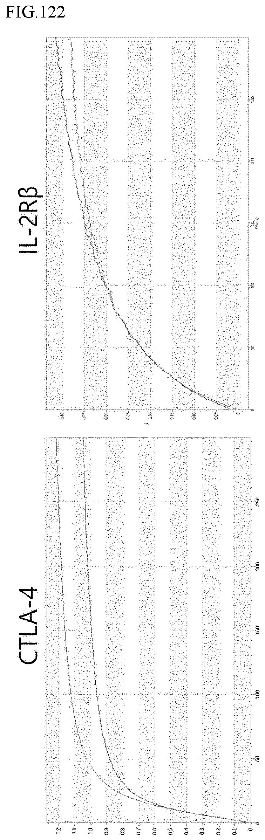

As a result, the binding affinity between hIL-2 beta receptor and GI-101_P1 to GI-101_P35 was measured as illustrated in FIGs. 122 to 156, respectively.

### Experimental Example 3. Identification of binding affinity between hCTLA-4 and GI-101_P36 to GI-101_P49

Ni-NTA biosensor (Nickel charged Tris-NTA, ForteBio, 18-5101) was previously hydrated with 200 µl of 1X Ni-NTA kinetic buffer (10X Kinetics buffer, ForteBio, 18-1042) in a 96-well microplate (GreinerBio-one, Cat:655209). A ligand (CTLA-4 protein, Human CTLA-4/CD152, His-Tag, Aero, CT4-H5229) to be attached to the biosensor was diluted with 1X Ni-NTA kinetic buffer to a concentration of 1 µg/ml. The fusion protein (GI-101_P36 to GI-101_P47) to be attached to the ligand was diluted with 1X kinetic buffer to a concentration of 100 nM. 80 µl of each reagent was placed in a 384-well microplate (GreinerBio-one, Cat:781209), and the program was set up.

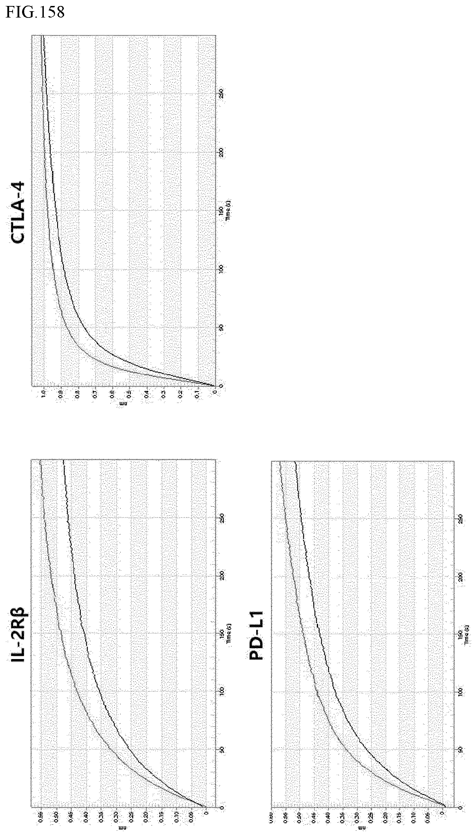

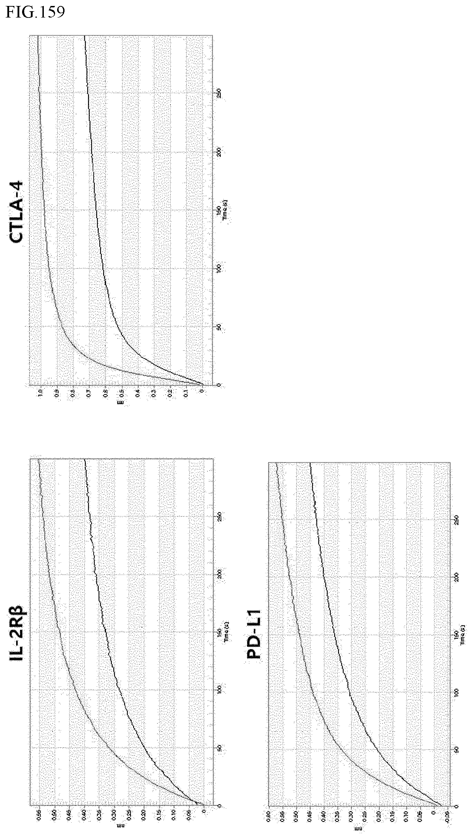

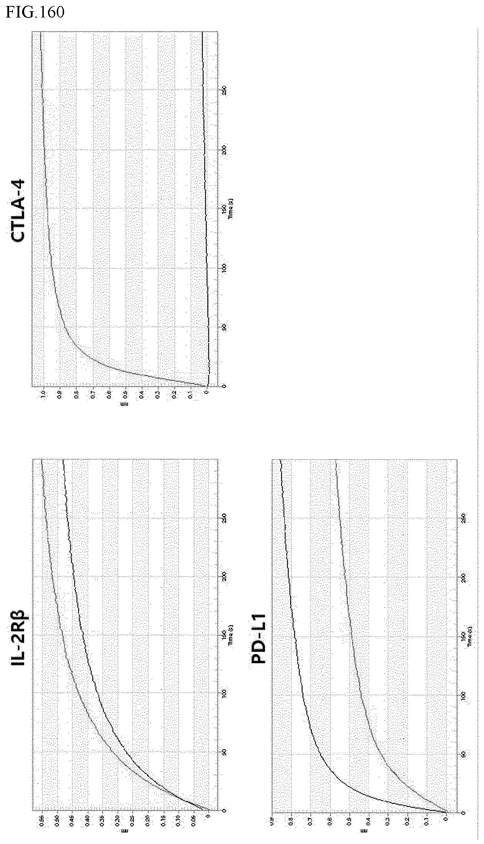

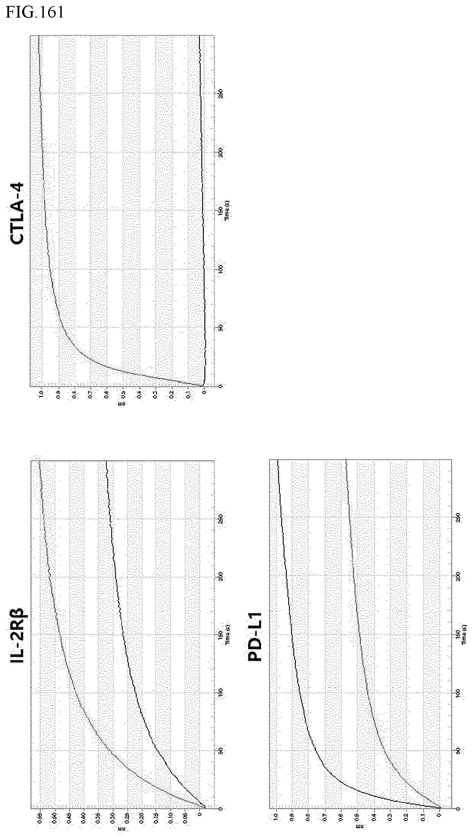

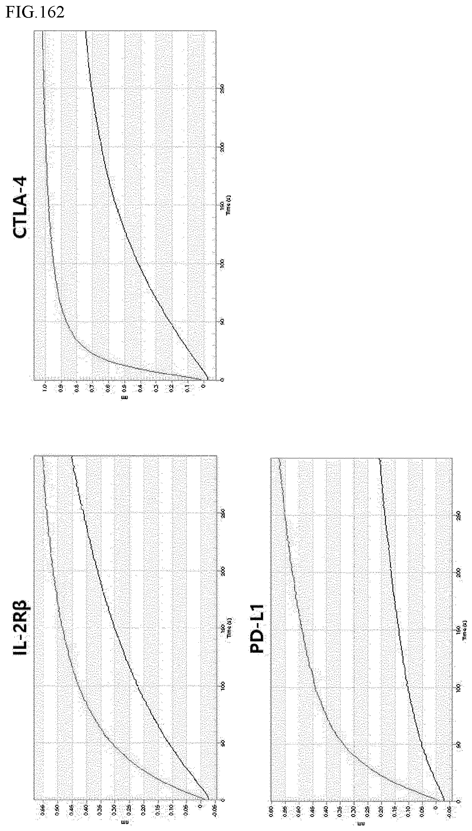

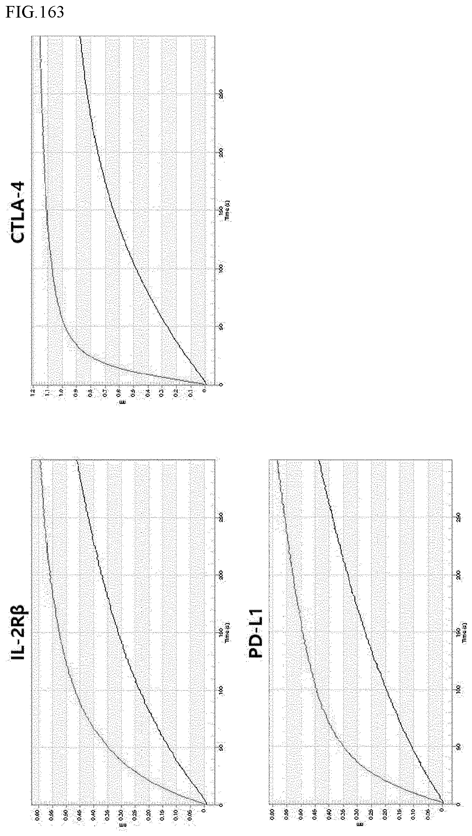

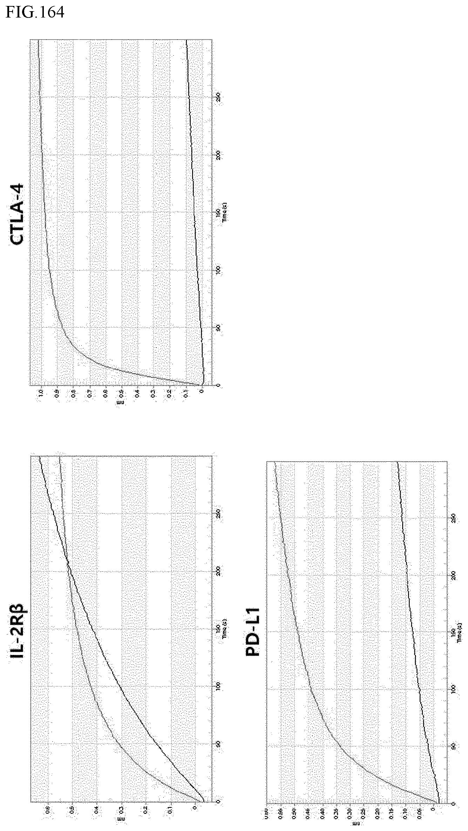

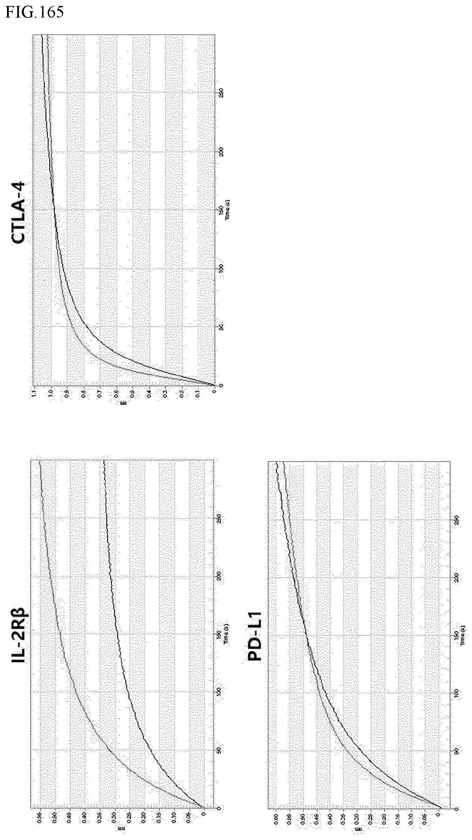

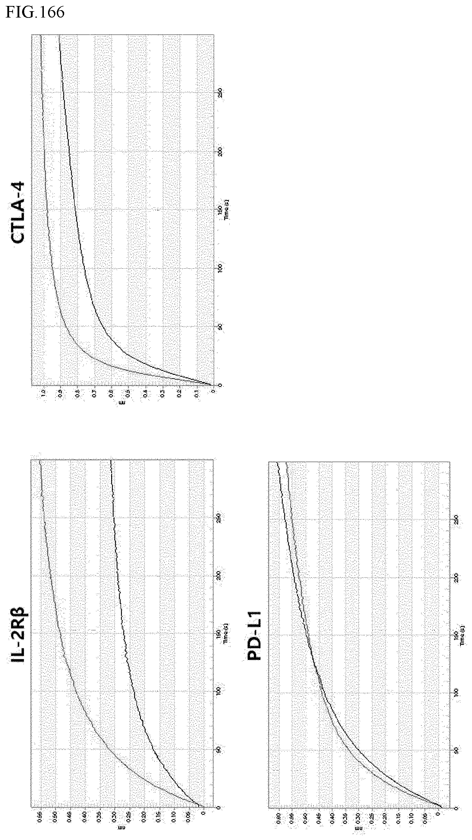

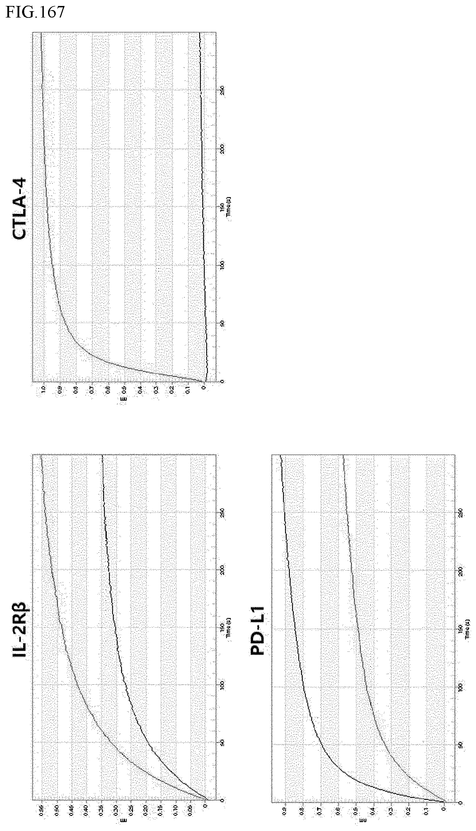

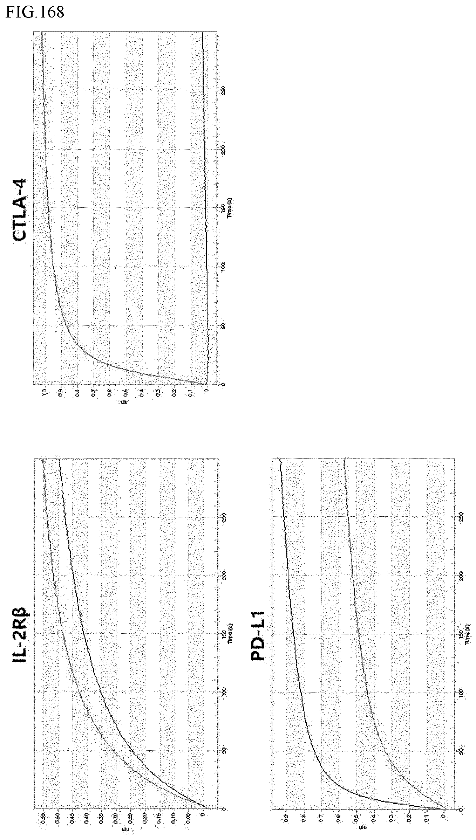

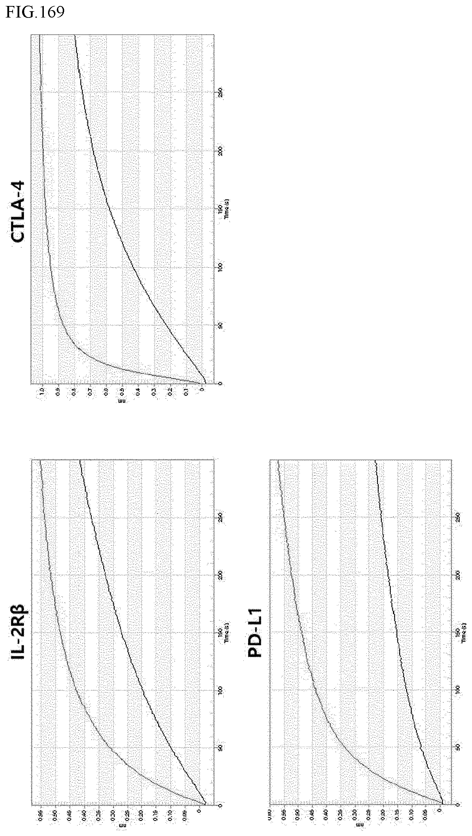

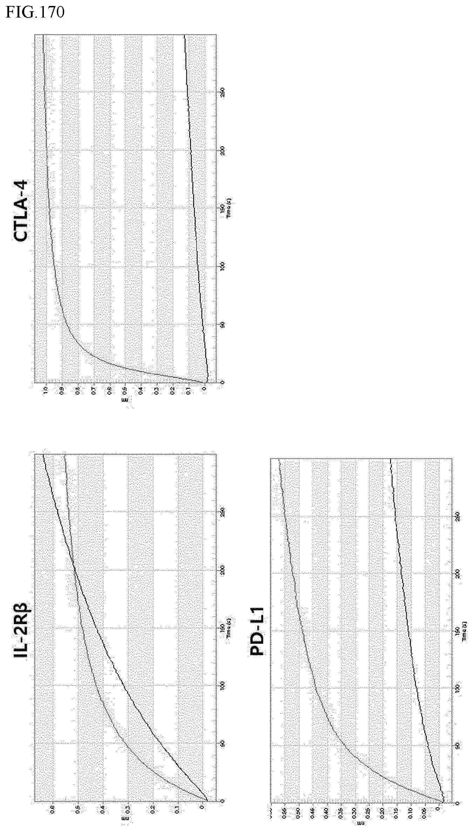

As a result, the binding affinity between hCTLA-4 and GI-101_P36 to GI-101_P49 was measured as illustrated in FIGs. 158 to 170, respectively.

### Experimental Example 4. Identification of binding affinity between hPD-L1 and GI-101_P36 to GI-101_P49

Ni-NTA biosensor (Nickel charged Tris-NTA, ForteBio, 18-5101) was previously hydrated with 200 µl of 1X Ni-NTA kinetic buffer (10X Kinetics buffer, ForteBio, 18-1042) in a 96-well microplate (GreinerBio-one, Cat:655209). A ligand (PD-L1 protein, Human PD-L1/B7-H1 Protein, His-Tag Acro, PD1-H5229) to be attached to the biosensor was diluted with 1X Ni-NTA kinetic buffer to a concentration of 1 µg/ml. The fusion protein (GI-101_P36 to GI-101_P49) to be attached to the ligand was diluted with 1X kinetic buffer to a concentration of 100 nM. 80 µl of each reagent was placed in a 384-well microplate (GreinerBio-one, Cat:781209), and the program was set up.

As a result, the binding affinity between hPD-L1 and GI-101_P36 to GI-101_P49 was measured as illustrated in FIGs. 158 to 170, respectively.

### Experimental Example 5. Identification of binding affinity between hIL-2 beta receptor and GI-101_P36 to GI-101_P49

Ni-NTA biosensor (Nickel charged Tris-NTA, ForteBio, 18-5101) was previously hydrated with 200 µl of 1X Ni-NTA kinetic buffer (10X Kinetics buffer, ForteBio, 18-1042) in a 96-well microplate (GreinerBio-one, Cat:655209). A ligand (Human IL-2 R beta/CD122 protein, His-Tag, Acro, CD2-H5221) to be attached to the biosensor was diluted with 1X Ni-NTA kinetic buffer to a concentration of 2 µg/ml. The fusion protein (GI-101 P1 to GI-101 P35) to be attached to the ligand was diluted with 1X kinetic buffer to a concentration of 100 nM. 80 µl of each reagent was placed in a 384-well microplate (GreinerBio-one, Cat:781209), and the program was set up.

As a result, the binding affinity between hIL-2 beta receptor and GI-101_P36 to GI-101_P49 was measured as illustrated in FIGs. 158 to 170, respectively.

## Claims

1. A fusion protein comprising an IL-2 protein and a CD80 protein fragment or a variant thereof.

2. The fusion protein of claim 1, wherein the IL-2 protein and the CD80 protein fragment or variant thereof are bound to each other via a linker.

3. The fusion protein of claim 1, wherein the IL-2 protein has the amino acid sequence of SEQ ID NO: 10.

4. The fusion protein of claim 1, wherein the IL-2 protein is an IL-2 variant.

5. The fusion protein of claim 4, wherein the IL-2 variant is obtained by substitution of at least one selected from the 38^{th}, 42^{nd}, 45^{th}, 61^{st}, and 72^{nd} amino acids in the amino acid sequence of SEQ ID NO: 10.

6. The fusion protein of claim 4, wherein the IL-2 variant is obtained by at least one substitution selected from the group consisting of R38A, F42A, Y45A, E61R, and L72G in the amino acid sequence of SEQ ID NO: 10.

7. The fusion protein of claim 4, wherein the IL-2 variant is obtained by any one selected from the following substitution combinations (a) to (d) in the amino acid sequence of SEQ ID NO: 10:
(a) R38A/F42A
(b) R38A/F42A/Y45A
(c) R38A/F42A/E61R
(d) R38A/F42A/L72G.

8. The fusion protein of claim 4, wherein the IL-2 variant has the amino acid sequence of SEQ ID NO: 6, 22, 23, or 24.

9. The fusion protein of claim 1, wherein the CD80 has the amino acid sequence of SEQ ID NO: 11.

10. The fusion protein of claim 1, wherein the CD80 protein fragment consists of the 35^{th} to 242^{nd} amino acids in the amino acid sequence of SEQ ID NO: 11.

11. The fusion protein of claim 1, wherein the CD80 protein fragment is a V domain of CD80.

12. The fusion protein of claim 1, wherein the variant of the CD80 protein fragment is one in which at least one amino acid is substituted with another amino acid.

13. The fusion protein of claim 1, wherein the variant of the CD80 protein fragment is obtained by any one substitution combination selected from the group consisting of K89E/T130A, K89E/K93E/T130A, H 18L/R29D/Y31L/Q33H/K3 6G/M3 8I/T41 A/M43R/M47T/E81 V/L85R/K89N/A91 T/F92P/K93 V/R94L/L97R/T130A/N149S, R29D/Y31L/Q33H/K36G/M38I/T41A/M43R/M47T/E81V/L85R/K89N/A91T/F92P/K93V/R94 L/A12G/V68A/L97R/T130A/L183H, A12G/H18L/V22A/N64S/V68M/T130A, and A12G/H18L/V22A/N64S/V68M/T130A.

14. The fusion protein of claim 2, wherein the linker is an albumin or an Fc domain of an immunoglobulin.

15. The fusion protein of claim 14, wherein the Fc domain is a wild type or a variant thereof.

16. The fusion protein of claim 14, wherein the Fc domain has the amino acid sequence of SEQ ID NO: 4.

17. The fusion protein of claim 15, wherein the variant of the Fc domain has the amino acid sequence of SEQ ID NO: 12.

18. The fusion protein of claim 1, wherein the fusion protein consists of the following structural formula (I) or (II):
N'-X-[linker (1)]n-Fc domain-[linker (2)]m-Y-C' (I)
N'-Y-[linker (1)]n-Fc domain-[linker (2)]m-X-C' (II)
in the structural formulas (I) and (II),
N' is the N-terminus of the fusion protein,
C' is the C-terminus of the fusion protein,
X is the CD80 protein fragment or variant thereof,
Y is the IL-2 protein,
the linkers (1) and (2) are peptide linkers, and
n and m are each independently 0 or 1.

19. The fusion protein of claim 18, wherein the linker (1) is a peptide linker consisting of the amino acid sequence of SEQ ID NO: 3.

20. The fusion protein of claim 18, wherein the linker (2) is a peptide linker consisting of the amino acid sequence of SEQ ID NO: 5.

21. The fusion protein of claim 18, wherein the fusion protein consists of the structural formula (I).

22. The fusion protein of claim 1, wherein the fusion protein has a sequence identity of 85% or higher to the amino acid sequence of SEQ ID NO: 9, 26, 28, or 30.

23. A fusion protein dimer wherein two of the fusion proteins of any one of claims 1 to 22 are bound to each other.

24. The fusion protein dimer of claim 23, wherein the fusion protein dimer is a homodimer.

25. A polynucleotide encoding the fusion protein of any one of claims 1 to 22.

26. The polynucleotide of claim 25, wherein the polynucleotide has a sequence identity of 85% or higher to the nucleotide sequence of SEQ ID NO: 8, 25, 27, or 29.

27. A vector comprising the polynucleotide of claim 25.

28. A transformed cell into which the vector of claim 25 has been introduced.

29. A pharmaceutical composition for preventing or treating cancer or an infectious disease, comprising, as an active ingredient, the fusion protein of any one of claims 1 to 22; or the fusion protein dimer of claim 23 or 24.

30. The pharmaceutical composition of claim 29, wherein the pharmaceutical composition further comprises a pharmaceutically acceptable carrier.

31. The pharmaceutical composition of claim 29, wherein the cancer is any one selected from the group consisting of gastric cancer, liver cancer, lung cancer, colorectal cancer, breast cancer, prostate cancer, ovarian cancer, pancreatic cancer, cervical cancer, thyroid cancer, laryngeal cancer, acute myeloid leukemia, brain tumor, neuroblastoma, retinoblastoma, head and neck cancer, salivary gland cancer, and lymphoma.

32. The pharmaceutical composition of claim 29, wherein the infectious disease is any one selected from the group consisting of hepatitis B, hepatitis C, human papilloma virus infection, cytomegalovirus infection, viral respiratory disease, and influenza.
